# EUROPEAN PATENT APPLICATION

(11) **EP 4 169 919 A1**
(43) Date of publication of application: **26.04.2023**
(21) Application number: 21825269.0
(22) Date of filing: 16.06.2021
(51) Int. Cl.: C07D 471/04, C07D 487/04, C07D 491/048, A61P 35/00, A61K 31/519

(54) **PYRAZOLO[3,4-D]PYRIMIDINE-3-KETONE DERIVATIVE AS WEE-1 INHIBITOR**

(30) Priority: 17.06.2020 CN 202010557580
(71) Applicant: Wigen Biomedicine Technology (Shanghai) Co., Ltd., Shanghai 201203 (CN)
(72) Inventor: XIE, Yuli, Shanghai 201203 (CN); FAN, Houxing, Shanghai 201203 (CN); QIAN, Lihui, Shanghai 201203 (CN)
(74) Representative: Dr. Langfinger & Partner
(86) International application number: PCT/CN2021/100347
(87) International publication number: WO 2021/254389

(57) **Abstract**

The present invention relates to a novel compound of general formula (1) and/or a pharmaceutically acceptable salt thereof, a composition containing the compound of general formula (1) and/or the pharmaceutically acceptable salt thereof, a method for preparing the same, and use of the same as a Wee-1 inhibitor in preparing anti-tumor drugs.

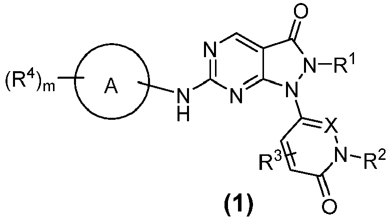

## Description

The present application claims priority to Chinese Patent Application CN 202010557580.X filed on Jun. 17, 2020, which is incorporated herein by reference in its entirety.

### TECHNICAL FIELD

The present invention relates to the field of pharmaceutical chemistry, and particularly to a novel compound with inhibitory effect on Wee-1 kinase, a method for preparing the same and use of such compounds in preparing anti-tumor drugs.

### BACKGROUND

Wee-1 protein kinase is an important negative regulatory protein in cell cycle checkpoints. The cell cycle checkpoints include a G1 checkpoint for the transition from G1 phase (cell resting phase) to S phase (DNA synthesis phase), a G2 checkpoint for the transition from G2 phase (cell division preparation phase) to M phase (cell division phase), and a spindle checkpoint for the transition from metaphase (cell division metaphase) to anaphase (cell division anaphase) of the M phase. The Wee-1 protein kinase plays an important role in the G2 phase checkpoint. Cell entry into M phase depends on CDK1 kinase activity, and Wee-1 inhibits the activity of CDK1 by phosphorylating Tyr 15 of CDK1 protein, preventing cells from entering M phase (cell division phase). In contrast, polo kinase phosphorylates Wee-1 activates the degradation of Wee-1 protein, promoting cells to enter M phase. Thus, Wee-1 kinase activity determines the activity of the G2 checkpoint, thereby regulating the transition from G2 to M phase of cells. The cell cycle checkpoints are activated primarily following DNA damage and play an important role in the repair of DNA in cells. The normal activation of the cell cycle checkpoints blocks the cell cycle and promotes DNA repair. If the functions of the checkpoints are inhibited, the DNA damage is unable to be repaired, and the cells undergo apoptosis. Compared with normal cells, a plurality of tumor cells repair DNA damage and avoid apoptosis mainly depending on the activation of the G2 phase checkpoint due to the impaired function of the important protein p53 protein of the G1 phase checkpoint. Therefore, tumor cells can be selectively killed by inhibiting the G2 phase checkpoint. The important role of Wee-1 kinase activity in the G2 phase checkpoint suggests that Wee-1 kinase determines the repair or death of tumor cells after DNA damage, and inhibition of Wee-1 activity can promote unrepaired tumor cells after DNA damage to enter M phase and induce apoptosis.

Studies have shown that in addition to its role in the G2 checkpoint, Wee-1 is involved in DNA synthesis, DNA homologous repair, post-translational modification of chromosomal histones, and other functions closely related to the development and progression of tumors. The expression of Wee-1 is greatly increased in a large number of tumors including liver cancer, breast cancer, cervical cancer, melanoma, lung cancer and the like. The high expression of Wee-1 is in positive correlation with the tumor development and poor prognosis, suggesting that Wee-1 kinase may be involved in the occurrence and progression of tumors. Studies on *in vitro* cell models and *in vivo* animal models have shown that inhibiting Wee-1 activity while inducing DNA damage can significantly inhibit the growth of a variety of tumors.

Therefore, the development of specific and highly active small-molecule inhibitors against Wee-1 kinase would be of important clinical value for tumor treatment, especially targeting tumors with impaired G1 checkpoints such as P53 deletion.

At present, the Wee-1 inhibitor AZD-1775 of AstraZeneca has entered the clinical phase II stage, and more than 30 clinical trials are under development and have shown good therapeutic effects. Patents related to AZD-1775 include US20070254892, WO2007126122, EP2213673, WO2008133866, WO2011034743 and the like. Abbott and Abbvie also have conducted research on Wee-1 inhibitors. The related patents include mainly US2012220572, WO2013126656, WO2013012681, WO2013059485, WO2013013031 and the like. Patents related to Wee-1 inhibitors of Almac include WO2014167347, WO2015019037, WO2015092431, WO2018011570, WO2018062932, WO2019138227 and the like. Patents related to Wee-1 of Girafpharma include WO2019074979 and WO2019074981. Patents related to Wee-1 research of Zeno include WO2018028008 and WO2019173082.

Currently, there are still some problems with Wee-1 inhibitors under study, for example, the metabolic properties of AZD-1775 are not good enough, and there is a large space for optimization.

### SUMMARY

The present invention provides a compound with a structure as shown in general formula (1) or isomers, crystalline forms, pharmaceutically acceptable salts, hydrates or solvates thereof: 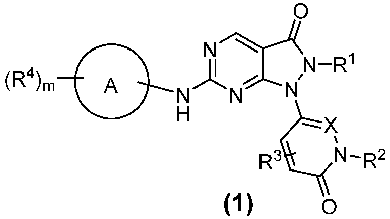 wherein
m is an integer of 1, 2 or 3;
X is Nor CH;
A is a divalent or more than divalent aryl, a divalent or more than divalent heteroaryl, a divalent or more than divalent cycloalkyl-aryl, a divalent or more than divalent heterocycloalkyl-aryl, or a divalent or more than divalent heterocycloalkyl-heteroaryl;
R¹ is C1-C6 alkyl, halogen-substituted C1-C3 alkyl, C3-C6 cycloalkyl, -CH₂(C3-C6) cycloalkyl or C3-C5 alkenyl;
R² is C1-C6 alkyl, C3-C6 cycloalkyl or (4- to 6-membered) heterocycloalkyl, wherein the alkyl, cycloalkyl and heterocycloalkyl can be optionally substituted with 1 to 3 of the following groups: H, halogen, OH, Me or OMe;
R³ is H, halogen, CN, C1-C3 alkyl, halogen-substituted C1-C3 alkyl or C1-C3 alkoxy;
each R⁴ is independently H, halogen, C1-C3 alkyl, halogen-substituted C1-C3 alkyl, hydroxyl-substituted C1-C3 alkyl, C1-C3 alkoxy, NMe₂-substituted C1-C3 alkyl, NMe₂-substituted C1-C3 alkoxy, 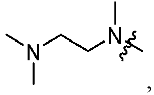 NMe₂, C3-C6 cycloalkyl, (4- to 12-membered) heterocycloalkyl or -CH₂(4- to 12-membered) heterocycloalkyl, wherein the (4- to 12-membered) heterocycloalkyl can be optionally substituted with 1 to 3 R⁵, and each R⁵ is independently H, halogen, CN, OH, C1-C3 alkyl, halogen-substituted C1-C3 alkyl, hydroxyl-substituted C1-C3 alkyl, cyano-substituted C1-C3 alkyl, C3-C6 cycloalkyl, halogen-substituted C3-C6 cycloalkyl, 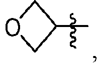 NR⁶R⁷ or -(C1-C3 alkyl)-NR⁶R⁷, wherein R⁶ and R⁷ are independently H or C1-C3 alkyl, or R⁶ and R⁷ form a 4- to 7-membered heterocycloalkyl with an N atom to which they are both connected; wherein two R⁴ can, together with C atoms, form a C2-C3 alkylene; wherein R⁴ or R⁵ cannot be halogen when connected to a heteroatom.

In some embodiments of the present invention, the R¹ is Me, Et,

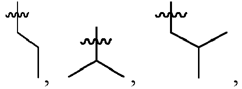

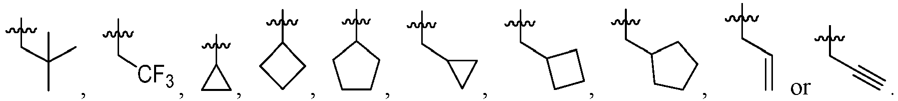

In some embodiments of the present invention, the R² is Me, Et,

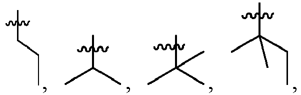

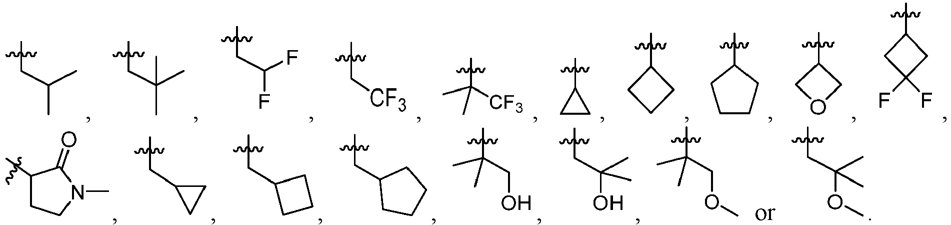

In some embodiments of the present invention, the R³ is H, F, Me, Et, 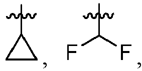 CF₃, OMe or OEt.

In some embodiments of the present invention, the 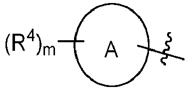 is the following group: 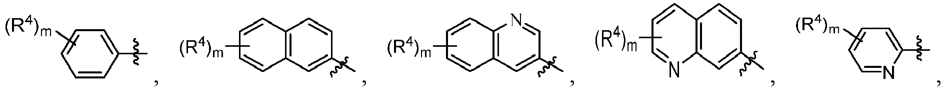 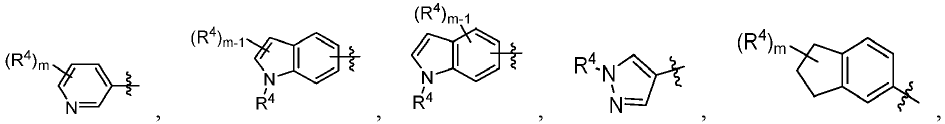 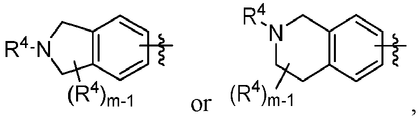 wherein m is an integer of 1, 2 or 3, each R⁴ is independently H, halogen, C1-C3 alkyl, halogen-substituted C1-C3 alkyl, hydroxyl-substituted C1-C3 alkyl, C1-C3 alkoxy, NMe₂-substituted C1-C3 alkyl, NMe₂-substituted C1-C3 alkoxy, 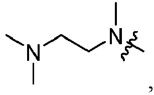 NMe₂, C3-C6 cycloalkyl, (4- to 12-membered) heterocycloalkyl or -CH₂(4- to 12-membered) heterocycloalkyl, wherein the (4- to 12-membered) heterocycloalkyl can be optionally substituted with 1 to 3 R⁵, and R⁵ is independently H, halogen, CN, OH, C1-C3 alkyl, halogen-substituted C1-C3 alkyl, hydroxyl-substituted C1-C3 alkyl, cyano-substituted C1-C3 alkyl, C3-C6 cycloalkyl, halogen-substituted C3-C6 cycloalkyl, 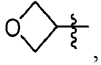 NR⁶R⁷ or - (C1-C3 alkyl)-NR⁶R⁷, wherein R⁶ and R⁷ are independently H or C1-C3 alkyl, or R⁶ and R⁷ form a 4- to 7-membered heterocycloalkyl with an N atom to which they are both attached; wherein two R⁴ can, together with C atoms, form a C2-C3 alkylene; wherein R⁴ or R⁵ cannot be halogen when connected to a heteroatom.

In some embodiments of the present invention, R⁴ is independently H, F, Cl, Me, Et, 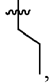 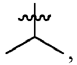 CF₃, CH₂CF₃, CH₂OH, CH₂CH₂OH, OMe, OEt, 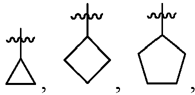 NMe₂, 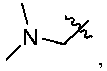 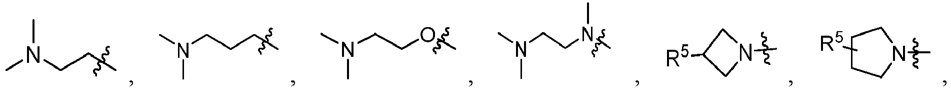 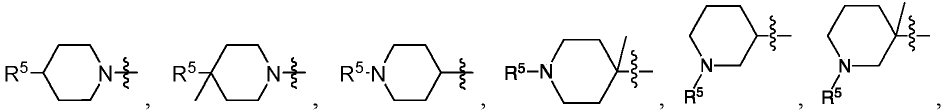 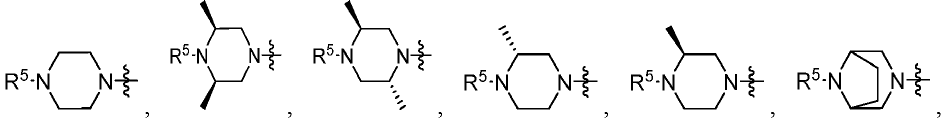 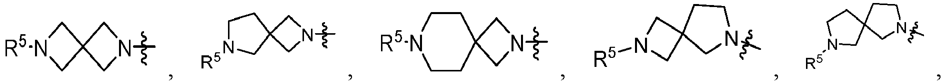 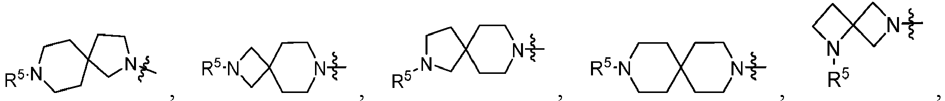 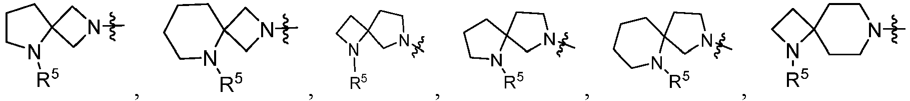 or 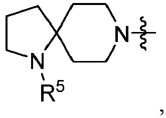 or wherein two R⁴, together with C atoms, form spirocyclopropyl ( 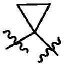 ) or spirocyclobutyl ( 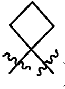 ), wherein R⁵ is H, halogen, CN, OH, C1-C3 alkyl, halogen-substituted C1-C3 alkyl, hydroxyl-substituted C1-C3 alkyl, cyano-substituted C1-C3 alkyl, C3-C6 cycloalkyl, halogen-substituted C3-C6 cycloalkyl, NH₂, NHMe, NMe₂

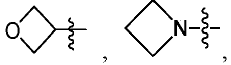

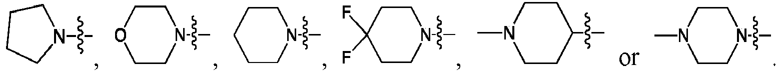

In some embodiments of the present invention, the 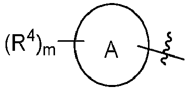 is the following group:

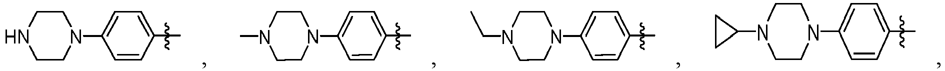

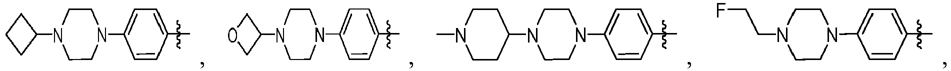

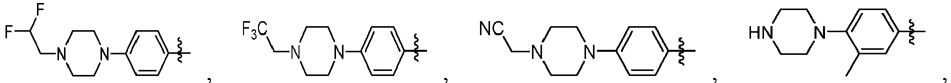

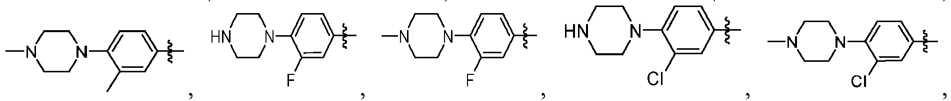

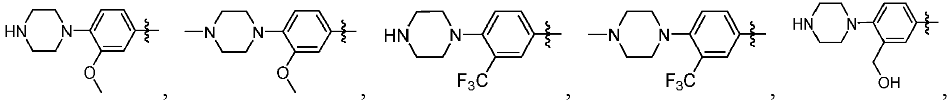

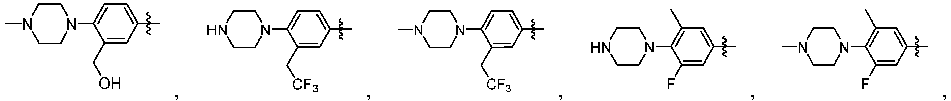

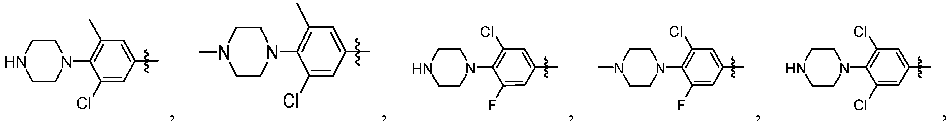

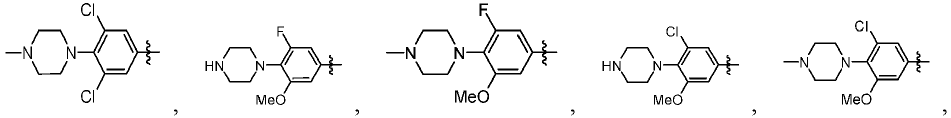

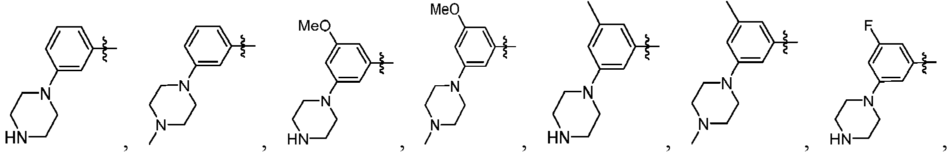

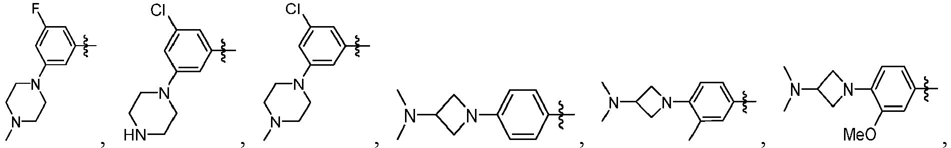

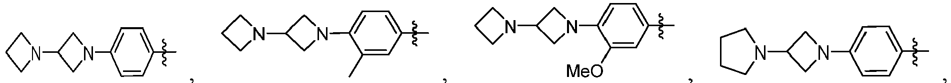

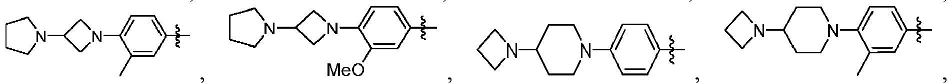

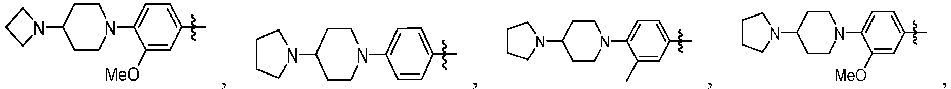

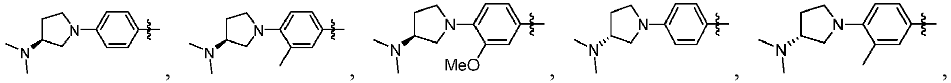

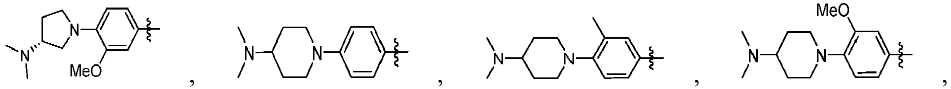

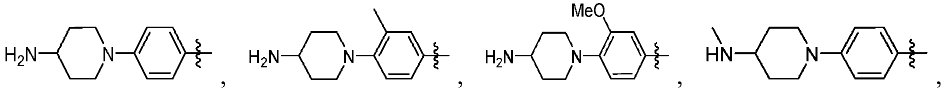

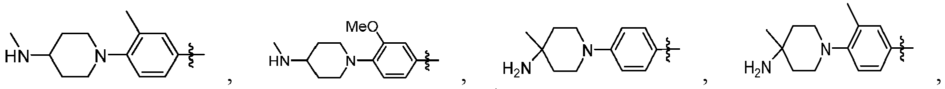

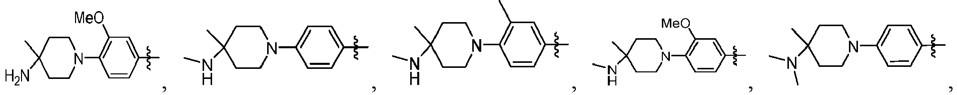

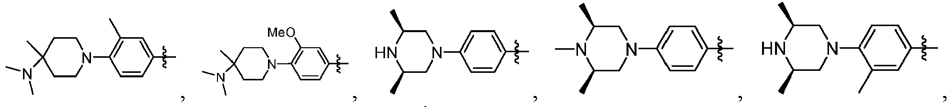

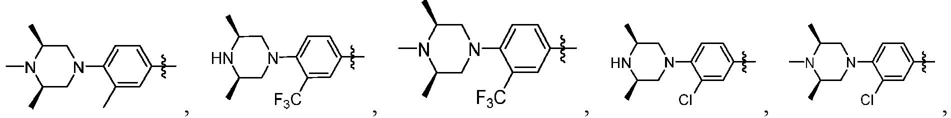

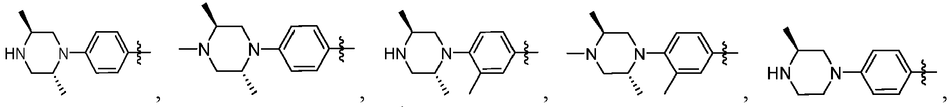

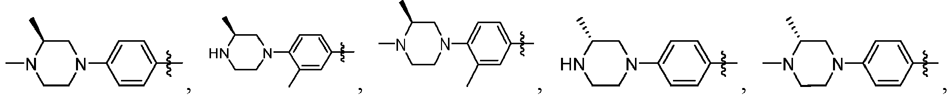

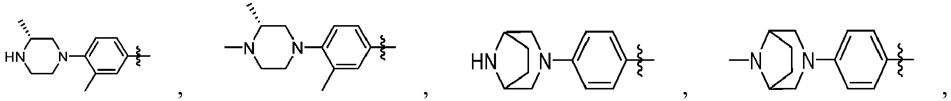

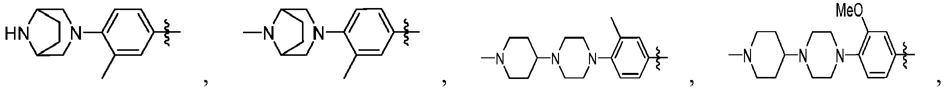

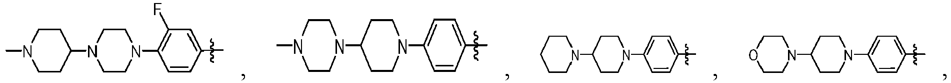

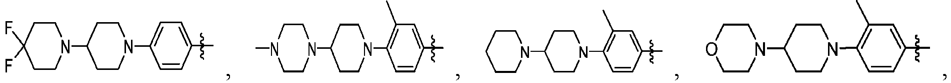

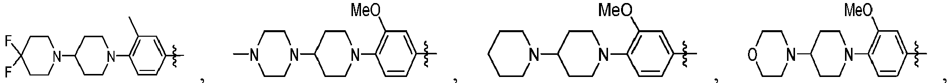

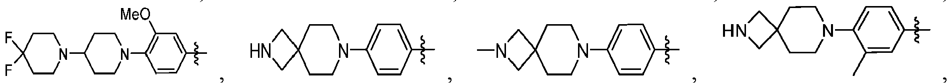

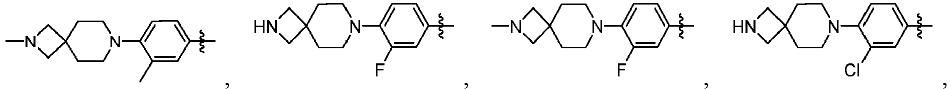

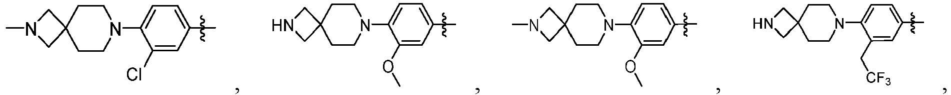

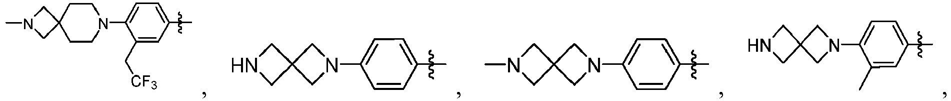

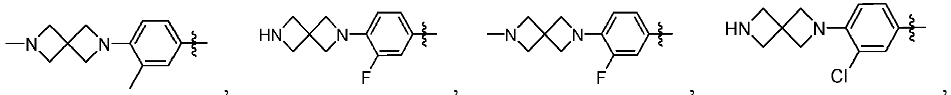

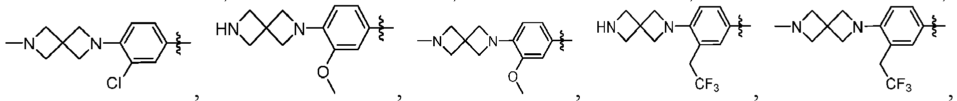

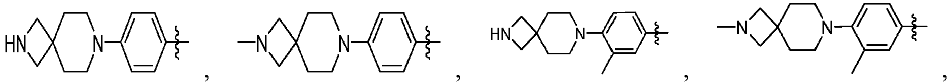

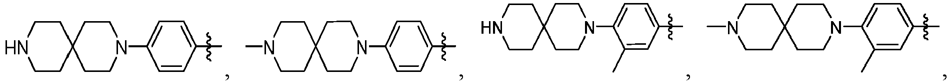

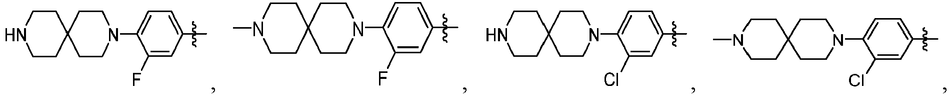

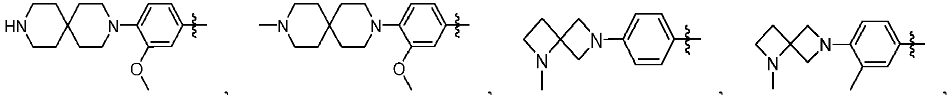

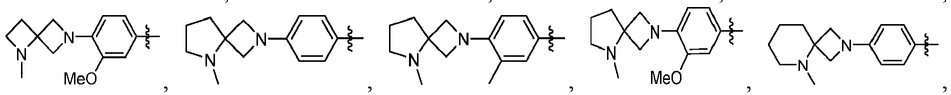

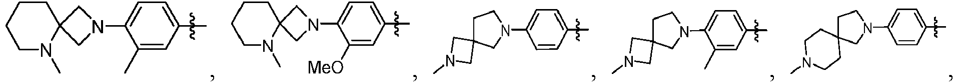

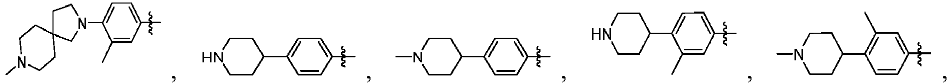

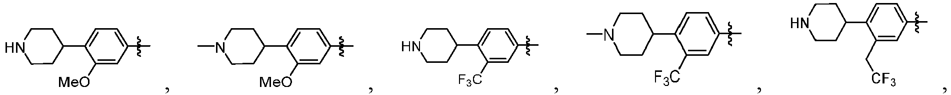

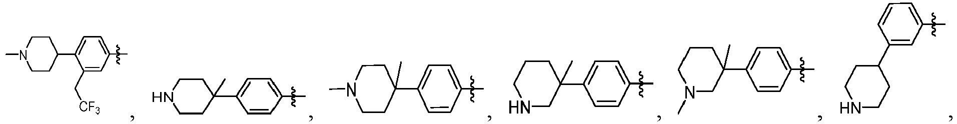

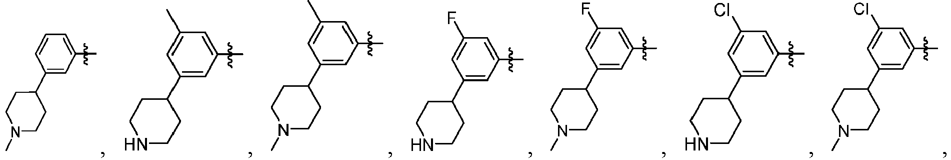

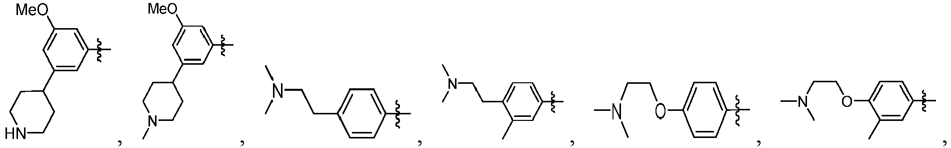

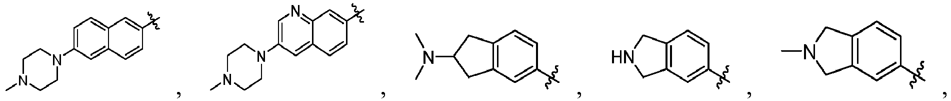

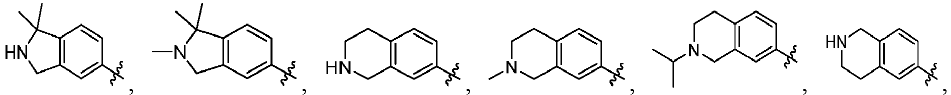

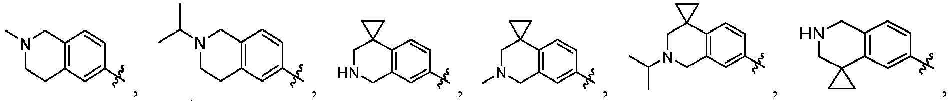

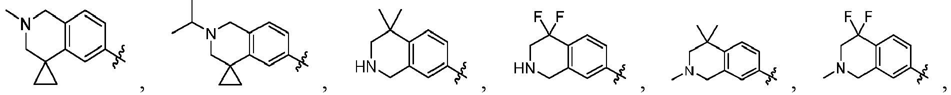

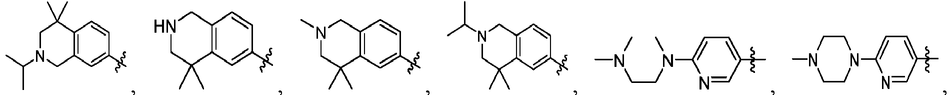

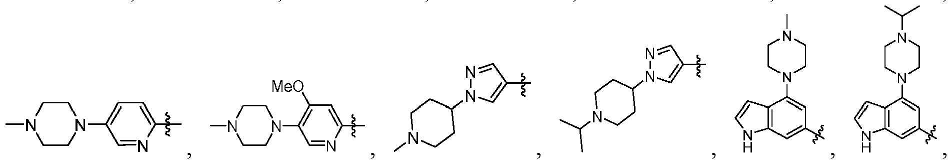

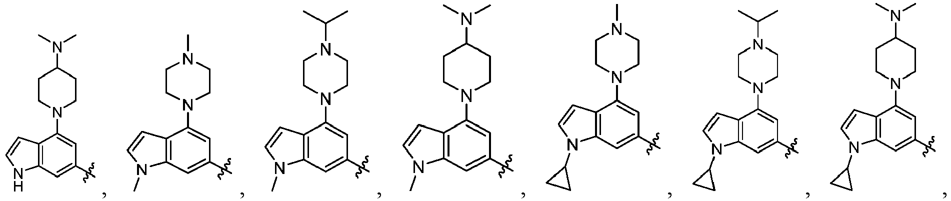

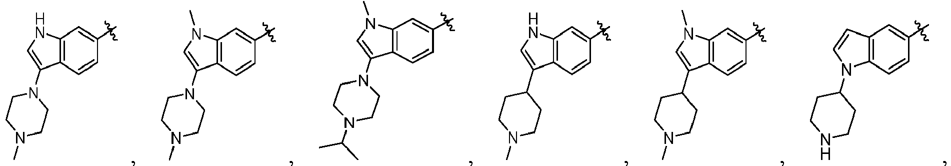

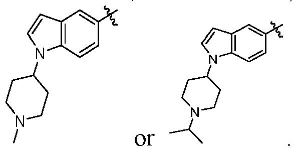

In some embodiments of the present invention, the compound, isomers or pharmaceutically acceptable salts are selected from:

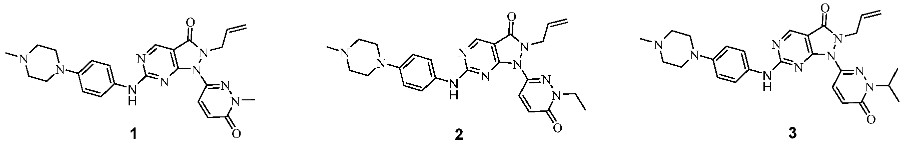

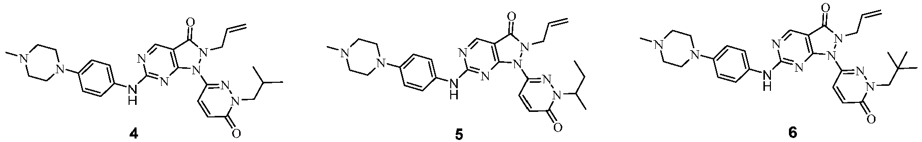

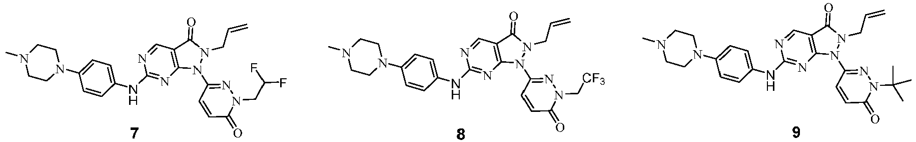

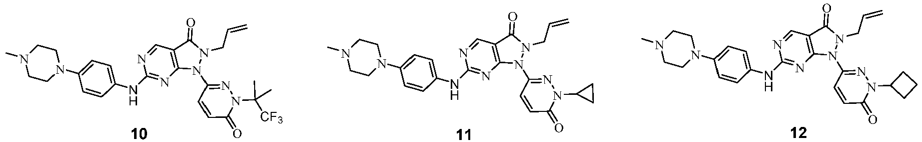

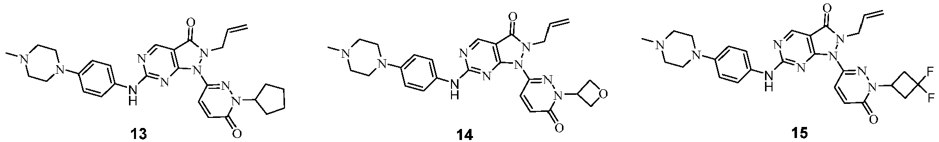

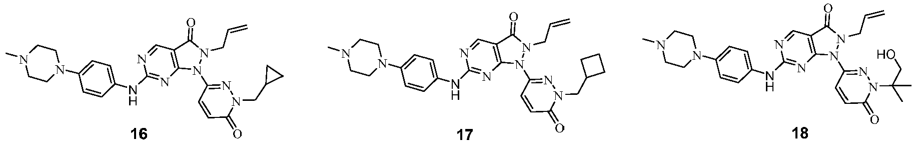

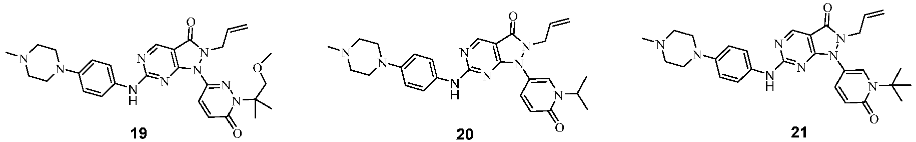

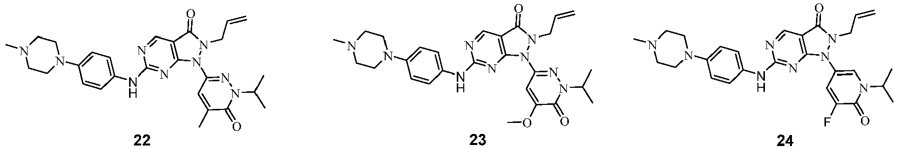

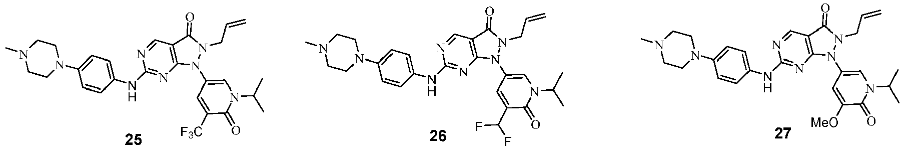

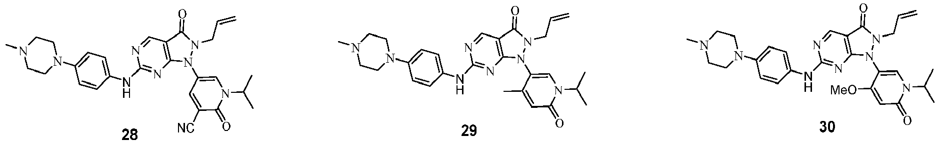

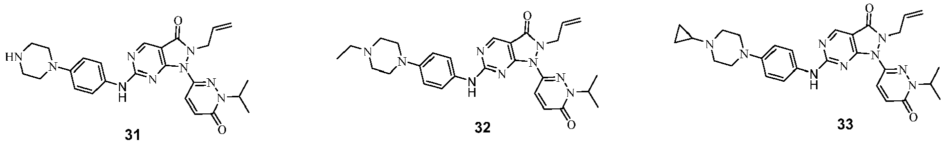

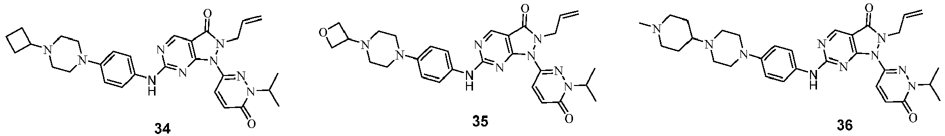

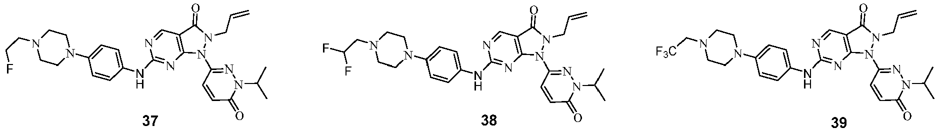

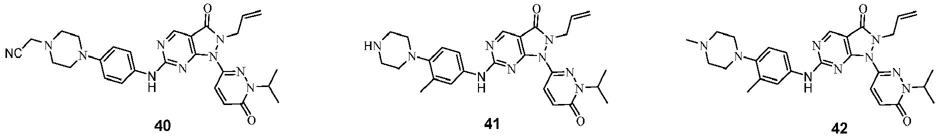

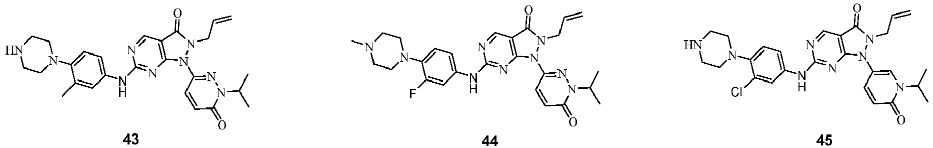

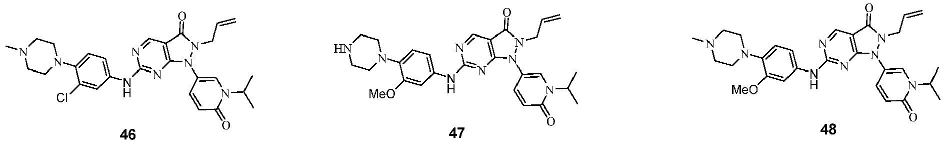

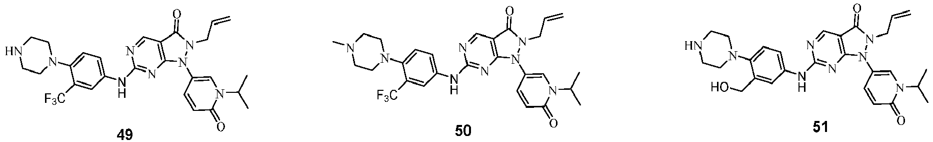

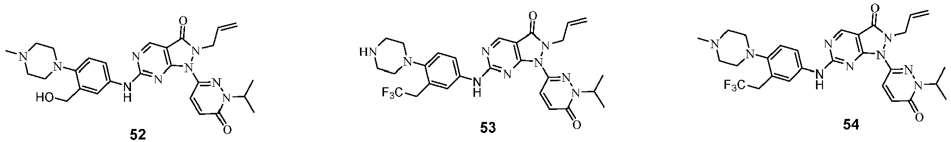

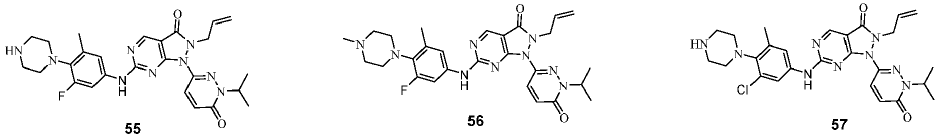

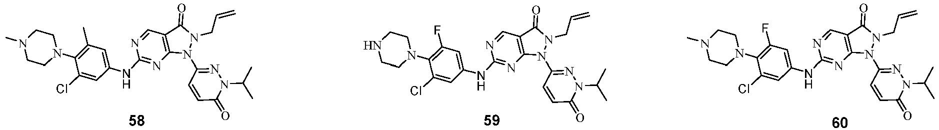

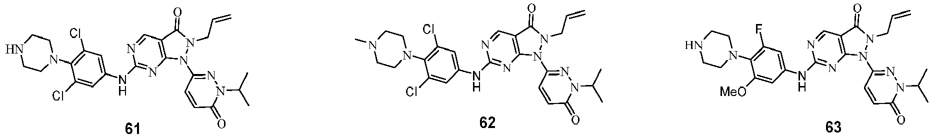

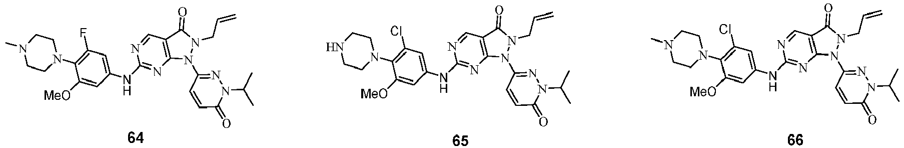

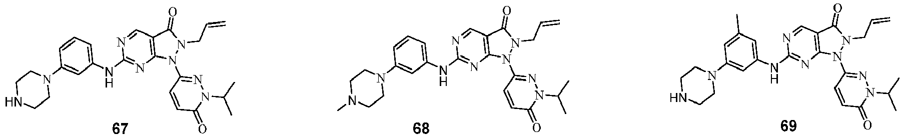

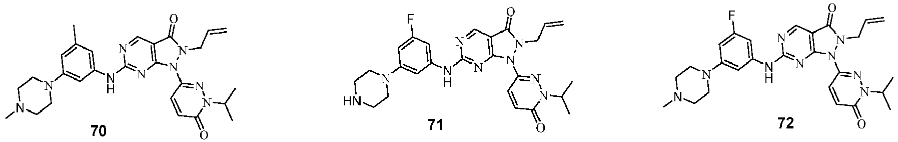

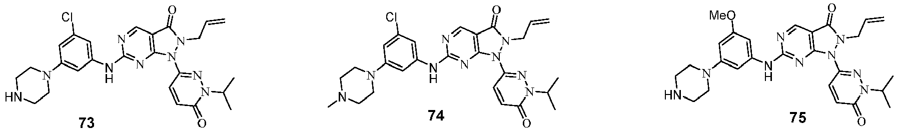

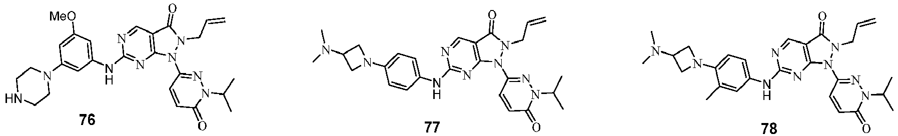

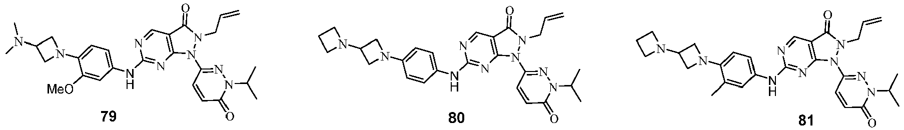

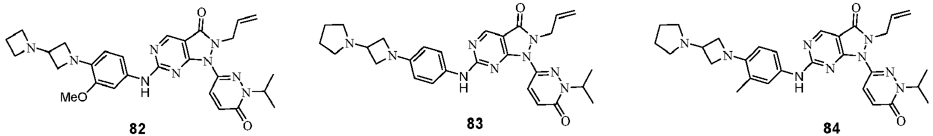

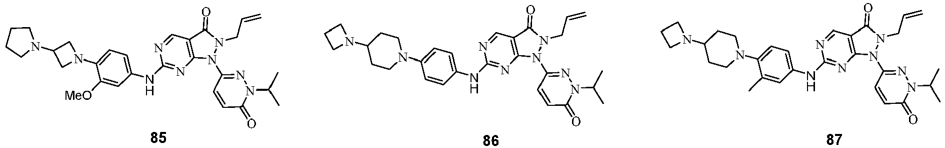

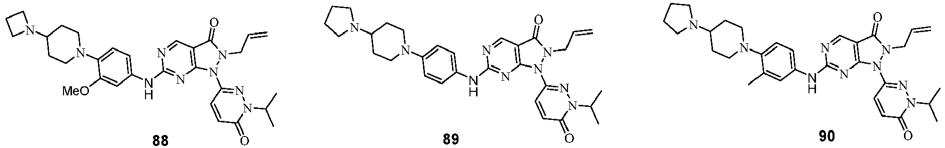

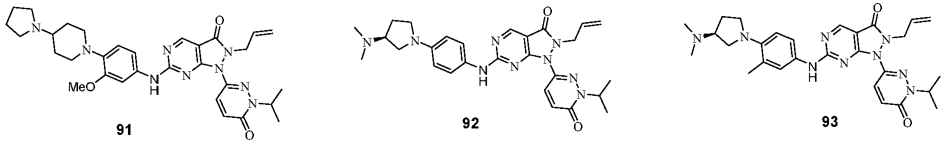

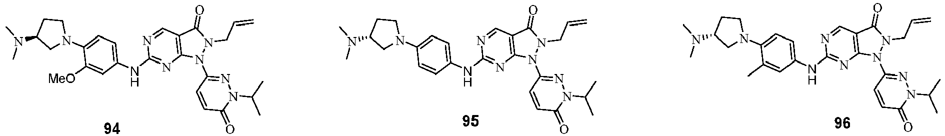

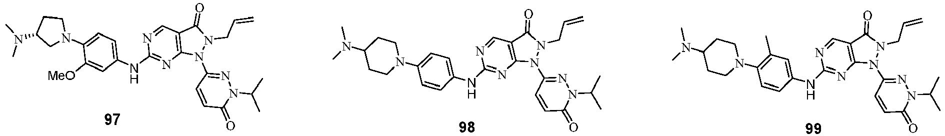

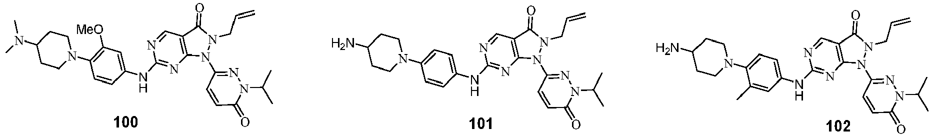

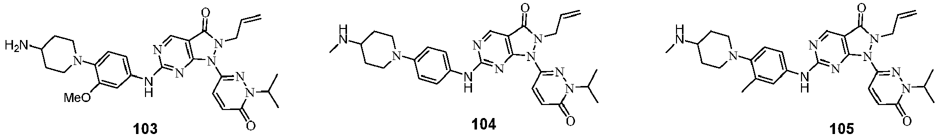

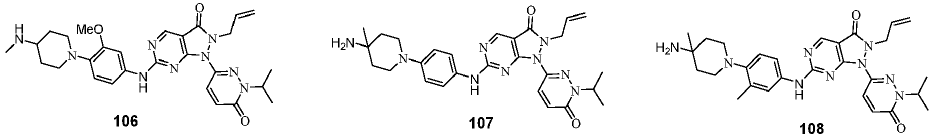

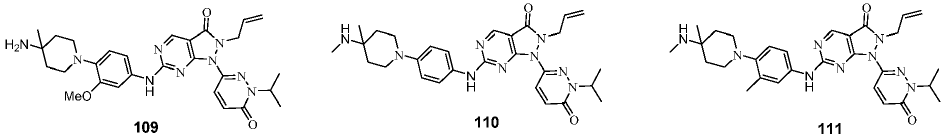

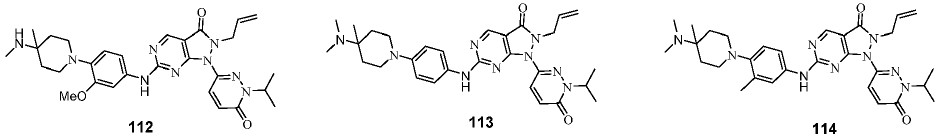

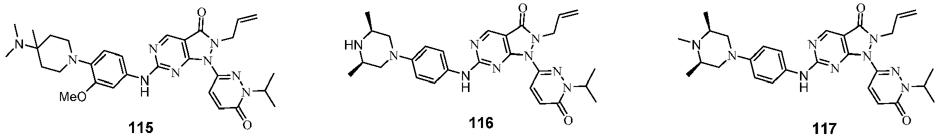

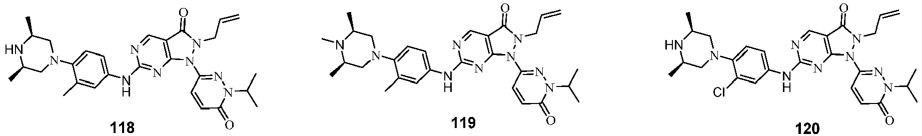

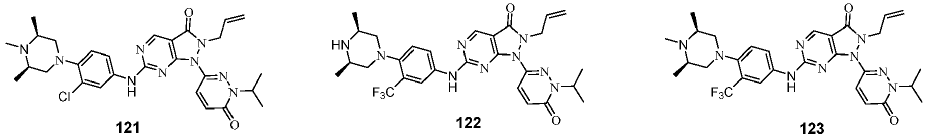

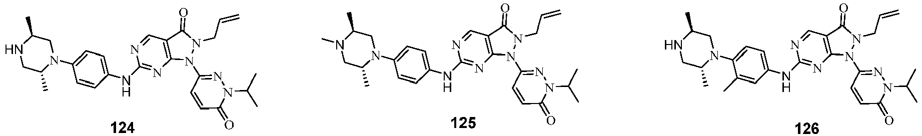

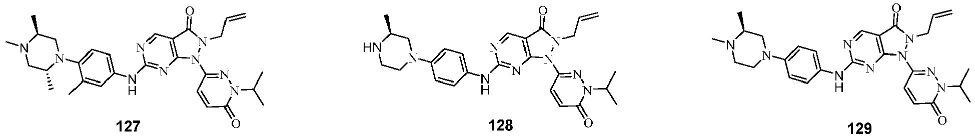

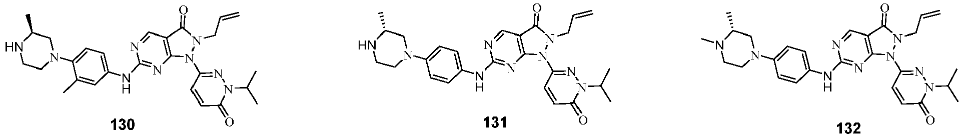

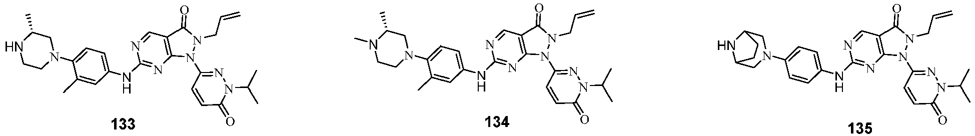

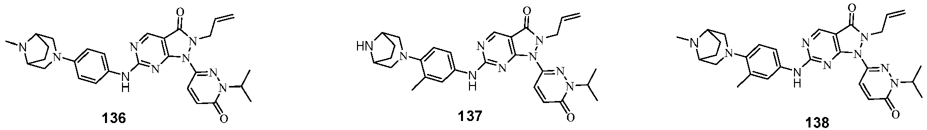

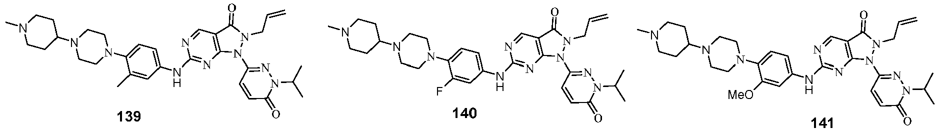

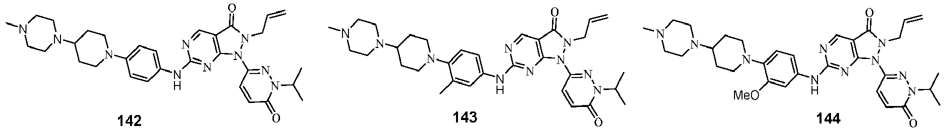

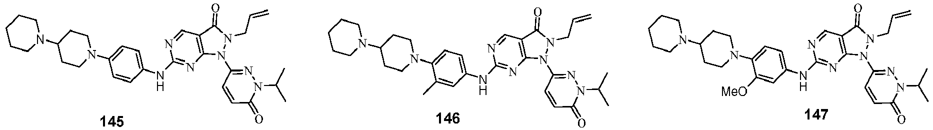

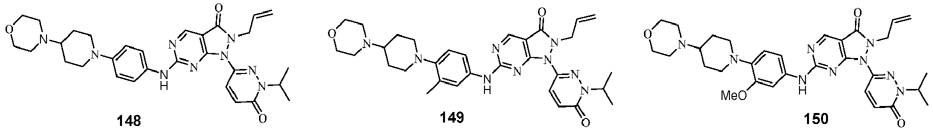

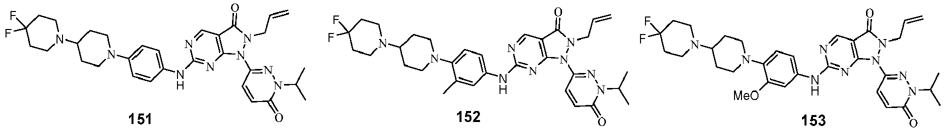

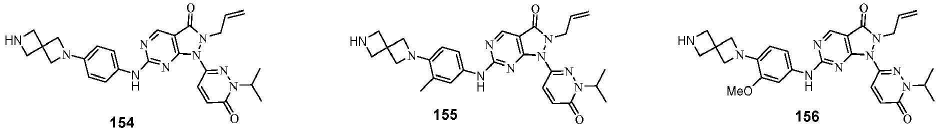

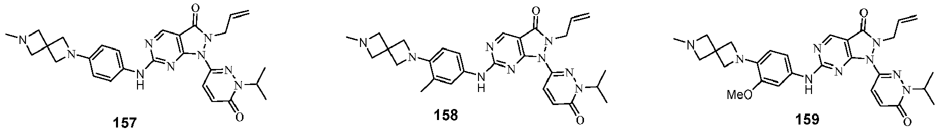

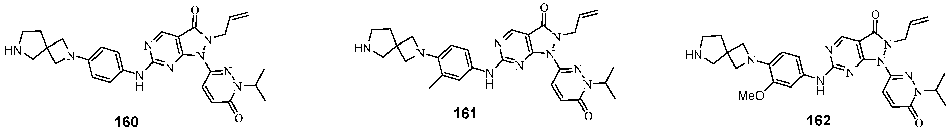

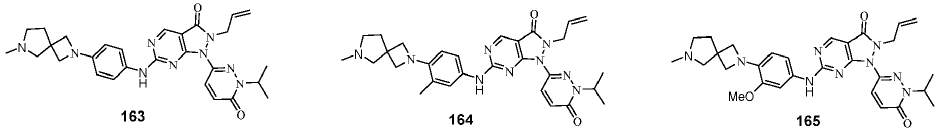

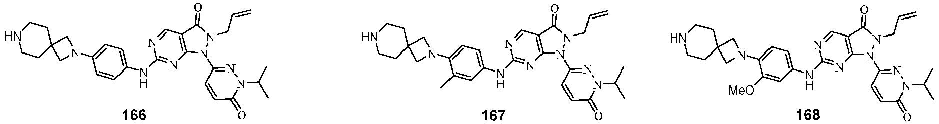

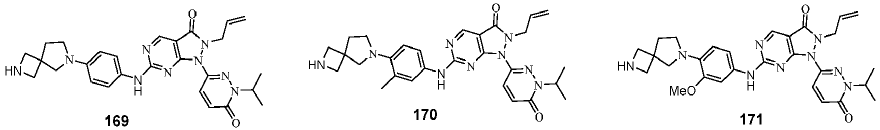

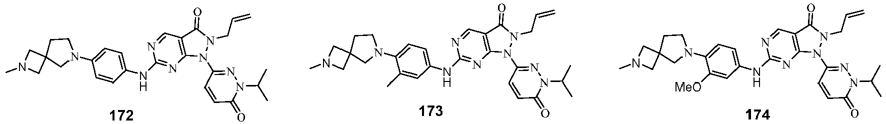

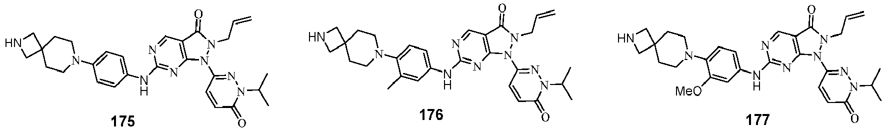

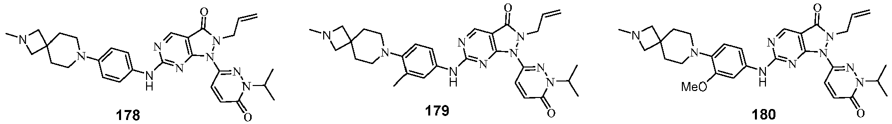

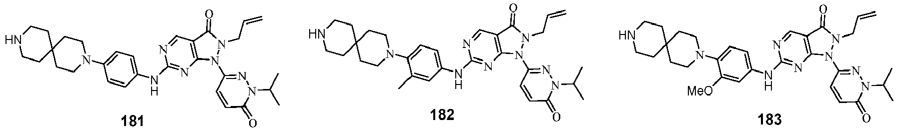

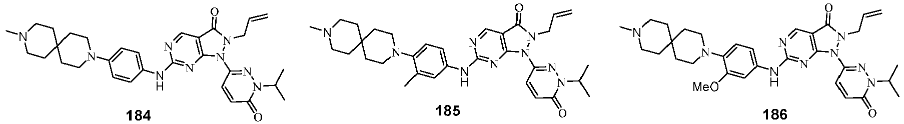

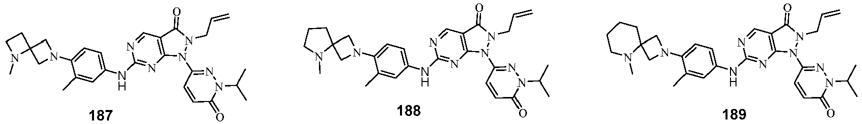

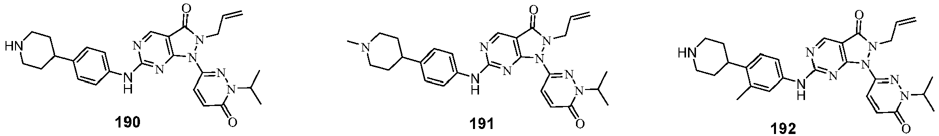

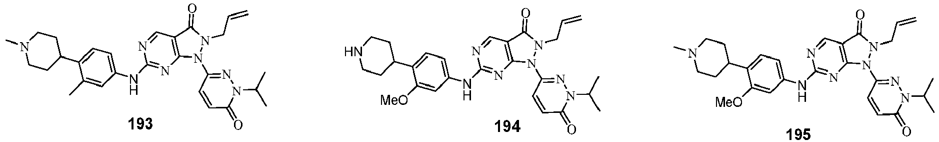

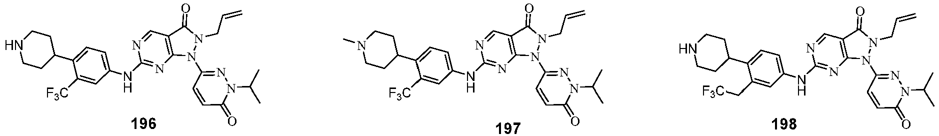

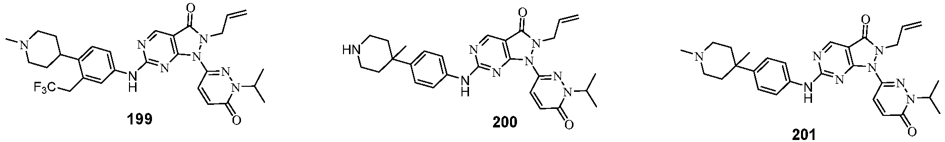

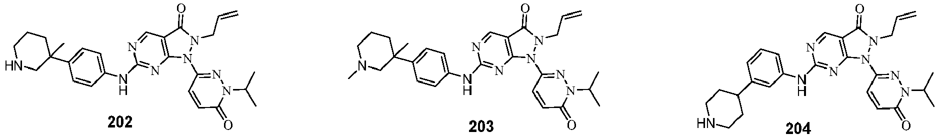

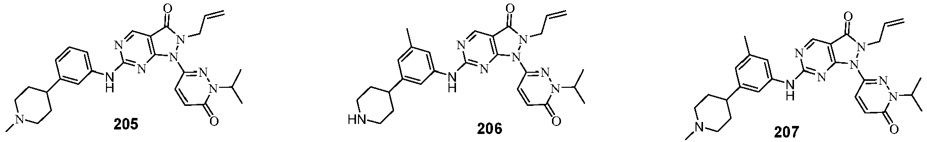

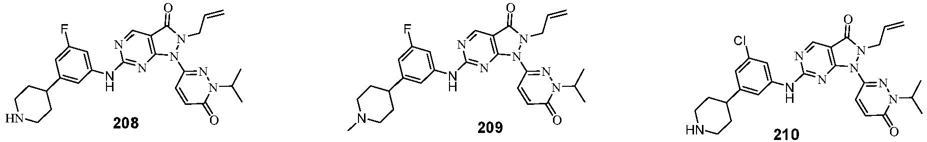

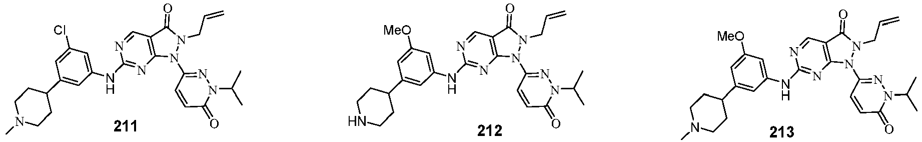

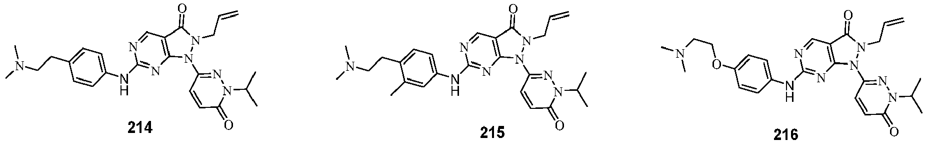

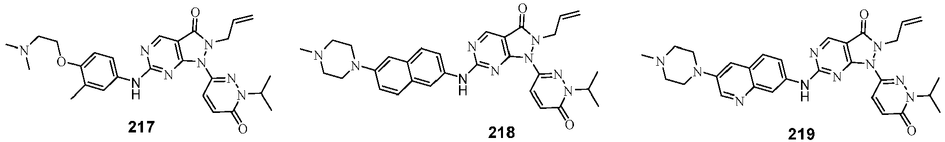

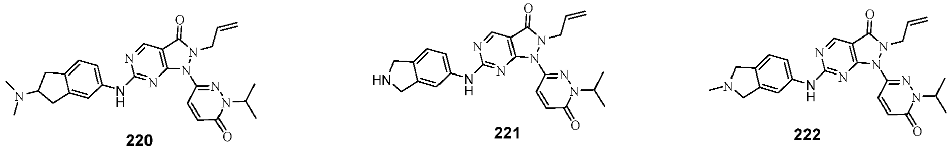

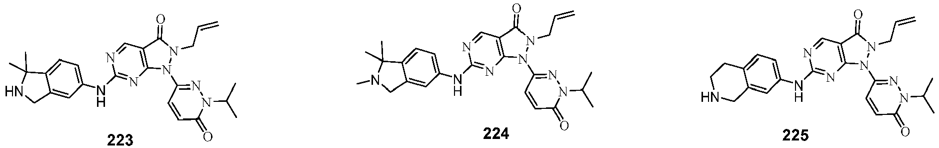

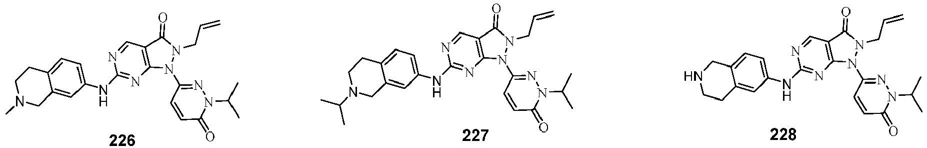

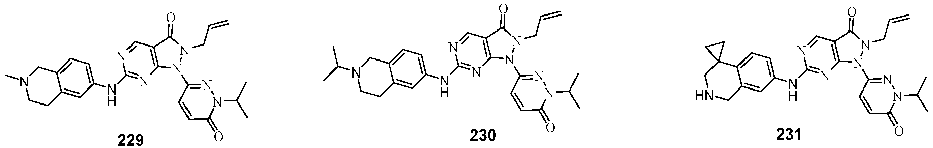

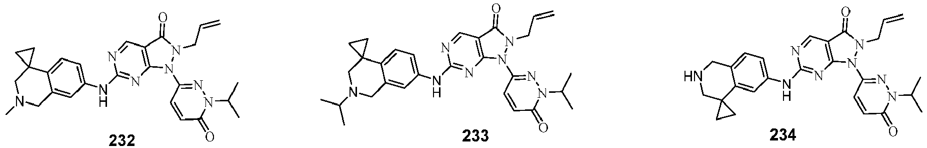

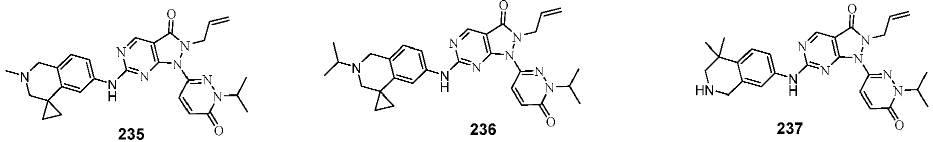

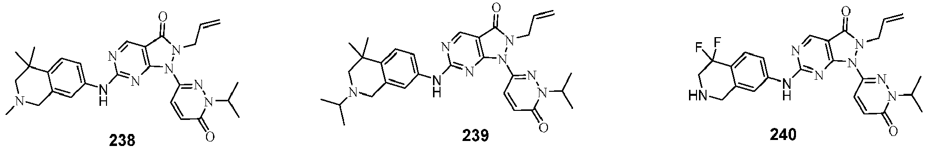

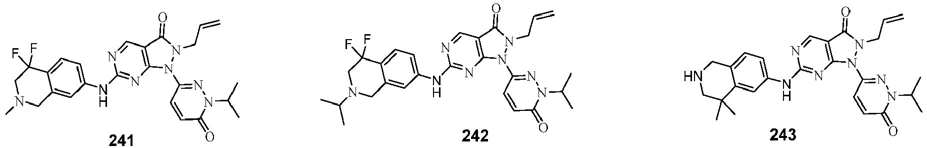

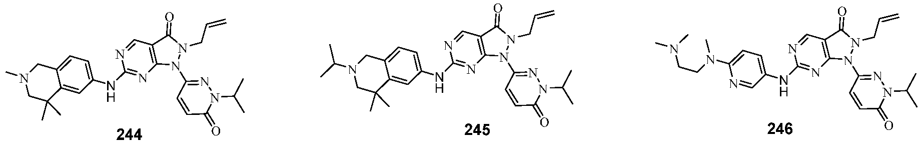

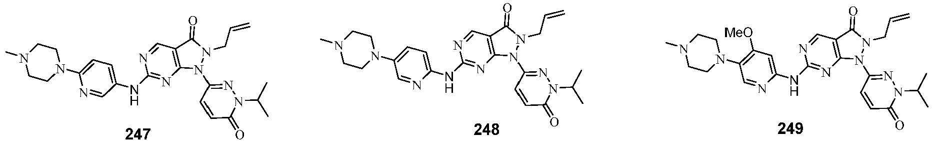

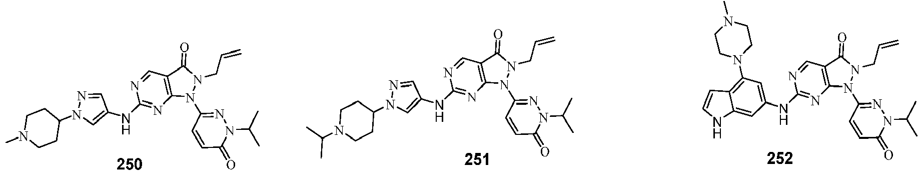

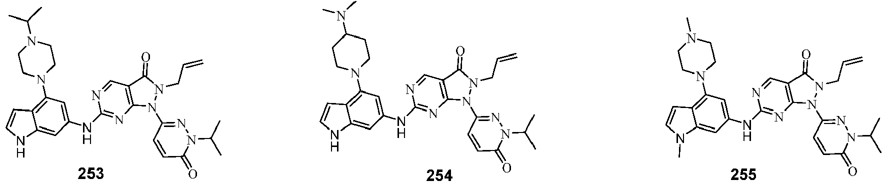

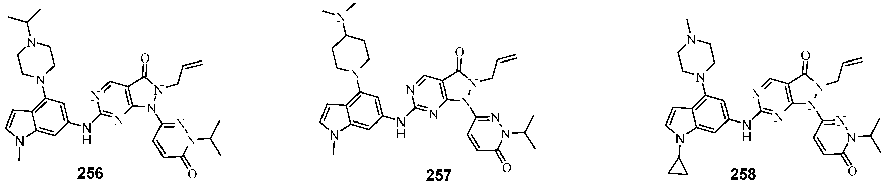

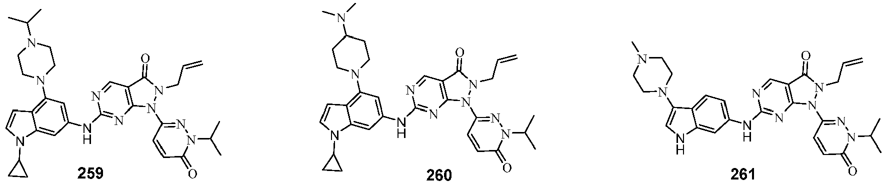

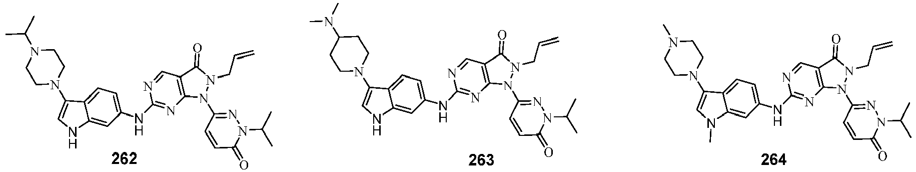

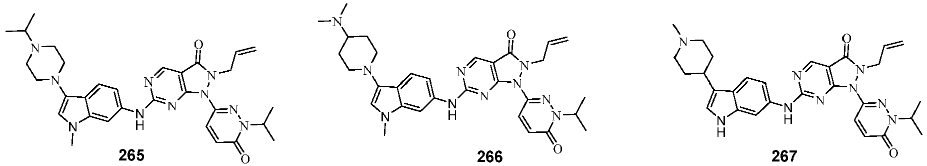

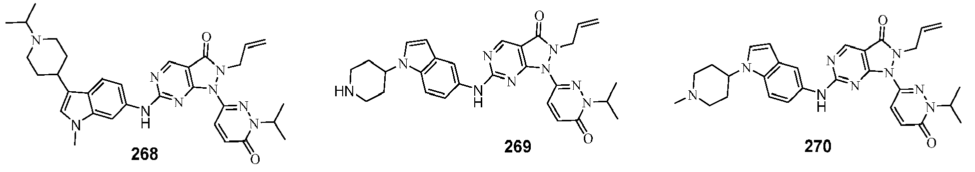

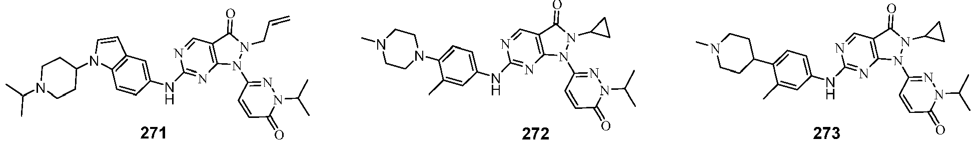

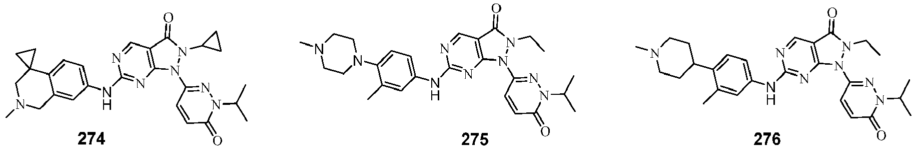

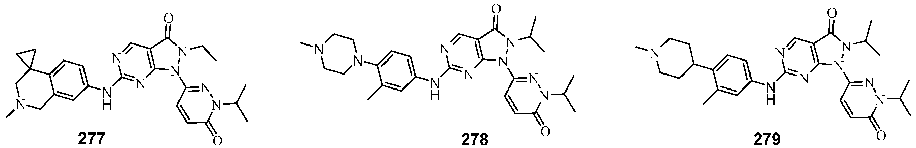

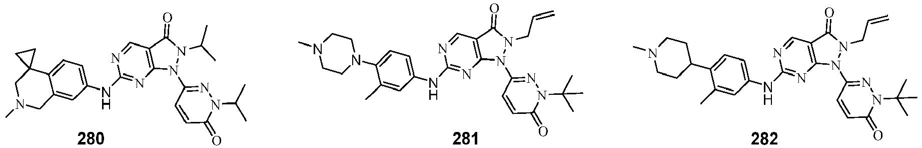

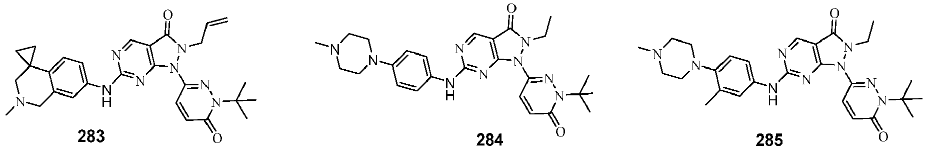

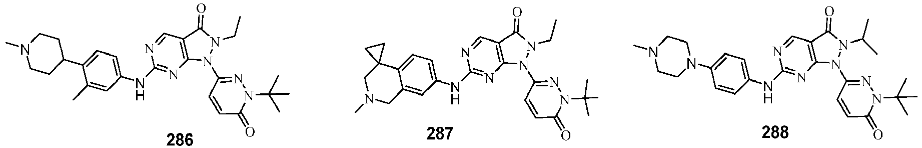

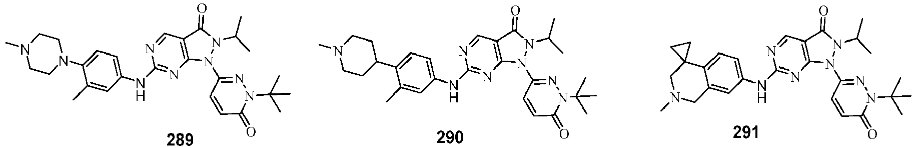

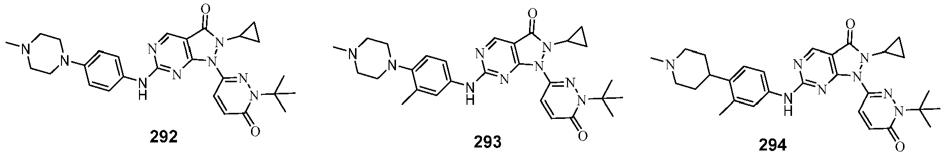

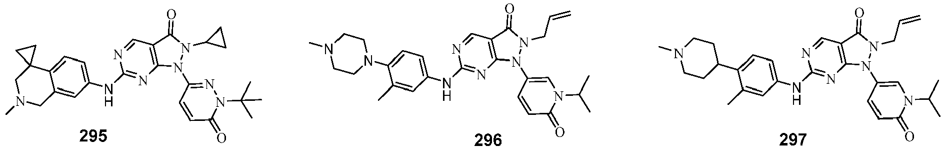

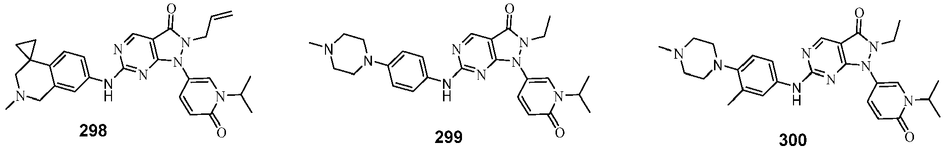

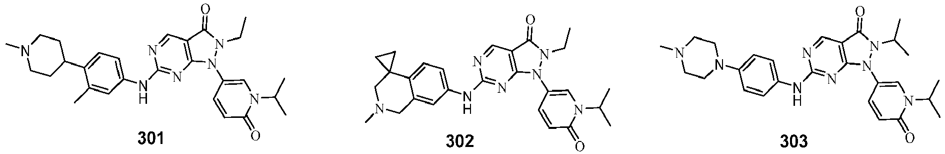

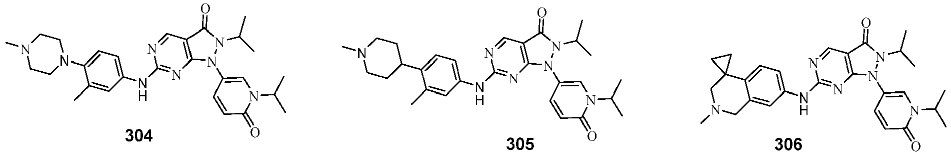

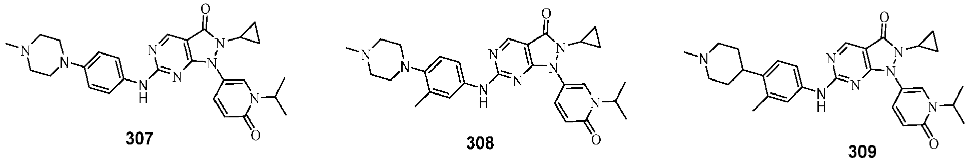

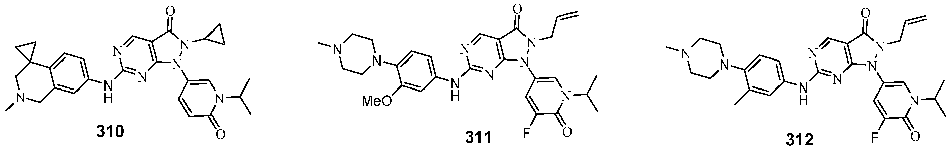

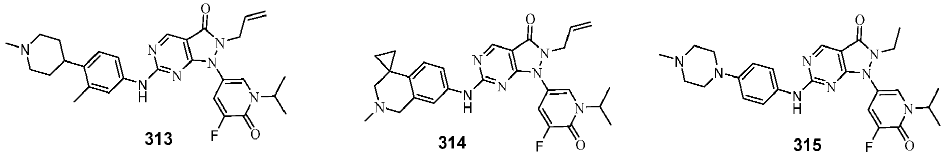

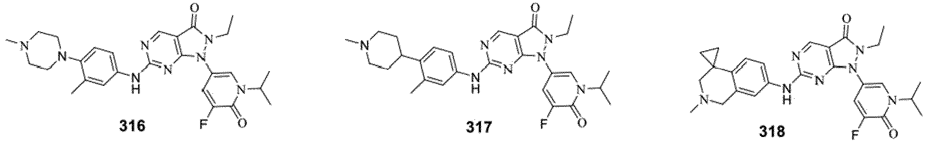

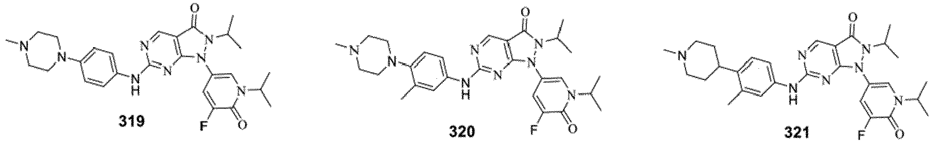

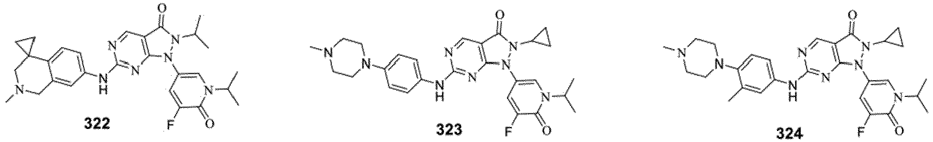

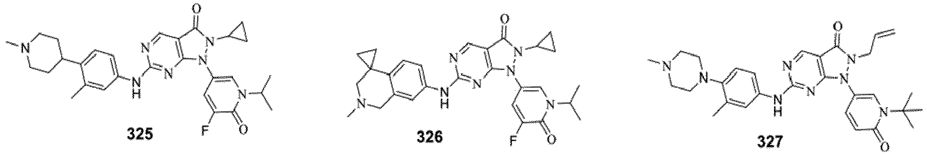

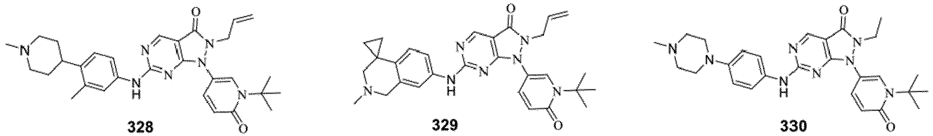

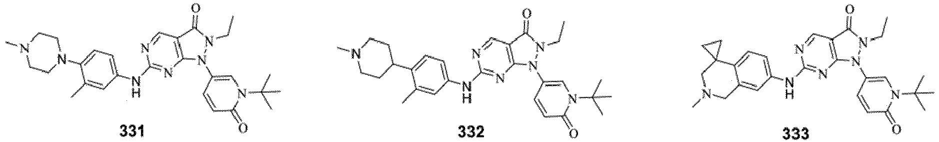

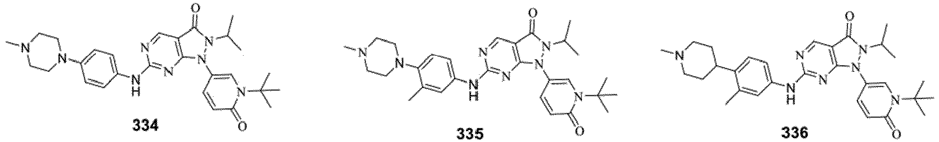

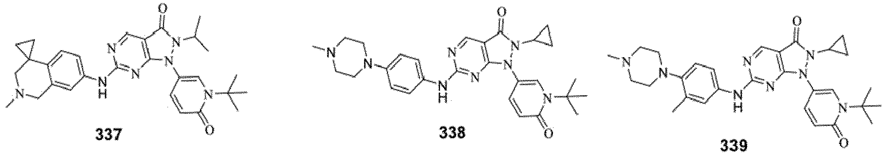

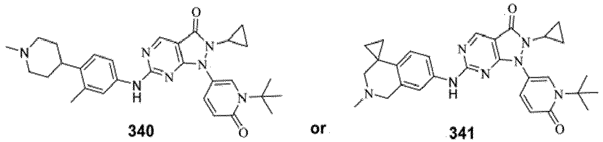

The present invention is further intended to provide a pharmaceutical composition comprising a pharmaceutically acceptable excipient or carrier, and the compound of general formula (1) or the isomers, the crystalline forms, the pharmaceutically acceptable salts, the hydrates or the solvates thereof of the present invention as an active ingredient.

The present invention is still further intended to provide use of the compound or the isomers, the crystalline forms, the pharmaceutically acceptable salts, the hydrates or the solvates thereof of the present invention in preparing a medicament for treating related diseases mediated by Wee-1.

It should be understood that both the above general description and the following detailed description of the present invention are exemplary and explanatory, and are intended to provide further explanation of the present invention claimed.

### Definitions and Explanations

Unless otherwise indicated, the following terms and phrases used herein are intended to have the following meanings. A particular term or phrase, unless otherwise specifically defined, should not be considered uncertain or unclear, but construed according to a common definition. When referring to a trade name herein, it is intended to refer to its corresponding commodity or its active ingredient. The term "pharmaceutically acceptable" is used herein for those compounds, compositions and/or formulations which are, within the scope of sound medical judgment, suitable for use in contact with the tissues of human beings and animals without excessive toxicity, irritation, allergic responses, or other problems or complications, commensurate with a reasonable benefit/risk ratio.

The term "pharmaceutically acceptable salt" refers to a form of a compound that does not cause significant irritation to the organism for drug administration or eliminate the biological activity and properties of the compound. In certain specific aspects, pharmaceutically acceptable salts are obtained by subjecting the compound of general formula (1) to a reaction with acids, e.g., inorganic acids such as hydrochloric acid, hydrobromic acid, hydrofluoric acid, sulfuric acid, phosphoric acid, nitric acid, phosphoric acid and the like, organic acids such as formic acid, acetic acid, propionic acid, oxalic acid, trifluoroacetic acid, malonic acid, succinic acid, fumaric acid, maleic acid, lactic acid, malic acid, tartaric acid, citric acid, picric acid, methanesulfonic acid, benzenesulfonic acid, *p*-toluenesulfonic acid and the like, and acidic amino acids such as aspartic acid, glutamic acid and the like.

It should be understood that references to pharmaceutically acceptable salts include solvent addition forms or crystalline forms, especially solvates or polymorphs. A solvate contains either stoichiometric or non-stoichiometric amount of solvent and is selectively formed during crystallization with pharmaceutically acceptable solvents such as water and ethanol. Hydrates are formed when the solvent is water, or alcoholates are formed when the solvent is ethanol. The solvates of the compound of general formula (1) are conveniently prepared or formed according to methods described herein. For example, the hydrates of the compound of general formula (1) are conveniently prepared by recrystallization in a mixed solvent of water/organic solvent, wherein the organic solvent used includes, but is not limited to, acetonitrile, tetrahydrofuran, ethanol or methanol. Furthermore, the compounds mentioned herein can exist in both non-solvated and solvated forms. In general, the solvated forms are considered equivalent to the non-solvated forms for purposes of the compounds and methods provided herein.

In other specific examples, the compound of general formula (1) is prepared in different forms, including but not limited to amorphous, pulverized and nanoparticle forms. In addition, the compound of general formula (1) includes crystalline forms, and may also be polymorphs. Polymorphs include different lattice arrangements of the same elements of a compound. Polymorphs usually have different X-ray diffraction patterns, infrared spectra, melting points, density, hardness, crystalline forms, optical and electrical properties, stability and solubility. Different factors such as recrystallization solvent, crystallization rate and storage temperature may lead to monocrystalline form being dominant.

In another aspect, the compound of general formula (1) has one or more stereocenters and thus occurs in the form of a racemate, racemic mixture, single enantiomer, diastereomeric compound and single diastereomer. Asymmetric centers that may be present depend on the nature of the various substituents on the molecule. Each of these asymmetric centers will independently produce two optical isomers, and all possible optical isomers, diastereomeric mixtures and pure or partially pure compounds are included within the scope of the present invention. The present invention is meant to include all such isomeric forms of these compounds.

Unless otherwise indicated, the absolute configuration of a stereocenter is represented by wedge bonds ( 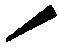 ) and dashed bonds ( 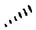 ), and wedge bonds or dashed bonds ( 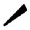 or 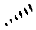 ) are represented by wavy lines ( 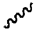 ).

The compound of the present invention may contain unnatural proportions of atomic isotopes at one or more of the atoms that constitute the compound. For example, the compound may be labeled with radioactive isotopes, such as tritium (³H), iodine-125 (¹²⁵I), or carbon-14 (¹⁴C). All isotopic variations of the compound of the present invention, whether radioactive or not, are included within the scope of the present invention.

The compound and the pharmaceutically acceptable salt thereof of the present invention can be prepared into various preparations comprising a safe and effective amount of the compound or the pharmaceutically acceptable salt thereof of the present invention, and a pharmaceutically acceptable excipient or carrier, wherein the "safe and effective amount" means that the amount of the compound is sufficient to significantly improve the condition without causing serious side effects. The safe and effective amount of the compound is determined according to the age, condition, course of treatment and other specific conditions of a treated subject.

The "pharmaceutically acceptable excipient or carrier" refers to one or more compatible solid or liquid fillers or gel substances which are suitable for human use and must be of sufficient purity and sufficiently low toxicity. Examples of pharmaceutically acceptable excipients or carriers include cellulose and its derivatives (e.g., sodium carboxymethylcellulose, sodium ethylcellulose or cellulose acetate), gelatin, talc, solid lubricants (e.g., stearic acid or magnesium stearate), calcium sulfate, vegetable oil (e.g., soybean oil, sesame oil, peanut oil or olive oil), polyols (e.g., propylene glycol, glycerol, mannitol or sorbitol), emulsifiers (e.g., Tween^{®}), wetting agents (e.g., sodium lauryl sulfate), colorants, flavoring agents, stabilizers, antioxidants, preservatives, pyrogen-free water, etc.

When the compound of the present invention is administered, it may be administered orally, rectally, parenterally (intravenously, intramuscularly or subcutaneously) or topically.

Unless otherwise specified, the term "alkyl" refers to a saturated aliphatic hydrocarbon group, including linear and branched chain groups containing 1 to 6 carbon atoms. Lower alkyls containing 1 to 4 carbon atoms, such as methyl, ethyl, propyl, 2-propyl, n-butyl, isobutyl or *tert*-butyl, are preferred. As used herein, "alkyl" includes unsubstituted and substituted alkyl, particularly alkyl substituted with one or more halogens. Preferred alkyl is selected from CH₃, CH₃CH₂, CF₃, CHF₂, CF₃CH₂, *i*-Pr, *n*-Pr, *i*-Bu, *c*-Pr, *n*-Bu and *t*-Bu.

Unless otherwise specified, "alkylene" refers to a divalent alkyl as defined above. Alkylene also includes spirocycloalkyl. Examples of alkylene include, but are not limited to, methylene, ethylene ( 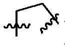 ), spirocyclopropyl ( 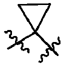 ) and spirocyclobutyl ( 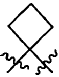 ).

Unless otherwise specified, "cycloalkyl" refers to a 3- to 14-membered all-carbon monocyclic aliphatic hydrocarbon group, wherein one or more of the rings may contain one or more double bonds, but none of them has a fully conjugated π-electron system, e.g., cyclopropyl, cyclobutyl, cyclopentyl, cyclohexane, and cyclohexadiene.

Unless otherwise specified, the term "heterocycloalkyl" refers to a saturated or partially saturated non-aromatic cyclic group consisting of carbon atoms and heteroatoms selected from nitrogen, oxygen or sulfur. The cyclic group may be monocyclic or polycyclic. In the present invention, the number of heteroatoms in the heterocycloalkyl is preferably 1, 2, 3 or 4, and the nitrogen, carbon or sulfur atom in the heterocycloalkyl may optionally be oxidized. The nitrogen atom may optionally be further substituted with other groups to form tertiary amines or quaternary ammonium salts. Examples of heterocycloalkyl include, but are not limited to: aziridinyl, azetidin-1-yl, *N*-alkylazetidin-3-yl, tetrahydrofuran-2-yl, tetrahydrofuran-3-yl, morpholin-4-yl, thiomorpholin-4-yl, thiomorpholin-*S*-oxide-4-yl, piperidin-1-yl, *N-*alkylpiperidin-4-yl, pyrrolidin-1-yl, *N*-alkylpyrrolidin-2-yl, piperazin-1-yl, 4-alkylpiperazin-1-yl and the like.

Unless otherwise specified, "alkoxy" refers to an alkyl group that bonds to the rest of the molecule through an ether oxygen atom. Representative alkoxy groups are those having 1-6 carbon atoms, such as methoxy, ethoxy, propoxy, isopropoxy, butoxy, isobutoxy, *sec*-butoxy and *tert*-butoxy. As used herein, "alkoxy" includes unsubstituted and substituted alkoxy, particularly alkoxy substituted with one or more halogens. Preferred alkoxy is selected from OCH₃, OCF₃, CHF₂O, CF₃CH₂O, *i*-PrO, *n*-PrO, *i*-BuO, *n*-BuO and *t*-BuO.

Unless otherwise specified, "aryl" refers to an aromatic hydrocarbon group, and it is monocyclic or polycyclic; for example, a monocyclic aryl ring may be fused with one or more carbocyclic aromatic groups. Examples of aryl include, but are not limited to, phenyl, naphthyl, and phenanthryl.

Unless otherwise specified, "heteroaryl" refers to an aromatic group containing one or more heteroatoms (O, S or N), and it is monocyclic or polycyclic; for example, a monocyclic heteroaryl ring may be fused with one or more carbocyclic aromatic groups or other monocyclic heterocyclyl groups. Examples of heteroaryl include, but are not limited to, pyridyl, pyridazinyl, imidazolyl, pyrimidinyl, pyrazolyl, triazolyl, pyrazinyl, quinolinyl, isoquinolinyl, furyl, thienyl, isoxazolyl, thiazolyl, oxazolyl, isothiazolyl, pyrrolyl, indolyl, benzimidazolyl, benzofuryl, benzothiazolyl, benzothienyl, benzoxazolyl, benzopyridyl, and pyrrolopyrimidinyl.

Unless otherwise specified, "alkenyl" refers to an unsaturated aliphatic hydrocarbon group containing carbon-carbon double bonds, including linear or branched groups containing 1 to 14 carbon atoms. Lower alkenyls containing 1 to 4 carbon atoms, such as vinyl, 1-propenyl, 1-butenyl or 2-methylpropenyl, are preferred.

Unless otherwise specified, "alkynyl" refers to an unsaturated aliphatic hydrocarbon group containing carbon-carbon triple bonds, including linear and branched groups containing 1 to 14 carbon atoms. Lower alkenyls containing 1 to 4 carbon atoms, such as ethynyl, 1-propynyl or 1-butynyl, are preferred.

Unless otherwise specified, the term "halogen substituted" or "halogen" by itself or as part of another substituent refers to a fluorine, chlorine, bromine or iodine atom. Further, "haloalkyl" is intended to include monohaloalkyl or polyhaloalkyl. For example, "halogenated C1-C3 alkyl" is intended to include, but is not limited to, trifluoromethyl, 2, 2, 2-trifluoroethyl, 2-chloropropyl, 3-bromopropyl, and the like.

The term "membered ring" includes any cyclic structure. The term "membered" is intended to refer to the number of backbone atoms that form a ring. For example, cyclohexyl, pyridyl, pyranyl and thiopyranyl are six-membered rings, and cyclopentyl, pyrrolyl, furanyl and thienyl are five-membered rings.

The term "moiety" refers to a specific portion or functional group of a molecule. Chemical moiety is generally considered to be a chemical entity contained in or attached to a molecule. "Optional" or "optionally" means that the subsequently described event or circumstance may, but does not necessarily, occur, and the description includes instances where the event or circumstance occurs and instances where it does not.

### Synthesis of the Compounds

Methods for preparing the compounds of general formulas (1) of the present invention are hereafter described in detail, but these specific methods do not limit the present invention in any way.

The compounds of general formulas (1) described above may be synthesized using standard synthetic techniques or well-known techniques in combination with the methods described herein. In addition, solvents, temperatures and other reaction conditions mentioned herein may vary. Starting materials for the synthesis of the compounds may be obtained synthetically or commercially. The compounds described herein and other related compounds having different substituents may be synthesized using well-known techniques and starting materials, including the methods found in March, ADVANCED ORGANIC CHEMISTRY, 4th Ed., (Wiley 1992); Carey and Sundberg, ADVANCED ORGANIC CHEMISTRY, 4th Ed., Vols. A and B (Plenum 2000, 2001), and Green and Wuts, PROTECTIVE GROUPS IN ORGANIC SYNTHESIS, 3rd Ed., (Wiley 1999). General methods for preparing a compound can be changed by using appropriate reagents and conditions for introducing different groups into the formulas provided herein.

In one aspect, the compounds described herein are prepared according to methods well known in the art. However, the conditions involved in the methods, such as reactants, solvent, base, amount of the compound used, reaction temperature and time required for the reaction are not limited to the following explanation. The compounds of the present invention can also be conveniently prepared by optionally combining various synthetic methods described herein or known in the art, and such combinations can be easily determined by those skilled in the art to which the present invention pertains. In one aspect, the present invention also provides a method for preparing the compounds of general formulas (1), which are prepared using Method A below:
Method A comprises the following steps: firstly, subjecting a compound A1 to a reaction with R²-Y to generate a compound A2; subjecting the compound A2 to a coupling reaction with a compound A3 to generate a compound A4; further subjecting the compound A4 to a reaction with a compound A5 to generate a target compound A6; and when 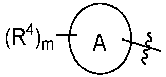 contains protective groups of primary amine and secondary amine, further removal of the protective groups is required to obtain the target compound.

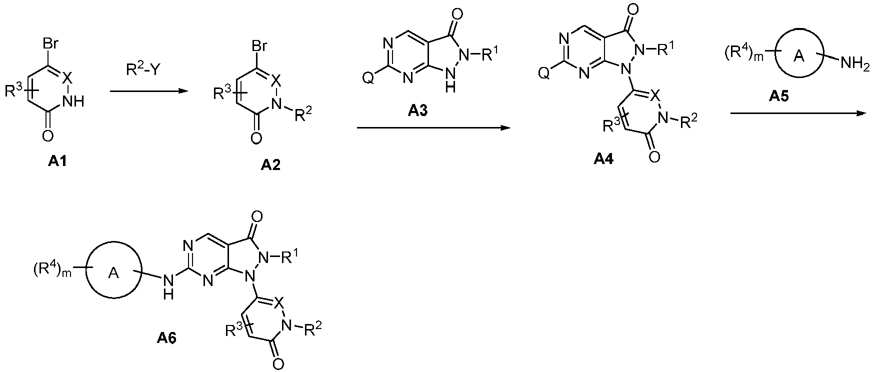

In the above reaction equation, A, R¹, R², R³, R⁴ and m are as defined above, Y is OH, Br or I, and Q is CH₃S, CH₃SO, CH₃SO₂, Br, Cl, I or the like.

### Therapeutic Use

The compounds or compositions described herein are generally useful for inhibiting Wee-1 kinase, and thus may be useful for treating one or more disorders related to Wee-1 kinase activity. Therefore, in certain embodiments, the present invention provides a method for treating a Wee-1 kinase-mediated disorder, which comprises the step of administering to a patient in need thereof the compound of the present invention or a pharmaceutically acceptable composition thereof.

Cancers that can be treated with the compound of the present invention include, but are not limited to, hematological malignancies (leukemias, lymphomas, myelomas including multiple myeloma, myelodysplastic syndrome and myeloproliferative syndrome), solid tumors (carcinomas such as prostate, breast, lung, colon, pancreas, kidney, ovary and soft tissue carcinomas, osteosarcoma and interstitial tumors), and the like.

### DETAILED DESCRIPTION

Various specific aspects, features and advantages of the compounds, methods and pharmaceutical compositions described above will be set forth in detail in the following description, which will make the content of the present invention very clear. It should be understood that the detailed description and examples below describe specific embodiments for reference only. After reading the description of the present invention, those skilled in the art can make various changes or modifications to the present invention, and such equivalents also fall within the scope of the present invention defined herein.

In all examples, melting points were measured using an X-4 melting point apparatus with the thermometer uncalibrated; ¹H-NMR spectra were recorded with a Varian Mercury 400 nuclear magnetic resonance spectrometer, and chemical shifts are expressed in δ (ppm); silica gel for separation was 200-300 mesh silica gel if not specified, and the ratio of the eluents was volume ratio.

In the present invention, the following abbreviations are used: CDCl₃ represents deuterated chloroform; CuI represents cuprous iodide; DCM represents dichloromethane; DIPEA represents diisopropylethylamine; DMF represents dimethylformamide; EA represents ethyl acetate; h represents hour; K₂CO₃ represents potassium carbonate; LC-MS represents liquid chromatography-mass spectrometry; m-CPBA represents m-chloroperoxybenzoic acid; MeI (CH₃I) represents methyl iodide; mL represents milliliter; MeOH represents methanol; min represents minute; MS represents mass spectrum; NaHCO₃ represents sodium bicarbonate; Na₂SO₄ represents sodium sulfate; NMR represents nuclear magnetic resonance; °C represents degree Celsius; PE represents petroleum ether; r.t. represents room temperature; TFA represents trifluoroacetic acid; and toluene represents methylbenzene.

### Preparation Example 1. Preparation of 2-allyl-1-(1-methyl-6-oxo-1,6-dihydropyridazin-3-yl)-6-(methylthio)-1,2-dihydro-3H-pyrazolo[3,4-d]pyrimidin-3-one (Intermediate B1)

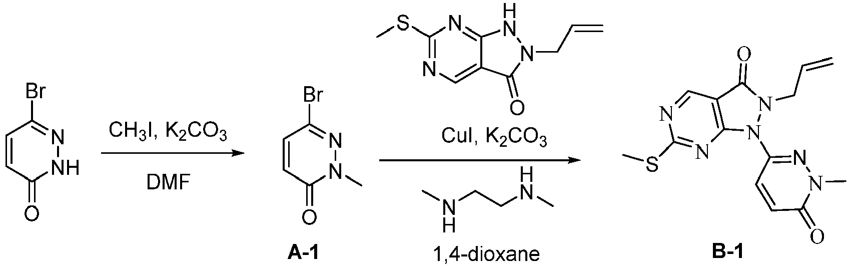

### Step 1: synthesis of compound A-1

In a 50 mL reaction flask, 6-bromo-3-pyridazinol (826 mg, 4.72 mmol) and K₂CO₃ (1.3 g, 9.44 mmol) were added to DMF (10 mL), followed by MeI (0.6 mL, 9.44 mmol). The mixture was stirred at r.t. The reaction was monitored by TLC (PE/EA = 1/1). After completion of the reaction, water (50 mL) was added to quench the reaction. The mixture was extracted with EA (50 mL×2) to obtain an organic phase. The organic phase was washed with saturated brine (30 mL), dried over anhydrous Na₂SO₄, distilled under reduced pressure to remove EA, and added with cold hydrazine to remove DMF. The residue was purified by column chromatography (PE/EA = 3/1) to give compound **A-1** (705 mg, 79% yield). ESI-MS m/z: 189 [M+H]⁺.

### Step 2: synthesis of compound B-1

In a 50 mLreaction flask, compound **A-1** (621 mg, 3.55 mmol) and 2-propenyl-6-(methylthio)-1,2-dihydro-3*H*-pyrazolo[3,4-*d*]pyrimidin-3-one (607 mg, 2.73 mmol, refer to patent US2019106427 for synthesis) were added and dissolved in dioxane (20 mL), followed by the addition of CuI (520 mg, 2.73 mmol) and K₂CO₃ (528 mg, 2.73 mmol). The mixture was heated to 80 °C under nitrogen atmosphere. *N*,*N'*-dimethylethylenediamine (0.59 mL, 5.46 mmol) was added. The reaction system was heated to 95 °C and stirred. The reaction was monitored by TLC (PE/EA = 1/1). After completion of the reaction, the reaction flask was cooled to room temperature. The mixture was distilled under reduced pressure and extracted with EA (50 mL×2) to obtain an organic phase. The organic phase was washed with saturated brine (50 mL), dried over anhydrous Na₂SO₄, and distilled under reduced pressure. The residue was purified by column chromatography (PE/EA = 2/1) to give compound **B-1** (309 mg, yield 34%). ESI-MS m/z: 333 [M+H]⁺.

By the procedures similar to those in the synthesis of compound **B-1,** the following intermediates **B2-B45** were obtained:

**Table 1. Structural formulas of intermediates B2-B45**

| **Interme diate** | **Compound structure** | **MS (M+H)⁺** | **Interme diate** | **Compound structure** | **MS (M+H)⁺** |
|---|---|---|---|---|---|
| **B-2** | 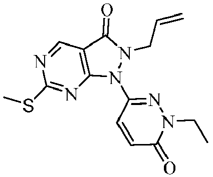 | 345 | **B-3** | 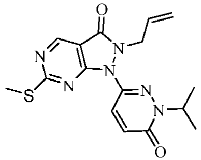 | 359 |
| **B-4** | 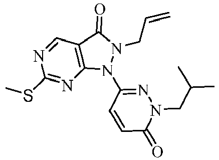 | 373 | **B-5** | 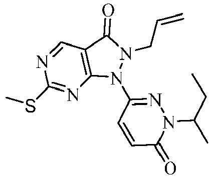 | 373 |
| **B-6** | 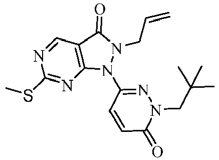 | 387 | **B-7** | 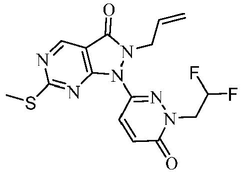 | 381 |
| **B-8** | 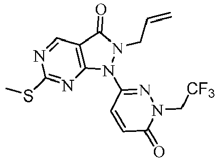 | 399 | **B-9** | 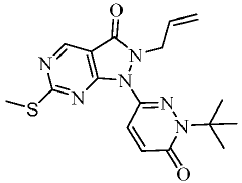 | 373 |
| **B-10** | 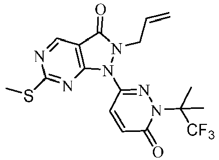 | 427 | **B-11** | 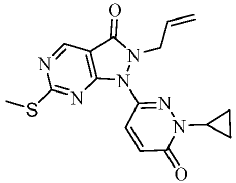 | 357 |
| **B-12** | 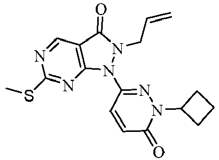 | 371 | **B-13** | 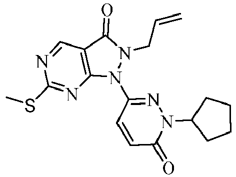 | 385 |
| **B-14** | 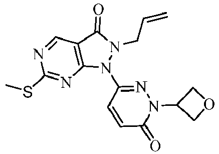 | 373 | **B-15** | 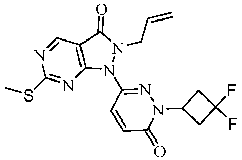 | 407 |
| **B-16** | 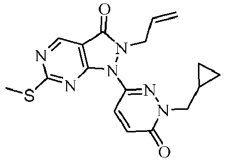 | 371 | **B-17** | 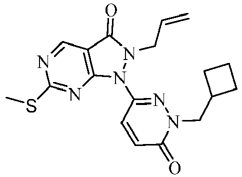 | 385 |
| **B-18** | 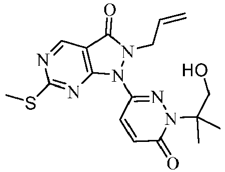 | 389 | **B-19** | 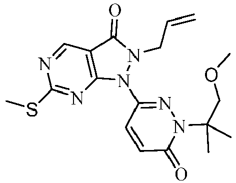 | 403 |
| **B-20** | 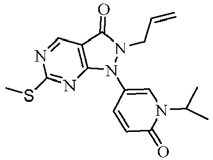 | 358 | **B-21** | 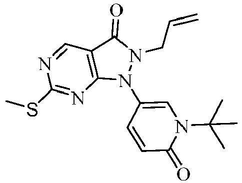 | 372 |
| **B-22** | 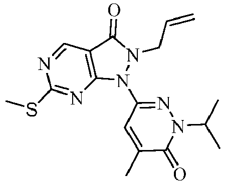 | 373 | **B-23** | 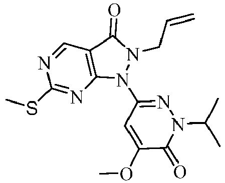 | 389 |
| **B-24** | 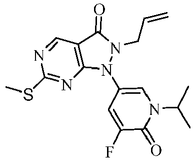 | 376 | **B-25** | 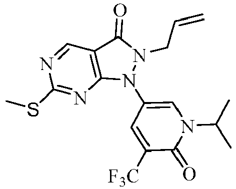 | 426 |
| **B-26** | 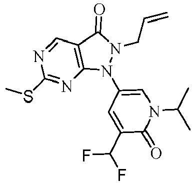 | 408 | **B-27** | 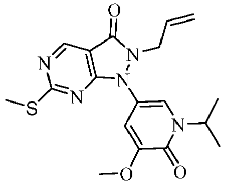 | 388 |
| **B-28** | 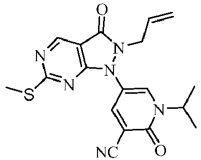 | 383 | **B-29** | 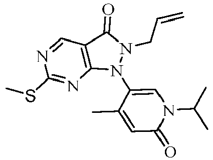 | 372 |
| **B-30** | 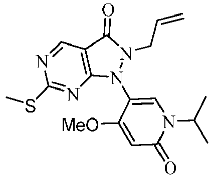 | 388 | **B-31** | 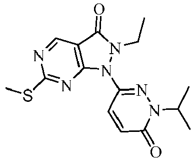 | 347 |
| **B-32** | 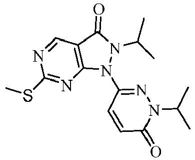 | 361 | **B-33** | 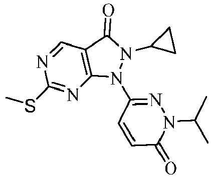 | 359 |
| **B-34** | 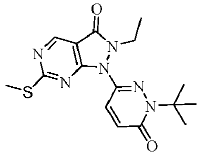 | 361 | **B-35** | 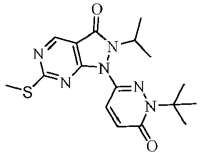 | 375 |
| **B-36** | 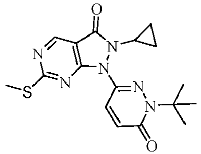 | 373 | **B-37** | 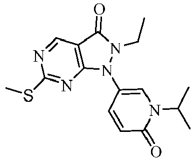 | 346 |
| **B-38** | 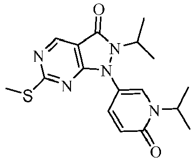 | 360 | **B-39** | 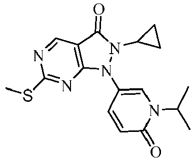 | 358 |
| **B-40** | 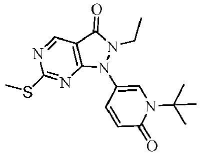 | 360 | **B-41** | 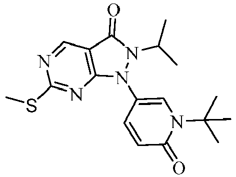 | 374 |
| **B-42** | 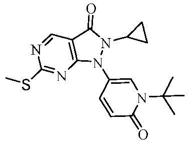 | 372 | **B-43** | 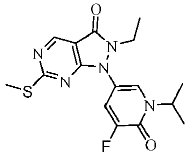 | 364 |
| **B-44** | 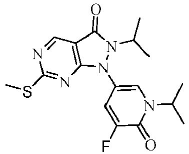 | 378 | **B-45** | 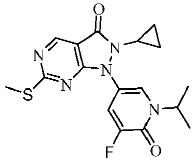 | 376 |

### Example 1. Synthesis of 2-allyl-1-(1-methyl-6-oxo-1,6-dihydropyridazin-3-yl)-6-((4-(4-methylpiperazin-1-yl)phenyl)amino)-1,2-dihydro-3H-pyrazolo[3,4-d]pyrimidin-3-one (Compound 1)

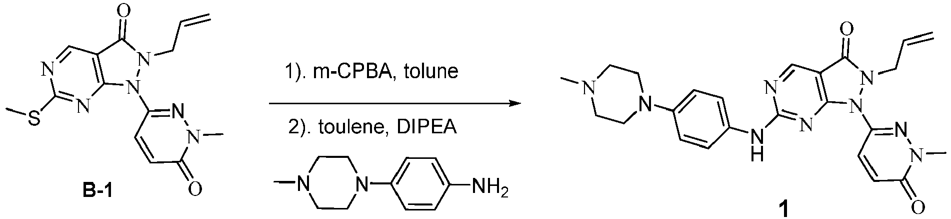

In a 50 mL reaction flask, compound **B-1** (100 mg, 0.3 mmol) was dissolved in toluene (10 mL), followed by addition of m-CPBA (76 mg, 0.33 mmol). The mixture was stirred at r.t. for 1 h. DIPEA (0.2 mL, 1.58 mmol) and 4-(4-methylpiperazine)aniline (74.6 mg, 0.39 mmol) were added. The mixture was stirred at room temperature for 3 h. The reaction was monitored by TLC (DCM/MeOH = 10/1). After completion of the reaction, the mixture was extracted with EA (30 mL×2) to obtain an organic phase. The organic phase was washed with saturated brine (30 mL), dried over anhydrous Na₂SO₄, and distilled under reduced pressure. The residue was purified by column chromatography (DCM/MeOH = 100/1) to give compound **1** (75 mg, yield 54%).

¹H NMR (400 MHz, CDCl₃) δ 8.80 (s, 1H), 7.99 (s, 1H), 7.87 (d, *J* = 9.8 Hz, 1H), 7.43-7.31 (m, 2H), 7.03 (d, *J* = 9.9 Hz, 1H), 6.96-6.84 (m, 2H), 5.68 (ddt, *J* = 16.6, 10.1, 6.3 Hz, 1H), 5.15-4.95 (m, 2H), 4.60 (d, *J* = 6.3 Hz, 2H), 3.79 (s, 3H), 3.25-3.13 (m, 4H), 2.64-2.56 (m, 4H), 2.36 (s, 3H); ESI-MS m/z: 474 [M+H]⁺.

### Examples 2-30. Synthesis of Compounds 2-30

By the procedures similar to those in the synthesis of compound **1,** the target compounds **2-30** in Table 2 can be obtained with **B2-B30** as starting materials.

**Table 2. Structures of compounds 2-30**

| **Compound** | **Compound structure** | **MS (M+H)** + | **Compound** | **Compound structure** | **MS (M+H)** + |
|---|---|---|---|---|---|
| **2** | 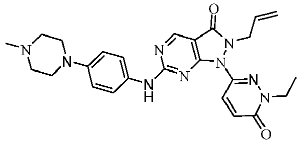 | 488 | **3** | 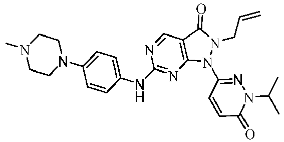 | 571 |
| **4** | 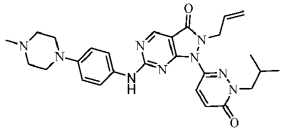 | 516 | **5** | 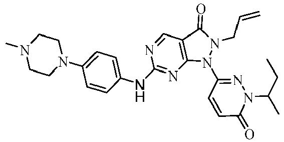 | 516 |
| **6** | 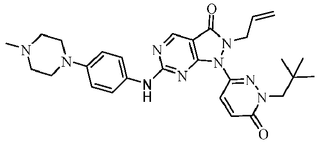 | 530 | **7** | 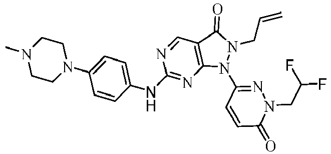 | 524 |
| **8** | 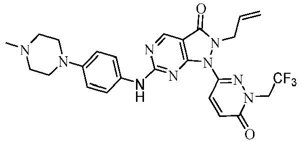 | 542 | **9** | 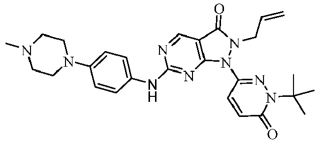 | 516 |
| **10** | 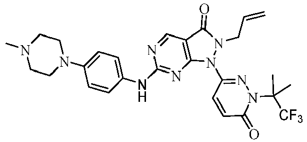 | 570 | **11** | 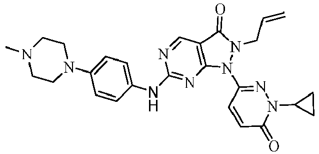 | 500 |
| **12** | 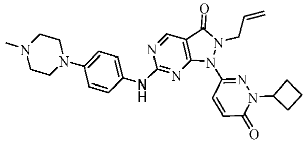 | 514 | **13** | 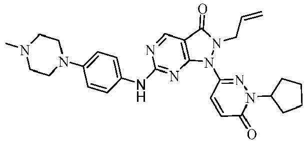 | 528 |
| **14** | 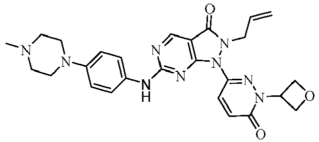 | 516 | **15** | 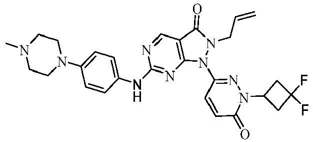 | 550 |
| **16** | 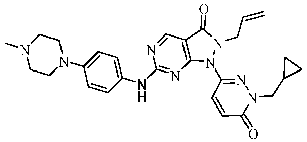 | 514 | **17** | 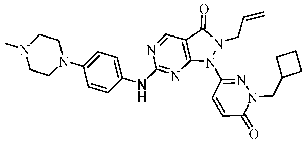 | 528 |
| **18** | 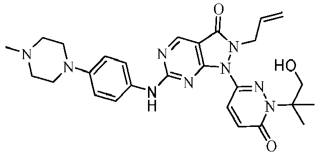 | 532 | **19** | 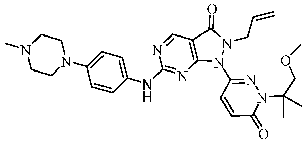 | 546 |
| **20** | 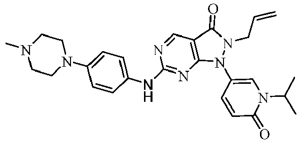 | 501 | **21** | 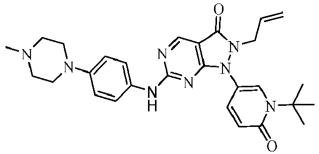 | 515 |
| **22** | 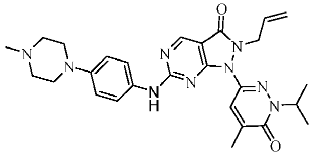 | 516 | **23** | 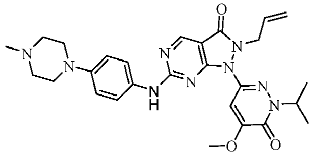 | 532 |
| **24** | 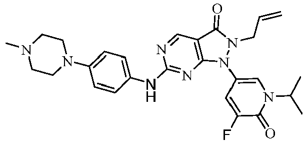 | 519 | **25** | 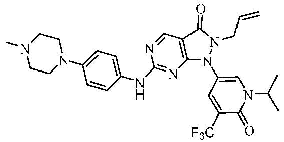 | 569 |
| **26** | 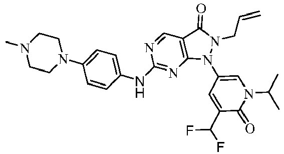 | 551 | **27** | 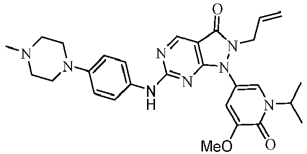 | 531 |
| **28** | 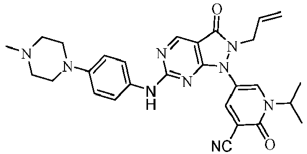 | 526 | **29** | 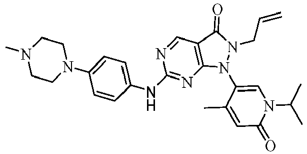 | 515 |
| **30** | 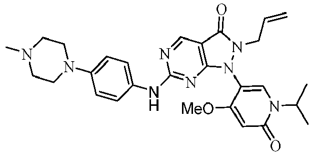 | 531 | | | |

### Example 31

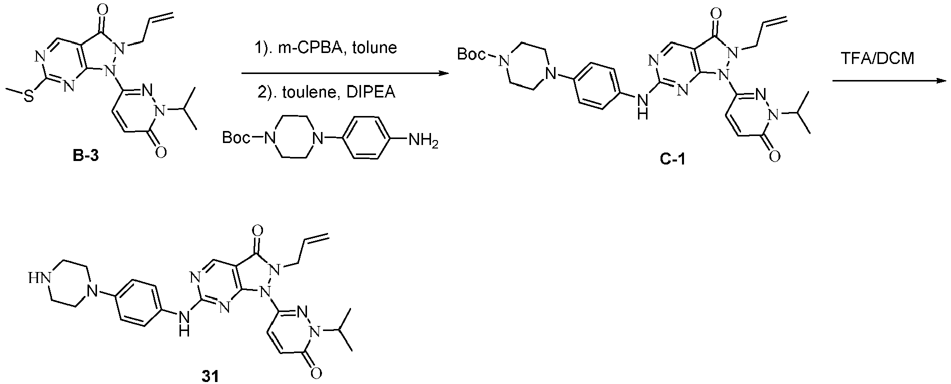

### Step 1: synthesis of compound C-1

In a 50 mL reaction flask, compound **B-3** (107 mg, 0.3 mmol) was dissolved in toluene (10 mL), followed by addition of M-CPBA (76 mg, 0.33 mmol). The mixture was stirred at r.t. for 1 h. DIPEA (0.2 mL, 1.58 mmol) and *tert*-butyl 4-(4-aminobenzene)piperazine-1-carboxylate (100 mg, 0.36 mmol) were added. The mixture was stirred at r.t. for 3 h. The reaction was monitored by TLC (DCM/MeOH = 20/1). After completion of the reaction, the mixture was extracted with EA (30 mL×2) to obtain an organic phase. The organic phase was washed with saturated brine (30 mL), dried over anhydrous Na₂SO₄, and distilled under reduced pressure. The residue was purified by column chromatography (DCM/MeOH = 100/1) to give compound **C1** (135 mg, 77% yield). ESI-MS m/z: 588 [M+H]⁺.

### Step 1: synthesis of compound 31

In a 20 mL reaction flask, compound **C-1** (117 mg, 0.2 mmol) was dissolved in DCM (5 mL), followed by the addition of TFA (1 mL) while cooling in an ice salt bath. After addition, the mixture was stirred at r.t. for 3 h. The reaction was monitored by TLC (DCM/MeOH = 20/1). After completion of the reaction, the mixture was diluted with DCM (50 mL), adjusted to alkalinity with a saturated NaHCOs solution, followed by liquid separation. The organic phase was washed with saturated brine (30 mL), dried over anhydrous Na₂SO₄, and distilled under reduced pressure. The residue was purified by column chromatography (DCM/MeOH = 50/1) to give compound **31** (58 mg, 59% yield).

¹H NMR (400 MHz, CDCl₃) δ: 8.80 (s, 1H), 7.99 (s, 1H), 7.84 (d, *J* = 9.8 Hz, 1H), 7.43-7.34 (m, 2H), 7.00 (d, *J* = 9.8 Hz, 1H), 6.94-6.87 (m, 2H), 5.66 (ddt, *J* = 16.7, 10.1, 6.4 Hz, 1H), 5.40-5.28 (m, 1H), 5.08 (dd, *J* = 10.1, 1.3 Hz, 1H), 5.00 (dd, *J* = 17.1, 1.4 Hz, 1H), 4.66 (d, *J* = 6.4 Hz, 2H), 3.25-3.14 (m, 4H), 2.60 (t, *J* = 5.0 Hz, 4H), 1.38 (d, *J* = 6.7 Hz, 6H); ESI-MS m/z: 488 [M+H]⁺.

### Examples 32-341: Synthesis of Compounds 32-341

By the procedures similar to those in the synthesis of compounds **1** and **31,** the target compounds **32-341** in Table 3 can be obtained with different intermediates in Table 1 as starting materials.

**Table 3. Structures of compounds 32-341**

| **Compound** | **Compound structure** | **MS (M+H)⁺** | **Compound** | **Compound structure** | **MS (M+H)⁺** |
|---|---|---|---|---|---|
| **32** | 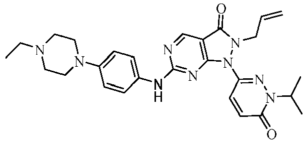 | 516 | **33** | 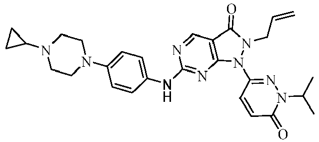 | 528 |
| **34** | 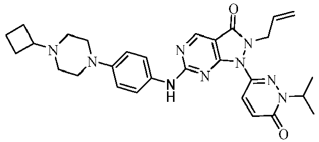 | 542 | **35** | 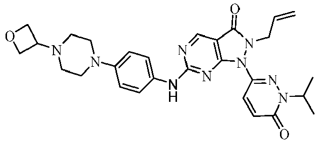 | 544 |
| **36** | 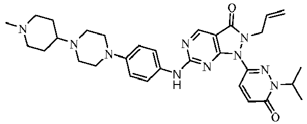 | 585 | **37** | 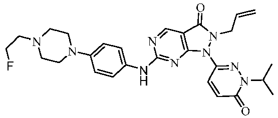 | 534 |
| **38** | 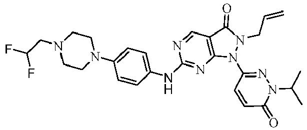 | 552 | **39** | 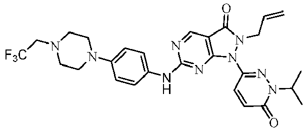 | 570 |
| **40** | 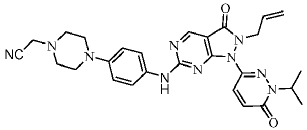 | 527 | **41** | 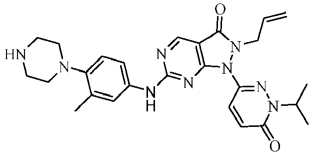 | 502 |
| **42** | 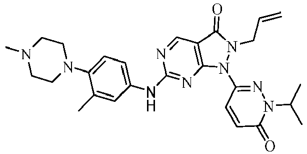 | 516 | **43** | 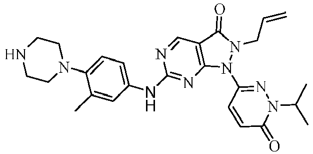 | 502 |
| **44** | 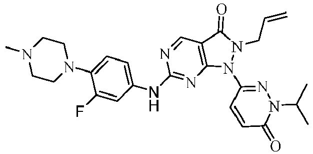 | 520 | **45** | 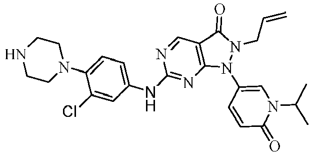 | 521 |
| **46** | 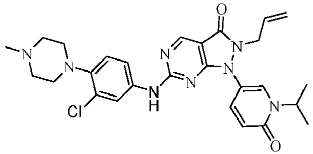 | 535 | **47** | 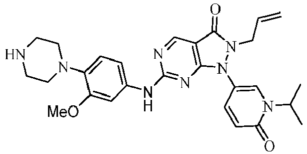 | 517 |
| **48** | 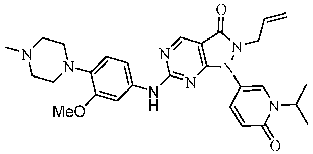 | 531 | **49** | 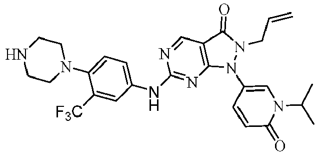 | 555 |
| **50** | 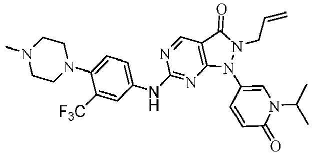 | 569 | **51** | 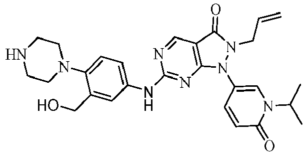 | 517 |
| **52** | 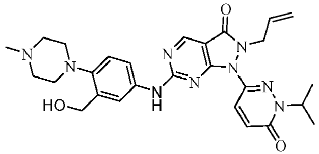 | 532 | **53** | 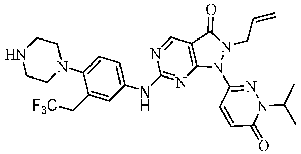 | 570 |
| **54** | 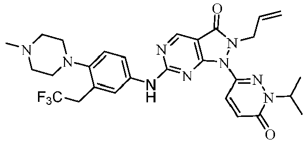 | 584 | **55** | 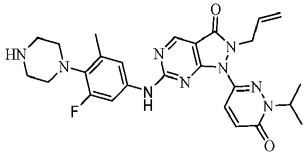 | 520 |
| **56** | 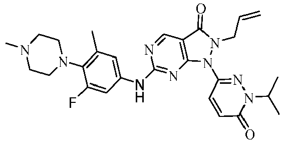 | 534 | **57** | 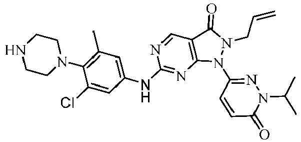 | 536 |
| **58** | 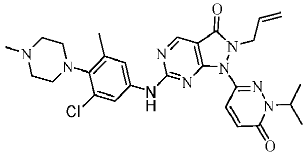 | 550 | **59** | 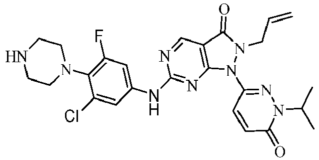 | 540 |
| **60** | 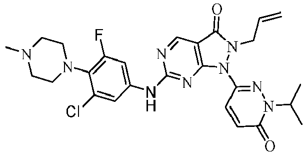 | 554 | **61** | 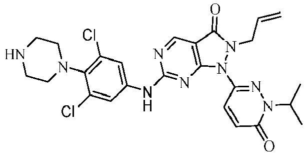 | 556 |
| **62** | 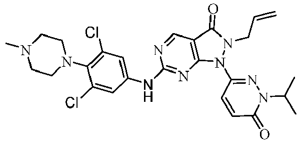 | 570 | **63** | 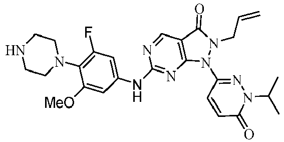 | 536 |
| **64** | 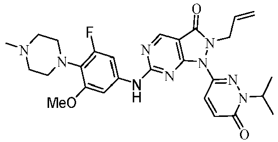 | 550 | **65** | 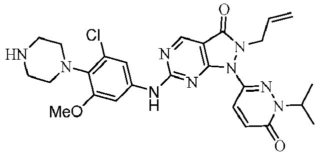 | 552 |
| **66** | 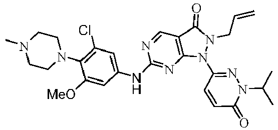 | 566 | **67** | 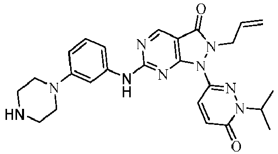 | 488 |
| **68** | 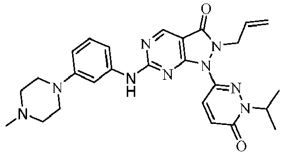 | 502 | **69** | 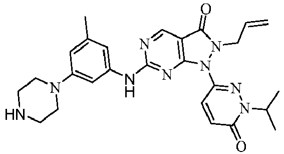 | 502 |
| **70** | 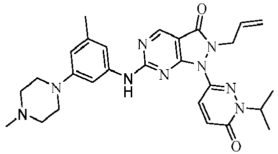 | 516 | **71** | 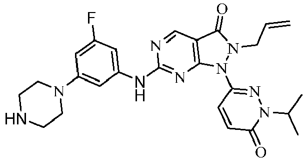 | 506 |
| **72** | 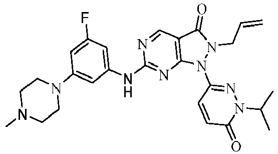 | 520 | **73** | 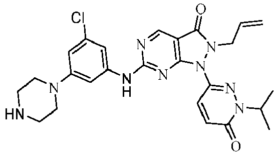 | 522 |
| **74** | 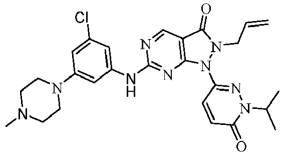 | 536 | **75** | 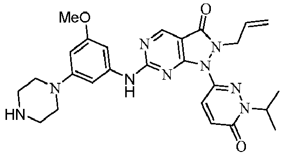 | 518 |
| **76** | 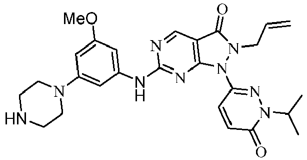 | 518 | **77** | 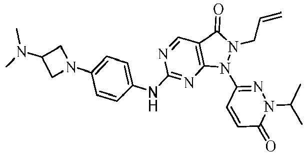 | 502 |
| **78** | 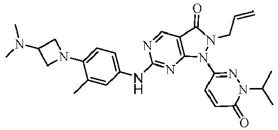 | 516 | **79** | 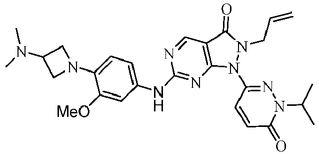 | 532 |
| **80** | 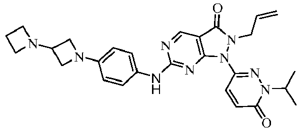 | 514 | **81** | 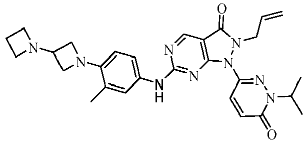 | 528 |
| **82** | 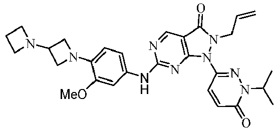 | 544 | **83** | 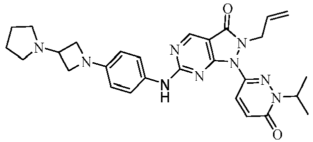 | 528 |
| **84** | 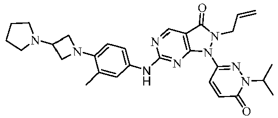 | 542 | **85** | 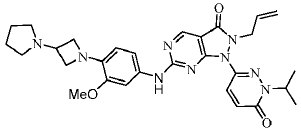 | 558 |
| **86** | 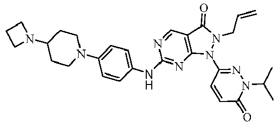 | 542 | **87** | 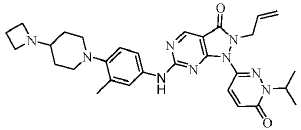 | 556 |
| **88** | 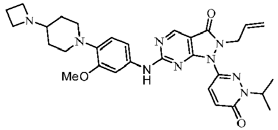 | 572 | **89** | 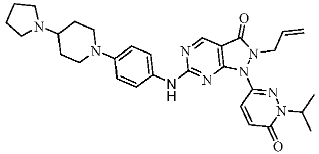 | 556 |
| **90** | 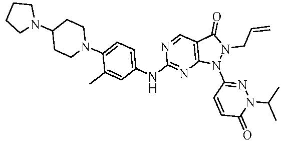 | 570 | **91** | 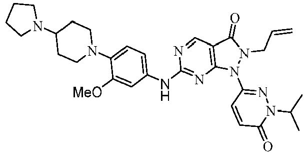 | 586 |
| **92** | 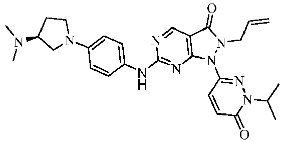 | 516 | **93** | 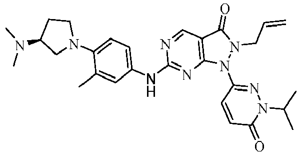 | 530 |
| **94** | 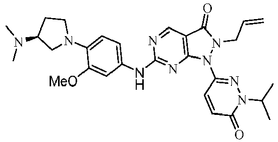 | 546 | **95** | 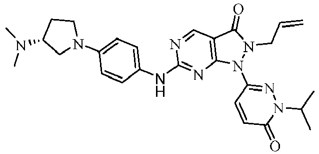 | 516 |
| **96** | 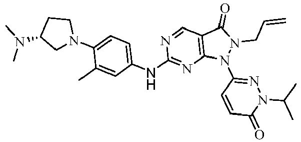 | 530 | **97** | 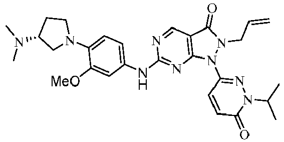 | 546 |
| **98** | 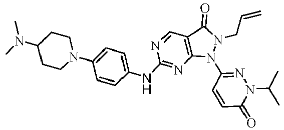 | 530 | **99** | 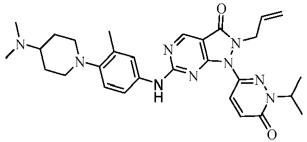 | 544 |
| **100** | 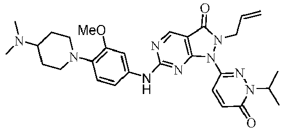 | 560 | **101** | 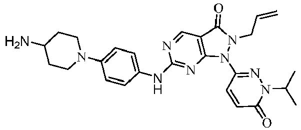 | 502 |
| **102** | 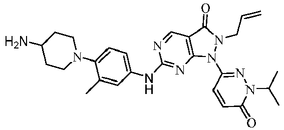 | 516 | **103** | 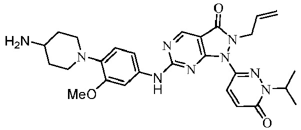 | 532 |
| **104** | 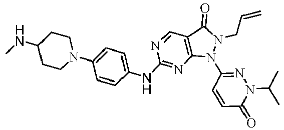 | 516 | **105** | 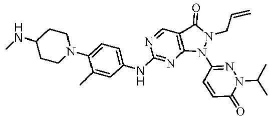 | 530 |
| **106** | 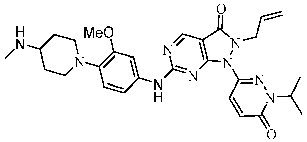 | 546 | **107** | 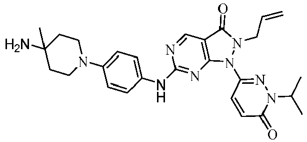 | 516 |
| **108** | 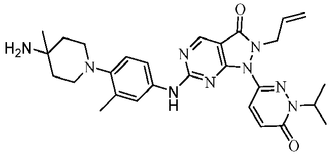 | 530 | **109** | 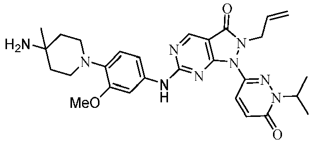 | 546 |
| **110** | 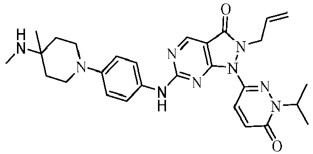 | 530 | **111** | 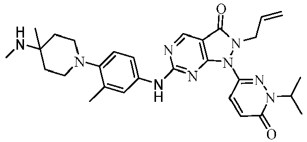 | 544 |
| **112** | 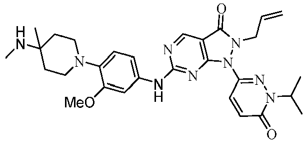 | 560 | **113** | 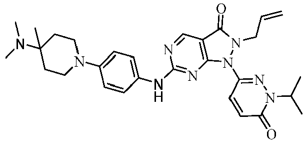 | 544 |
| **114** | 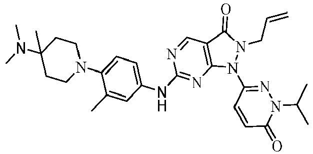 | 558 | **115** | 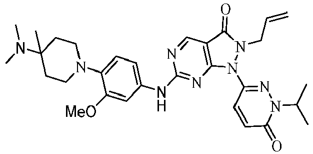 | 574 |
| **116** | 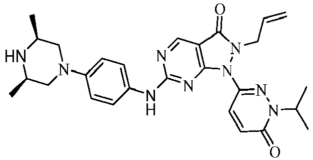 | 516 | **117** | 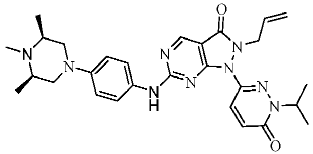 | 530 |
| **118** | 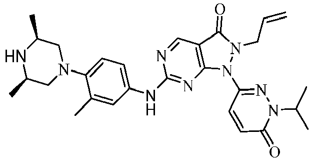 | 530 | **119** | 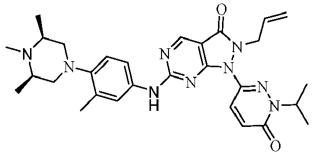 | 544 |
| **120** | 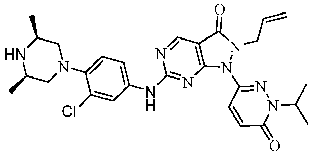 | 550 | **121** | 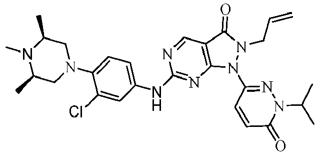 | 564 |
| **122** | 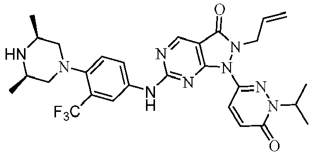 | 574 | **123** | 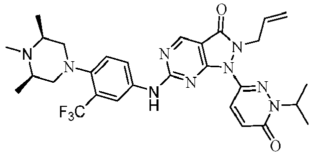 | 598 |
| **124** | 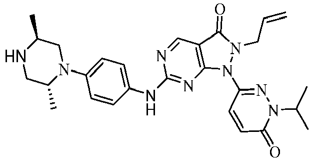 | 516 | **125** | 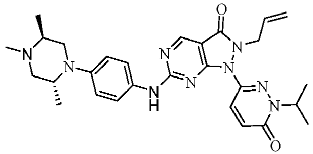 | 530 |
| **126** | 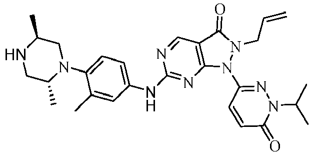 | 530 | **127** | 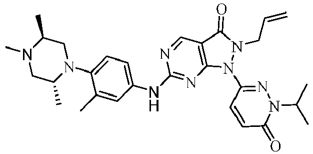 | 544 |
| **128** | 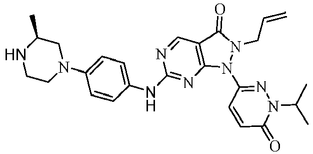 | 502 | **129** | 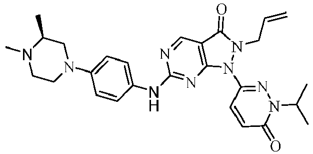 | 516 |
| **130** | 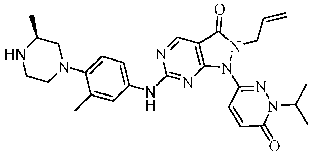 | 516 | **131** | 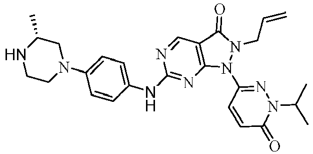 | 502 |
| **132** | 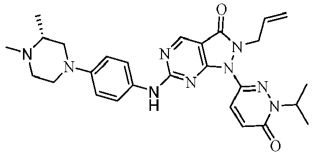 | 516 | **133** | 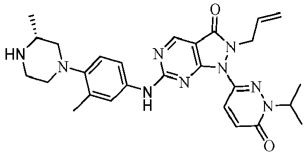 | 516 |
| **134** | 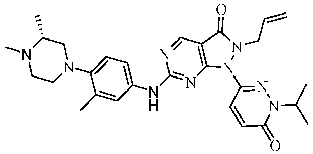 | 530 | **135** | 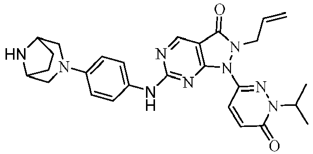 | 514 |
| **136** | 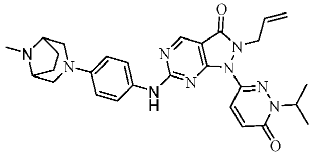 | 528 | **137** | 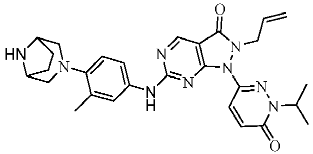 | 528 |
| **138** | 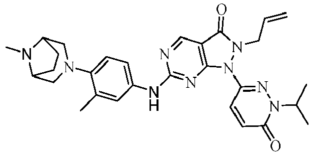 | 542 | **139** | 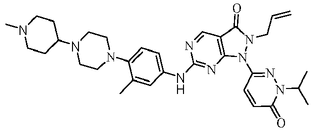 | 599 |
| **140** | 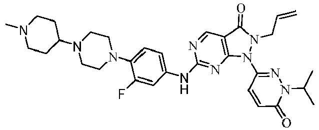 | 603 | **141** | 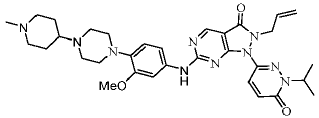 | 615 |
| **142** | 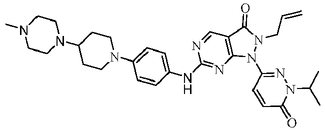 | 585 | **143** | 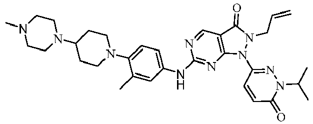 | 599 |
| **144** | 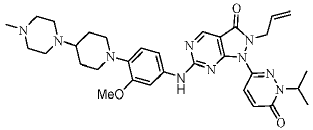 | 615 | **145** | 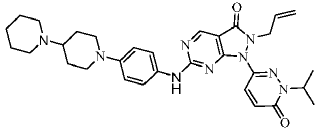 | 570 |
| **146** | 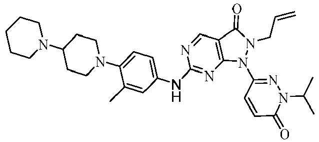 | 584 | **147** | 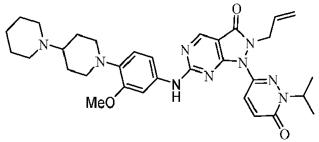 | 600 |
| **148** | 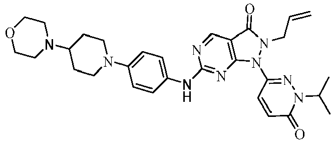 | 572 | **149** | 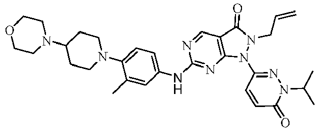 | 586 |
| **150** | 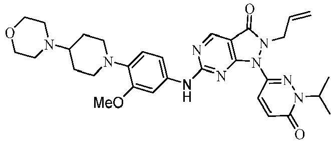 | 602 | **151** | 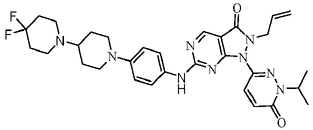 | 606 |
| **152** | 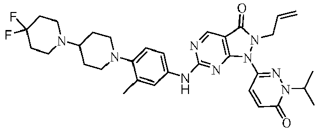 | 620 | **153** | 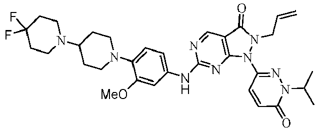 | 636 |
| **154** | 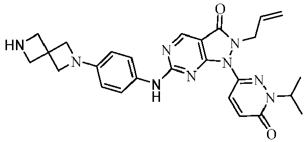 | 500 | **155** | 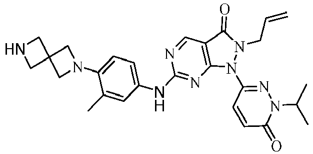 | 514 |
| **156** | 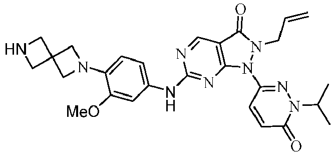 | 530 | **157** | 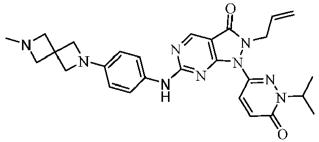 | 514 |
| **158** | 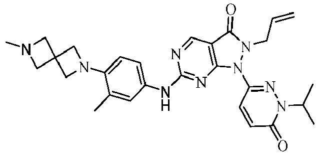 | 528 | **159** | 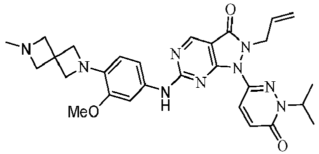 | 544 |
| **160** | 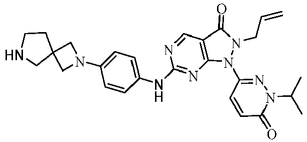 | 514 | **161** | 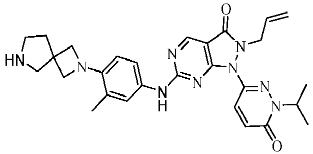 | 528 |
| **162** | 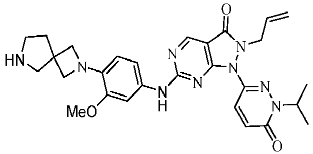 | 544 | **163** | 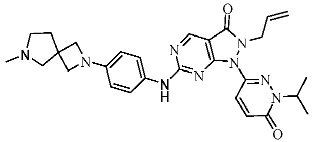 | 528 |
| **164** | 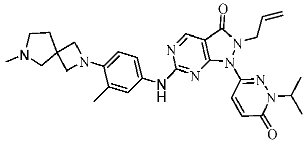 | 542 | **165** | 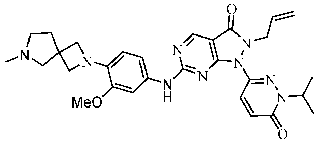 | 558 |
| **166** | 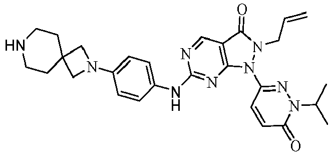 | 528 | **167** | 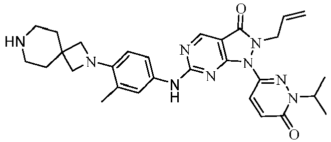 | 542 |
| **168** | 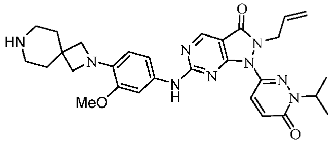 | 558 | **169** | 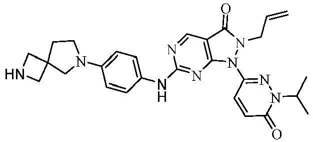 | 514 |
| **170** | 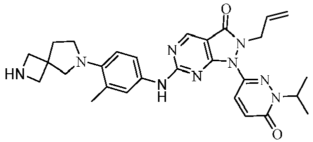 | 528 | **171** | 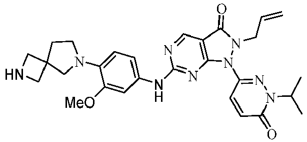 | 544 |
| **172** | 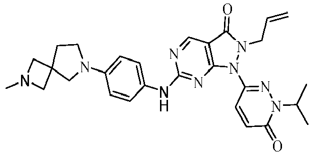 | 528 | **173** | 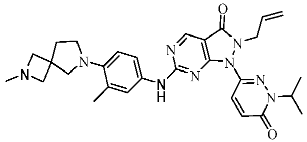 | 542 |
| **174** | 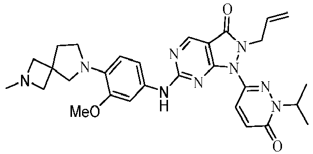 | 558 | **175** | 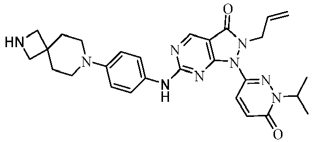 | 528 |
| **176** | 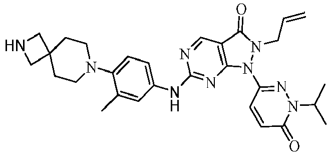 | 542 | **177** | 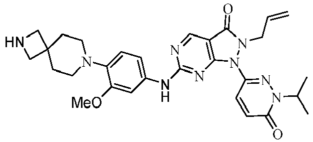 | 558 |
| **178** | 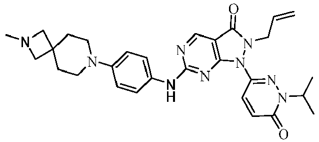 | 542 | **179** | 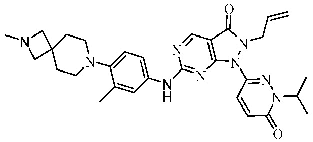 | 556 |
| **180** | 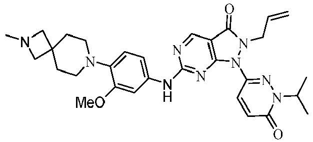 | 572 | **181** | 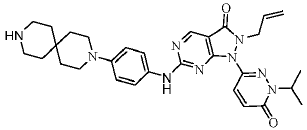 | 556 |
| **182** | 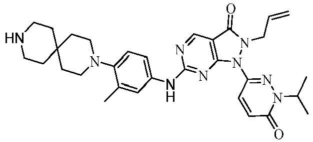 | 570 | **183** | 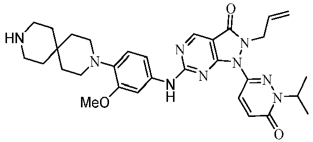 | 586 |
| **184** | 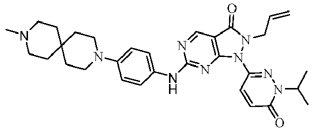 | 570 | **185** | 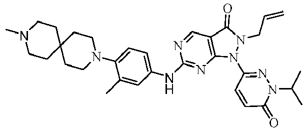 | 584 |
| **186** | 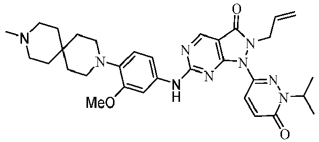 | 600 | **187** | 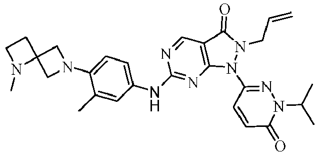 | 528 |
| **188** | 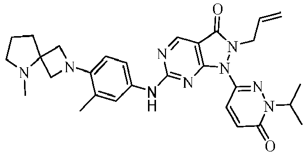 | 542 | **189** | 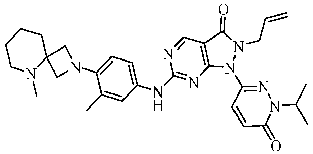 | 556 |
| **190** | 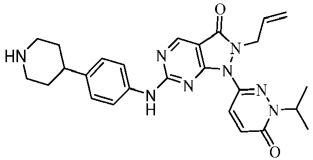 | 487 | **191** | 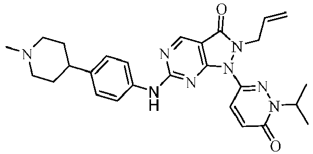 | 501 |
| **192** | 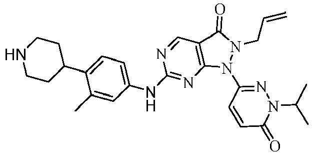 | 501 | **193** | 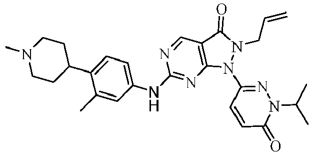 | 515 |
| **194** | 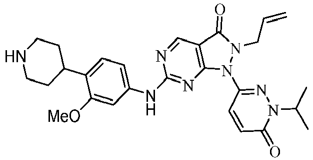 | 517 | **195** | 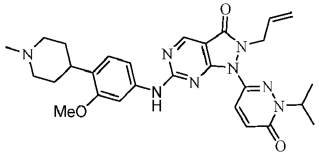 | 531 |
| **196** | 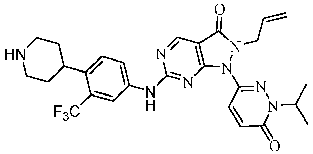 | 555 | **197** | 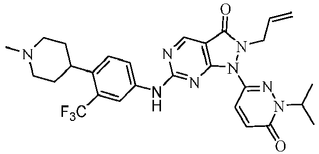 | 569 |
| **198** | 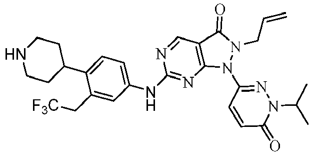 | 569 | **199** | 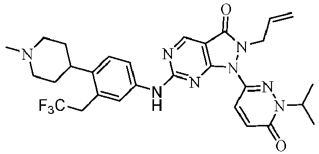 | 583 |
| **200** | 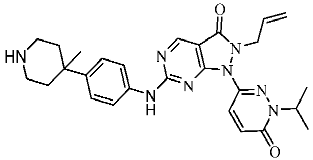 | 501 | **201** | 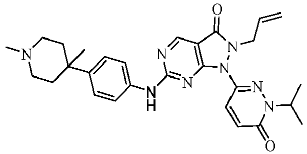 | 515 |
| **202** | 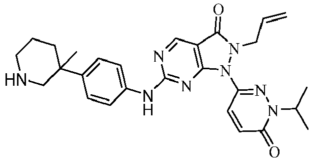 | 501 | **203** | 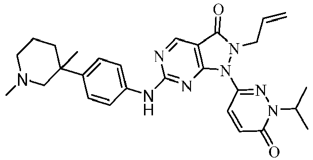 | 515 |
| **204** | 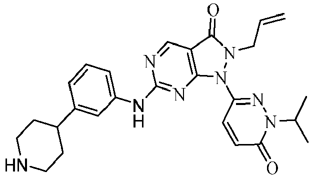 | 487 | **205** | 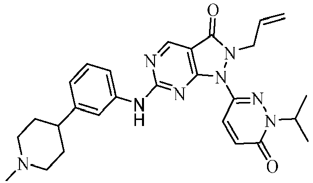 | 501 |
| **206** | 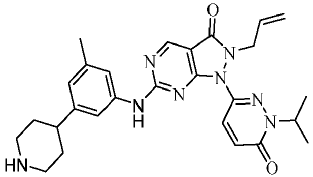 | 501 | **207** | 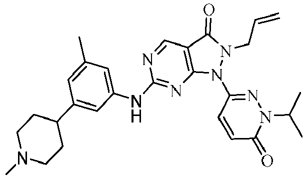 | 515 |
| **208** | 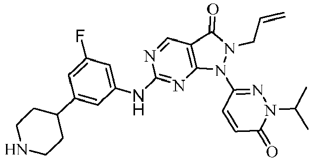 | 505 | **209** | 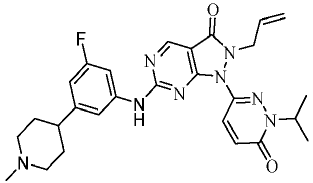 | 519 |
| **210** | 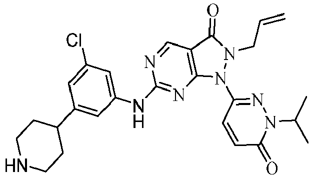 | 521 | **211** | 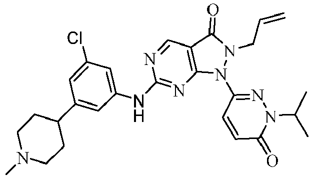 | 535 |
| **212** | 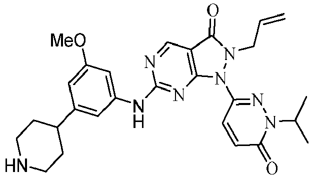 | 517 | **213** | 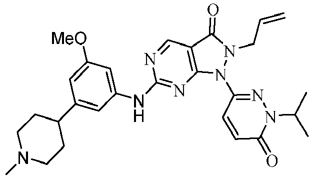 | 531 |
| **214** | 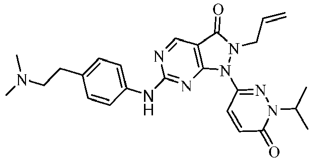 | 475 | **215** | 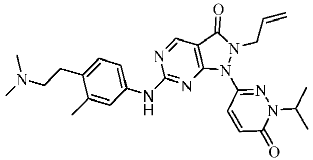 | 489 |
| **216** | 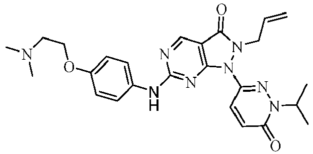 | 491 | **217** | 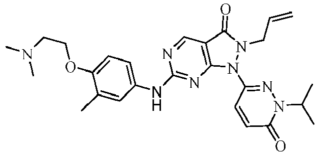 | 505 |
| **218** | 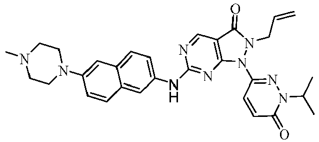 | 552 | **219** | 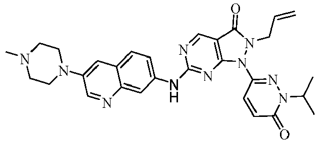 | 553 |
| **220** | 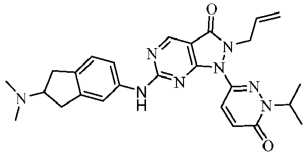 | 487 | **221** | 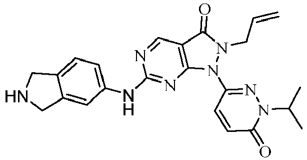 | 445 |
| **222** | 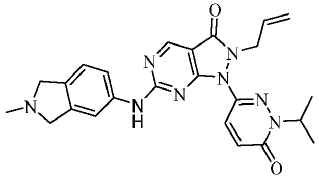 | 459 | **223** | 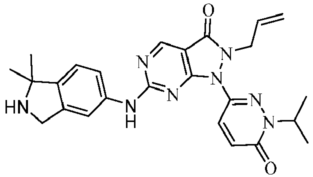 | 473 |
| **224** | 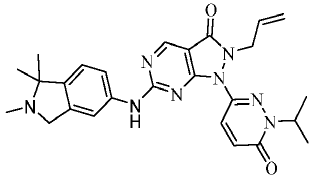 | 487 | **225** | 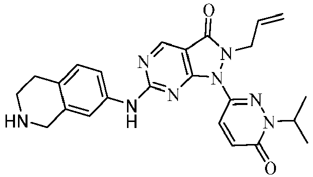 | 459 |
| **226** | 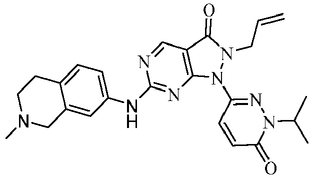 | 473 | **227** | 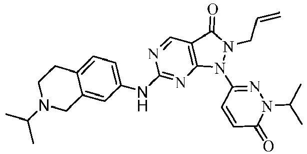 | 501 |
| **228** | 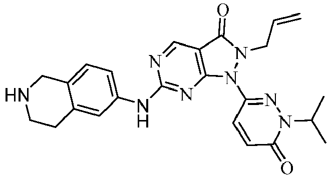 | 459 | **229** | 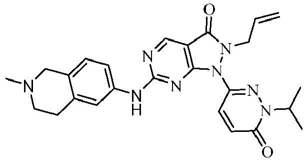 | 473 |
| **230** | 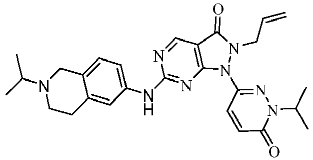 | 501 | **231** | 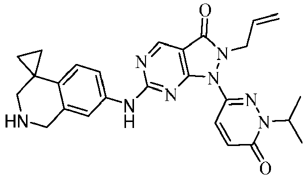 | 485 |
| **232** | 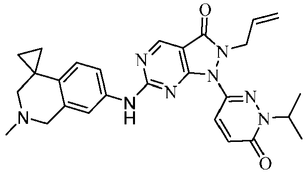 | 499 | **233** | 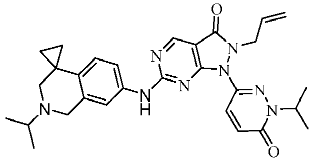 | 527 |
| 234 | 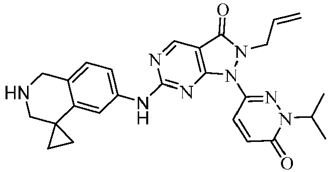 | 485 | **235** | 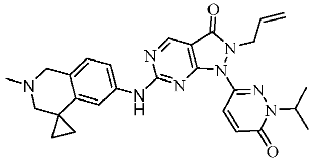 | 499 |
| **236** | 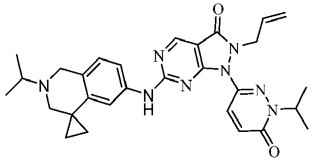 | 527 | **237** | 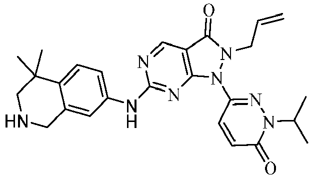 | 487 |
| **238** | 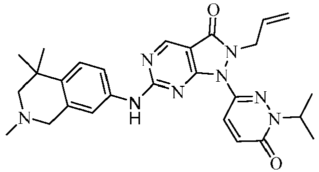 | 501 | **239** | 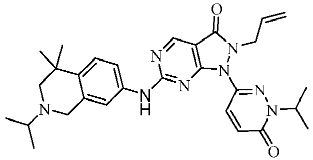 | 529 |
| **240** | 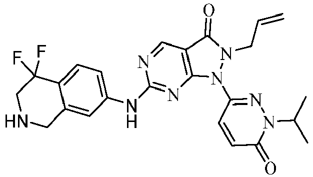 | 495 | **241** | 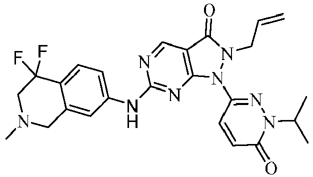 | 509 |
| **242** | 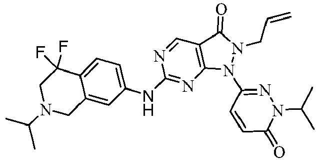 | 537 | **243** | 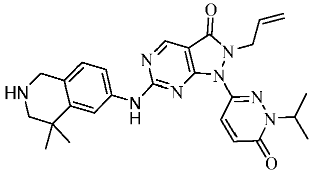 | 487 |
| **244** | 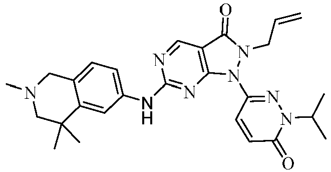 | 501 | **245** | 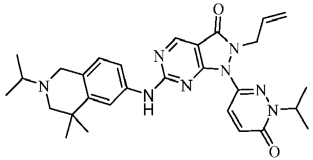 | 529 |
| **246** | 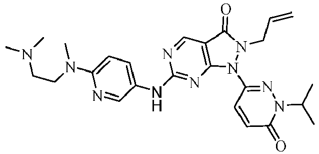 | 505 | **247** | 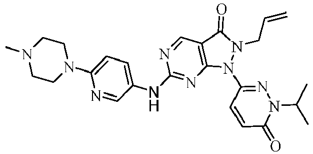 | 503 |
| **248** | 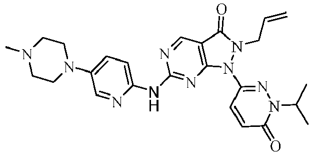 | 503 | **249** | 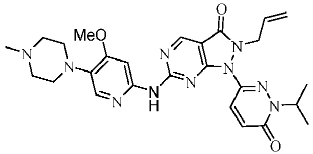 | 533 |
| **250** | 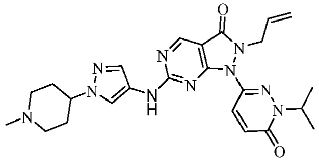 | 491 | **251** | 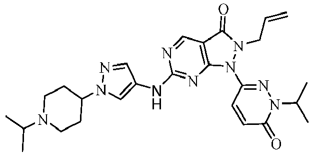 | 519 |
| **252** | 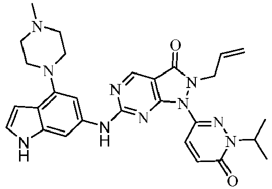 | 541 | **253** | 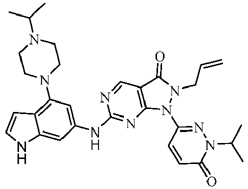 | 569 |
| **254** | 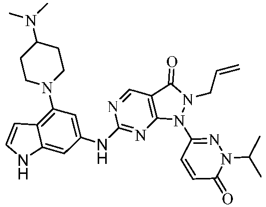 | 569 | **255** | 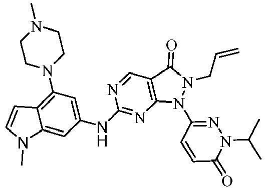 | 555 |
| **256** | 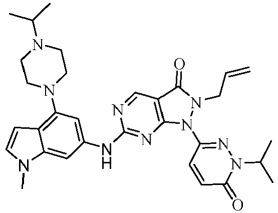 | 583 | **257** | 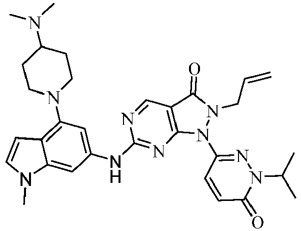 | 583 |
| **258** | 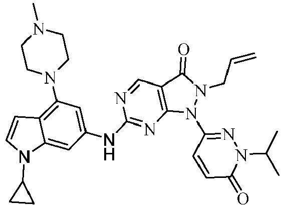 | 581 | **259** | 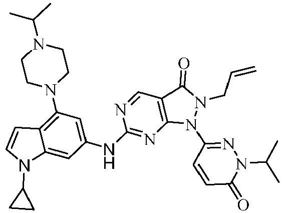 | 609 |
| **260** | 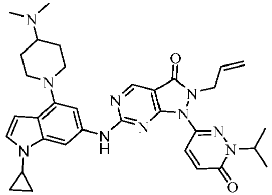 | 609 | **261** | 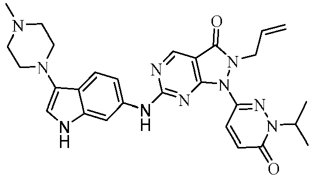 | 541 |
| **262** | 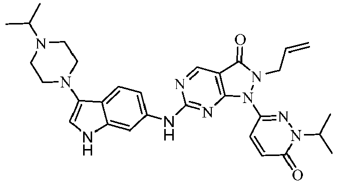 | 569 | **263** | 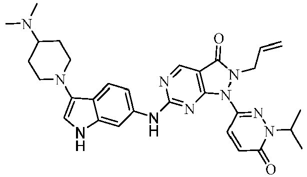 | 569 |
| **264** | 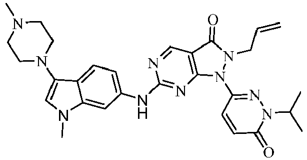 | 555 | **265** | 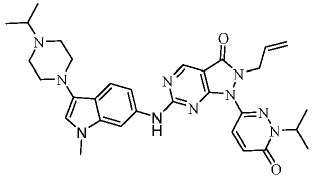 | 583 |
| **266** | 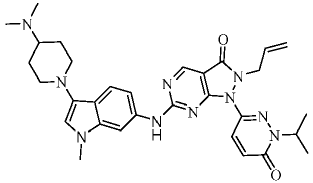 | 583 | **267** | 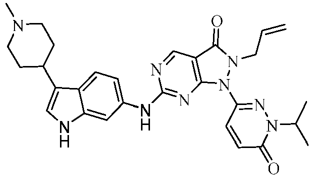 | 540 |
| **268** | 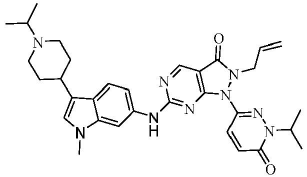 | 582 | **269** | 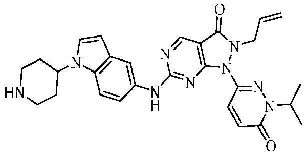 | 526 |
| **270** | 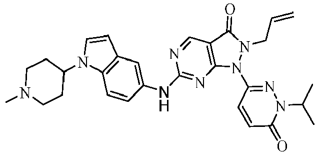 | 540 | **271** | 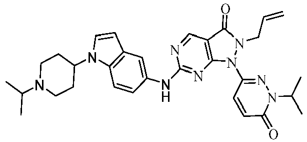 | 568 |
| **272** | 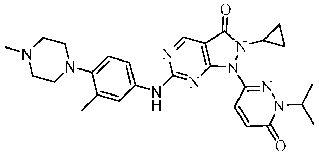 | 516 | **273** | 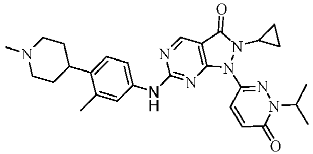 | 515 |
| **274** | 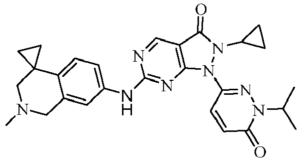 | 499 | **275** | 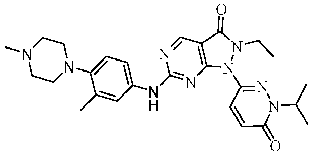 | 504 |
| **276** | 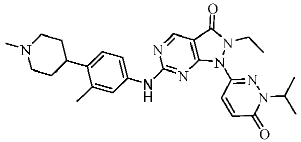 | 503 | **277** | 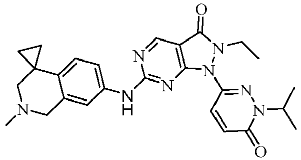 | 487 |
| **278** | 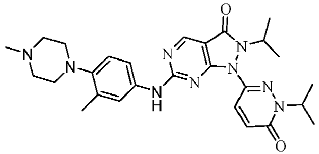 | 518 | **279** | 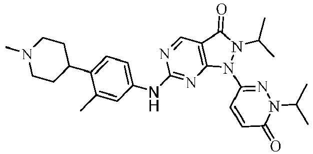 | 517 |
| **280** | 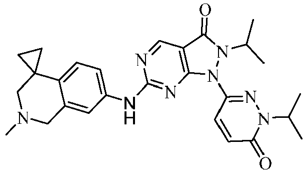 | 501 | **281** | 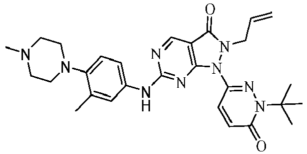 | 530 |
| **282** | 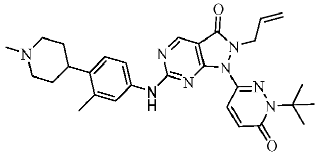 | 529 | **283** | 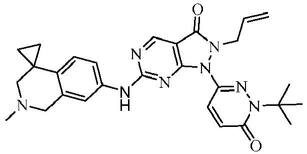 | 513 |
| **284** | 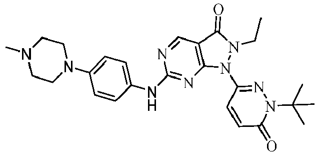 | 504 | **285** | 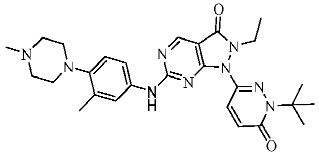 | 518 |
| **286** | 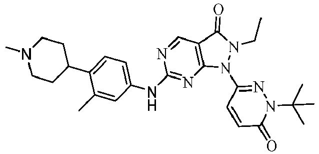 | 517 | **287** | 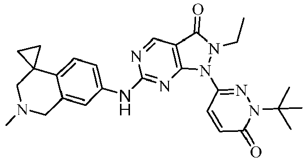 | 501 |
| **288** | 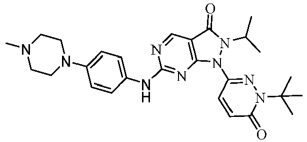 | 518 | **289** | 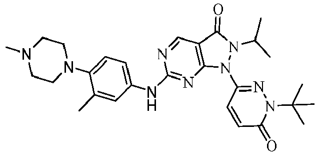 | 532 |
| **290** | 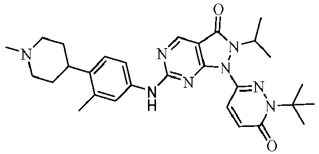 | 531 | **291** | 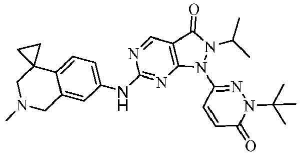 | 515 |
| **292** | 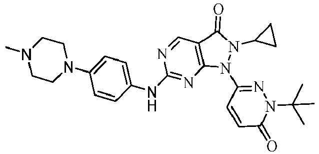 | 516 | **293** | 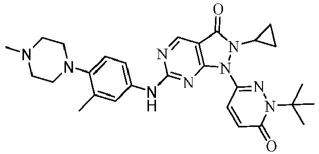 | 530 |
| **294** | 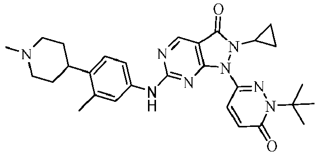 | 529 | **295** | 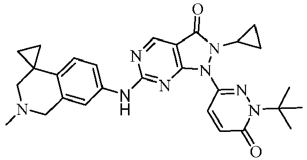 | 513 |
| **296** | 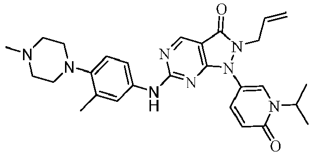 | 515 | **297** | 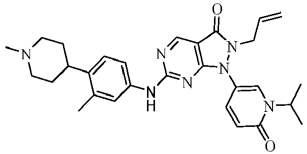 | 514 |
| **298** | 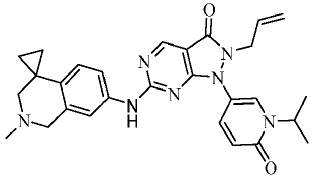 | 498 | **299** | 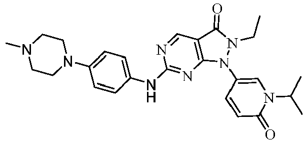 | 489 |
| **300** | 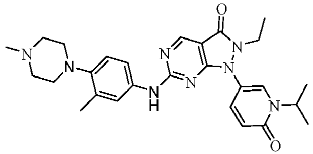 | 503 | **301** | 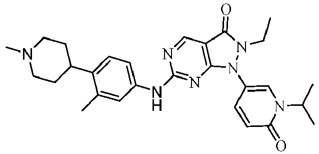 | 502 |
| **302** | 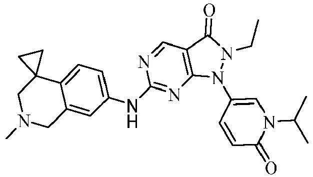 | 486 | **303** | 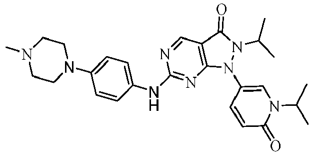 | 503 |
| **304** | 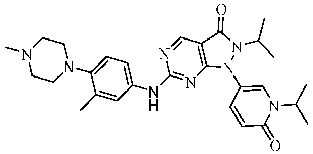 | 517 | **305** | 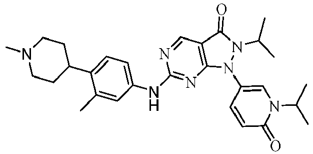 | 516 |
| **306** | 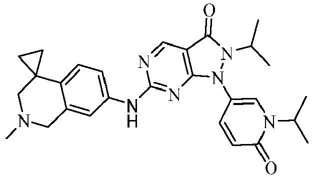 | 500 | **307** | 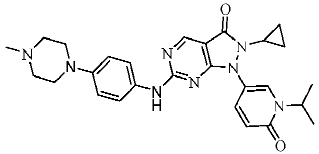 | 501 |
| **308** | 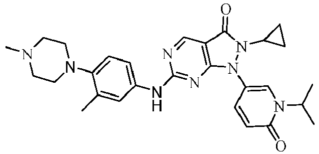 | 515 | **309** | 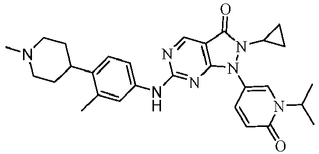 | 514 |
| **310** | 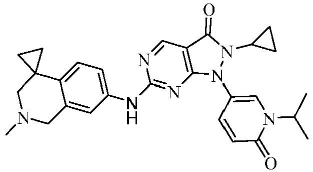 | 498 | **311** | 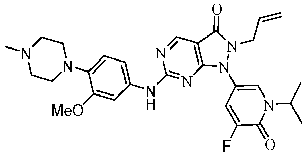 | 549 |
| **312** | 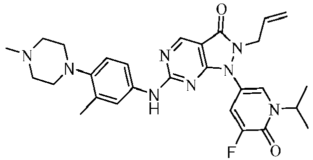 | 533 | **313** | 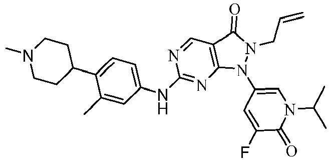 | 532 |
| **314** | 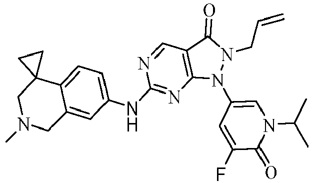 | 516 | **315** | 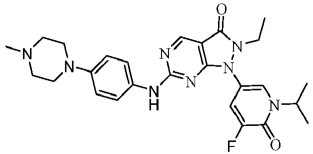 | 507 |
| **316** | 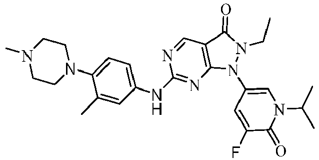 | 521 | **317** | 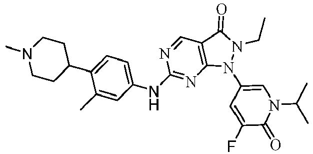 | 520 |
| **318** | 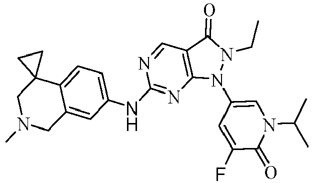 | 504 | **319** | 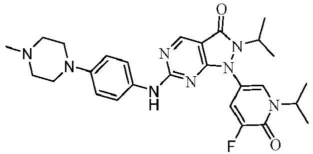 | 521 |
| **320** | 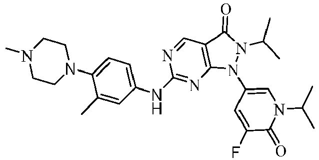 | 535 | **321** | 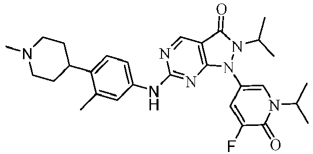 | 534 |
| **322** | 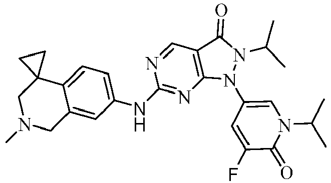 | 518 | **323** | 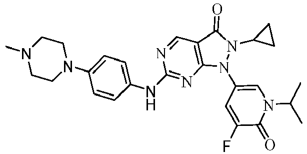 | 519 |
| **324** | 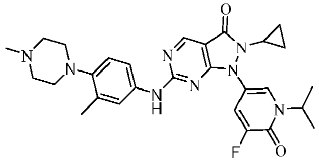 | 533 | **325** | 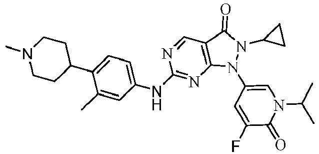 | 532 |
| **326** | 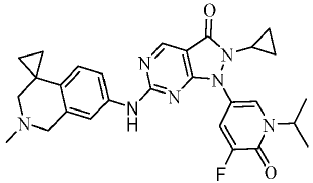 | 516 | **327** | 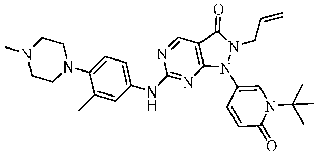 | 529 |
| **328** | 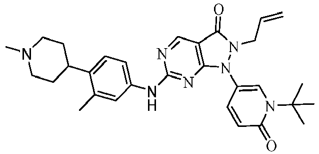 | 528 | **329** | 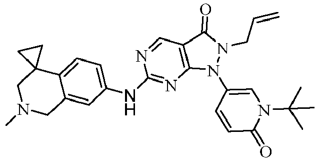 | 512 |
| **330** | 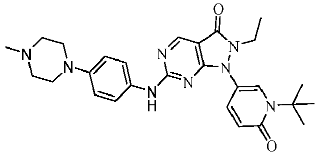 | 503 | **331** | 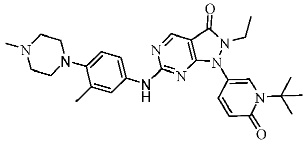 | 517 |
| **332** | 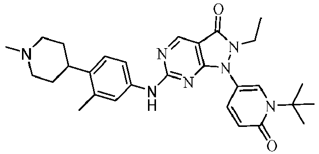 | 516 | **333** | 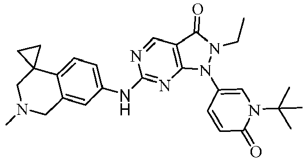 | 500 |
| **334** | 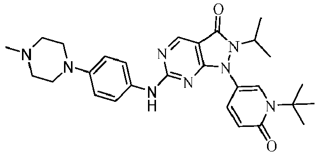 | 517 | **335** | 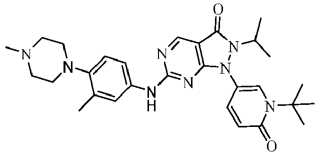 | 531 |
| **336** | 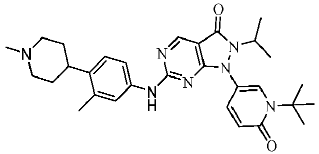 | 530 | **337** | 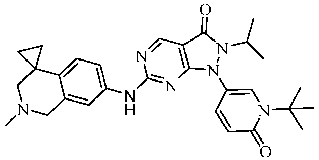 | 514 |
| **338** | 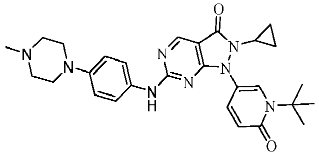 | 515 | **339** | 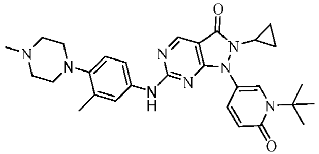 | 529 |
| **340** | 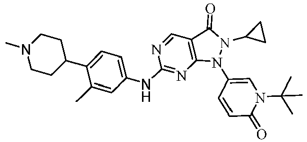 | 528 | **341** | 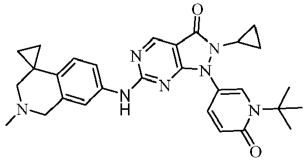 | 512 |

### Example 342. Assay for Inhibitory Activity of Compounds Against Wee-1 Kinase

The inhibitory activity of compounds against Wee-1 kinase was determined by using the Lanthra Screen Wee-1 kinase kit (Invitrogen). 5 µL of the compound diluted in a gradient with DMSO, 5 µL of Wee-1 kinase (at a final concentration of 5 nM), 5 µL of an Eu-Anti-GST antibody (at a final concentration of 2 nM) mixture and 5 µL of kinase Tracer 178 (at a final concentration of 50 nM) were mixed well. The plate was read after incubation at room temperature for one hour. For comparison with the DMSO solvent control group, IC₅₀ for the inhibition activity of the compounds against Wee-1 kinase was calculated.

**Table 4. IC₅₀ for the inhibitory activity of the compounds of the present invention against Wee-1 kinase**

| Compound | Inhibitory activity against Wee-1 | Compound | Inhibitory activity against Wee-1 | Compound | Inhibitory activity against Wee-1 |
|---|---|---|---|---|---|
| **1** | C | **2** | C | **3** | A |
| **4** | B | **5** | A | **6** | B |
| **7** | B | **8** | B | **9** | A |
| **10** | A | **11** | A | **12** | A |
| **13** | A | **14** | B | **15** | B |
| **16** | B | **17** | B | **18** | A |
| **19** | A | **20** | A | **21** | A |
| **22** | A | **23** | A | **24** | A |
| **25** | A | **26** | A | **27** | B |
| **28** | B | **29** | A | **30** | B |
| **31** | A | **32** | A | **33** | A |
| **34** | A | **35** | A | **36** | A |
| **37** | A | **38** | A | **39** | A |
| **40** | A | **41** | A | **42** | A |
| **43** | A | **44** | A | **45** | A |
| **46** | A | **47** | A | **48** | A |
| **49** | A | **50** | A | **51** | B |
| **52** | A | **53** | A | **54** | A |
| **55** | A | **56** | A | **57** | A |
| **58** | A | **59** | A | **60** | A |
| **61** | A | **62** | A | **63** | A |
| **64** | A | **65** | A | **66** | A |
| **67** | A | **68** | A | **69** | A |
| **70** | A | **71** | A | **72** | A |
| **73** | A | **74** | A | **75** | A |
| **76** | A | **77** | A | **78** | A |
| **79** | A | **80** | A | **81** | A |
| **82** | A | **83** | A | **84** | A |
| **85** | A | **86** | A | **87** | A |
| **88** | A | **89** | A | **90** | A |
| **91** | A | **92** | A | **93** | A |
| **94** | A | **95** | A | **96** | A |
| **97** | A | **98** | A | **99** | A |
| **100** | A | **101** | A | **102** | A |
| **103** | A | **104** | A | **105** | A |
| **106** | A | **107** | A | **108** | A |
| **109** | A | **110** | A | **111** | A |
| **112** | A | **113** | A | **114** | A |
| **115** | A | **116** | A | **117** | A |
| **118** | A | **119** | A | **120** | A |
| **121** | A | **122** | A | **123** | A |
| **124** | A | **125** | A | **126** | A |
| **127** | A | **128** | A | **129** | A |
| **130** | A | **131** | A | **132** | A |
| **133** | A | **134** | A | **135** | B |
| **136** | B | **137** | A | **138** | A |
| **139** | A | **140** | A | **141** | A |
| **142** | A | **143** | A | **144** | A |
| **145** | A | **146** | A | **147** | A |
| **148** | A | **149** | A | **150** | A |
| **151** | A | **152** | A | **153** | A |
| **154** | A | **155** | A | **156** | A |
| **157** | A | **158** | A | **159** | A |
| **160** | A | **161** | A | **162** | A |
| **163** | A | **164** | A | **165** | A |
| **166** | B | **167** | B | **168** | B |
| **169** | A | **170** | A | **171** | A |
| **172** | A | **173** | A | **174** | A |
| **175** | A | **176** | A | **177** | A |
| **178** | A | **179** | A | **180** | A |
| **181** | A | **182** | A | **183** | A |
| **184** | A | **185** | A | **186** | A |
| **187** | A | **188** | A | **189** | B |
| **190** | A | **191** | A | **192** | A |
| **193** | A | **194** | A | **195** | A |
| **196** | A | **197** | A | **198** | A |
| **199** | A | **200** | A | **201** | A |
| **202** | A | **203** | A | **204** | A |
| **205** | A | **206** | A | **207** | A |
| **208** | A | **209** | A | **210** | A |
| **211** | A | **212** | A | **213** | A |
| **214** | A | **215** | A | **216** | A |
| **217** | A | **218** | A | **219** | A |
| **220** | A | **221** | A | **222** | A |
| **223** | A | **224** | A | **225** | A |
| **226** | A | **227** | A | **228** | A |
| **229** | A | **230** | A | **231** | A |
| **232** | A | **233** | A | **234** | A |
| **235** | A | **236** | A | **237** | A |
| **238** | A | **239** | A | **240** | A |
| **241** | A | **242** | A | **243** | A |
| **244** | A | **245** | A | **246** | A |
| **247** | A | **248** | A | **249** | A |
| **250** | B | **251** | B | **252** | A |
| **253** | A | **254** | A | **255** | A |
| **256** | A | **257** | A | **258** | A |
| **259** | A | **260** | A | **261** | A |
| **262** | A | **263** | A | **264** | A |
| **265** | A | **266** | A | **267** | A |
| **268** | A | **269** | A | **270** | A |
| **271** | A | **272** | A | **273** | A |
| **274** | A | **275** | A | **276** | A |
| **277** | A | **278** | A | **279** | A |
| **280** | A | **281** | A | **282** | A |
| **283** | A | **284** | A | **285** | A |
| **286** | A | **287** | A | **288** | A |
| **289** | A | **290** | A | **291** | A |
| **292** | A | **293** | A | **294** | A |
| **295** | A | **296** | A | **297** | A |
| **298** | A | **299** | A | **300** | A |
| **301** | A | **302** | A | **303** | A |
| **304** | A | **305** | A | **306** | A |
| **307** | A | **308** | A | **309** | A |
| **310** | A | **311** | A | **312** | A |
| **313** | A | **314** | A | **315** | A |
| **316** | A | **317** | A | **318** | A |
| **319** | A | **320** | A | **321** | A |
| **322** | A | **323** | A | **324** | A |
| **325** | A | **326** | A | **327** | A |
| **328** | A | **329** | A | **330** | A |
| **331** | A | **332** | A | **333** | A |
| **334** | A | **335** | A | **336** | A |
| **337** | A | **338** | A | **339** | A |
| **340** | A | **341** | A | **AZD-1775** | A |

| | | | | | |
|---|---|---|---|---|---|
| A indicates that the IC₅₀ is less than or equal to 30 nM; B indicates that the IC₅₀ is greater than 30 nM but less than or equal to 100 nM; C indicates that the IC₅₀ is greater than 100 nM. | | | | | |

As can be seen from the data in Table 4, the compounds of the present invention have strong inhibitory effect on Wee-1 kinase.

### Example 343. Assay for Antiproliferative Activity against HT29 Cells

3000 HT29 cells were seed in a 384-well plate (Fisher 142762). After the cells adhered to the wall overnight, the compounds diluted in a gradient were added. 72 later, Cell Titer-Lumi (Beyotime C0068XL) was added to determine the content of ATP in the cells. The growth of the cells was evaluated, and the IC₅₀ for the inhibition of the compounds against cell growth was calculated.

**Table 5. IC₅₀ for the inhibition of the compounds of the present invention against HT-29 cell growth**

| **Compound** | **HT-29 cells Antiproliferative activity** | **Compound** | **HT-29 cells Antiproliferative activity** | **Compound** | **HT-29 cells Antiproliferative activity** |
|---|---|---|---|---|---|
| **1** | C | **2** | C | **3** | A |
| **4** | B | **5** | A | **6** | B |
| **7** | B | **8** | B | **9** | A |
| **10** | A | **11** | A | **12** | A |
| **13** | A | **14** | C | **15** | B |
| **16** | B | **17** | B | **18** | B |
| **19** | B | **20** | A | **21** | A |
| **22** | A | **23** | C | **24** | A |
| **25** | A | **26** | A | **27** | C |
| **28** | C | **29** | A | **30** | C |
| **31** | A | **32** | A | **33** | A |
| **34** | A | **35** | A | **36** | A |
| **37** | A | **38** | A | **39** | A |
| **40** | A | **41** | A | **42** | A |
| **43** | A | **44** | A | **45** | A |
| **46** | A | **47** | A | **48** | A |
| **49** | A | **50** | A | **51** | B |
| **52** | B | **53** | A | **54** | A |
| **55** | A | **56** | A | **57** | A |
| **58** | A | **59** | A | **60** | A |
| **61** | A | **62** | A | **63** | A |
| **64** | A | **65** | A | **66** | A |
| **67** | A | **68** | A | **69** | A |
| **70** | A | **71** | A | **72** | A |
| **73** | A | **74** | A | **75** | A |
| **76** | A | **77** | A | **78** | A |
| **79** | A | **80** | B | **81** | A |
| **82** | A | **83** | A | **84** | A |
| **85** | A | **86** | A | **87** | A |
| **88** | A | **89** | A | **90** | A |
| **91** | A | **92** | A | **93** | A |
| **94** | A | **95** | A | **96** | A |
| **97** | A | **98** | A | **99** | A |
| **100** | A | **101** | B | **102** | A |
| **103** | A | **104** | A | **105** | A |
| **106** | A | **107** | A | **108** | A |
| **109** | A | **110** | A | **111** | A |
| **112** | A | **113** | A | **114** | A |
| **115** | A | **116** | A | **117** | A |
| **118** | A | **119** | A | **120** | A |
| **121** | A | **122** | A | **123** | A |
| **124** | A | **125** | A | **126** | A |
| **127** | A | **128** | A | **129** | A |
| **130** | A | **131** | A | **132** | A |
| **133** | A | **134** | A | **135** | B |
| **136** | B | **137** | B | **138** | B |
| **139** | A | **140** | A | **141** | A |
| **142** | A | **143** | A | **144** | A |
| **145** | A | **146** | A | **147** | A |
| **148** | A | **149** | A | **150** | A |
| **151** | A | **152** | A | **153** | A |
| **154** | B | **155** | A | **156** | B |
| **157** | A | **158** | A | **159** | A |
| **160** | B | **161** | B | **162** | A |
| **163** | A | **164** | A | **165** | A |
| **166** | B | **167** | B | **168** | B |
| **169** | B | **170** | A | **171** | A |
| **172** | A | **173** | A | **174** | A |
| **175** | B | **176** | A | **177** | A |
| **178** | A | **179** | A | **180** | A |
| **181** | B | **182** | A | **183** | A |
| **184** | A | **185** | A | **186** | A |
| **187** | A | **188** | A | **189** | A |
| **190** | A | **191** | A | **192** | A |
| **193** | A | **194** | A | **195** | A |
| **196** | A | **197** | A | **198** | A |
| **199** | A | **200** | A | **201** | A |
| **202** | A | **203** | A | **204** | A |
| **205** | A | **206** | A | **207** | A |
| **208** | A | **209** | A | **210** | A |
| **211** | A | **212** | A | **213** | A |
| **214** | A | **215** | A | **216** | A |
| **217** | A | **218** | A | **219** | A |
| **220** | A | **221** | B | **222** | A |
| **223** | A | **224** | A | **225** | A |
| **226** | A | **227** | A | **228** | A |
| **229** | A | **230** | A | **231** | A |
| **232** | A | **233** | A | **234** | A |
| **235** | A | **236** | A | **237** | A |
| **238** | A | **239** | A | **240** | A |
| **241** | A | **242** | A | **243** | A |
| **244** | A | **245** | A | **246** | A |
| **247** | A | **248** | A | **249** | A |
| **250** | B | **251** | B | **252** | A |
| **253** | A | **254** | A | **255** | A |
| **256** | A | **257** | A | **258** | A |
| **259** | A | **260** | A | **261** | A |
| **262** | A | **263** | A | **264** | A |
| **265** | A | **266** | A | **267** | A |
| **268** | A | **269** | A | **270** | A |
| **271** | A | **272** | A | **273** | A |
| **274** | A | **275** | A | **276** | A |
| **277** | A | **278** | A | **279** | A |
| **280** | A | **281** | A | **282** | A |
| **283** | A | **284** | A | **285** | A |
| **286** | A | **287** | A | **288** | A |
| **289** | A | **290** | A | **291** | A |
| **292** | A | **293** | A | **294** | A |
| **295** | A | **296** | A | **297** | A |
| **298** | A | **299** | A | **300** | A |
| **301** | A | **302** | A | **303** | A |
| **304** | A | **305** | A | **306** | A |
| **307** | A | **308** | A | **309** | A |
| **310** | A | **311** | A | **312** | A |
| **313** | A | **314** | A | **315** | A |
| **316** | A | **317** | A | **318** | A |
| **319** | A | **320** | A | **321** | A |
| **322** | A | **323** | A | **324** | A |
| **325** | A | **326** | A | **327** | A |
| **328** | A | **329** | A | **330** | A |
| **331** | A | **332** | A | **333** | A |
| **334** | A | **335** | A | **336** | A |
| **337** | A | **338** | A | **339** | A |
| **340** | A | **341** | A | **AZD1775** | A |

| | | | | | |
|---|---|---|---|---|---|
| A indicates that the IC₅₀ is less than or equal to 1 µM; B indicates that the IC₅₀ is greater than 1 µM but less than or equal to 3 µM; C indicates that the IC₅₀ is greater than 3 µM. | | | | | |

As can be seen from the data in Table 5, the compounds of the present invention have strong antiproliferative activity against HT-29 cells.

### Example 344. Pharmacokinetic Evaluation in Mice

The compounds were administered by intravenous injection at a dose of 2 mg/kg and oral gavage at a dose of 10 mg/kg (0.5% CMC-Na suspension). 15 male ICR mice were selected for each group, and each mouse was subjected to blood collection at 3 discrete time points, with 3 mice at each time point. The time points of sampling were as follows: before administration, and 5 min, 15 min, 30 min, 1 h, 3 h, 5 h, 8 h, 12 h and 24 h after administration. 80 µL of blood was collected from the orbits or hearts of the mice at each time point after administration. All whole blood samples were collected in tubes containing EDTA K₂ and centrifuged (1500-1600 rmp/min) at 4 °C for 10 min to isolate plasma, which was then stored in a refrigerator at -90 °C to -60 °C for sample analysis. The compound concentration in the plasma was determined by liquid chromatography-tandem mass spectrometry, and the corresponding pharmacokinetic parameters were obtained according to a plasma concentration-time curve.

**Table 6. Pharmacokinetic parameters of compound 3 in mice**

| Compound | Route of adminis tration | Dose (mg/kg) | t _{½} (h) | Tₘₐₓ (h) | Cₘₐₓ (ng/mL) | AUC ₀₋ₜ (ng.h/L) | Vss (L/kg) | Cl (mL/h/kg) | F (%) |
|---|---|---|---|---|---|---|---|---|---|
| **3** | iv | 2 | 0.718 | NA | NA | 736 | 1.8 | 45.3 | NA |
| | po | 10 | 1.91 | 0.25 | 533 | 1210 | NA | NA | 32.9 |
| **AZD-1775** | iv | 2 | 0.30 | NA | NA | 152 | 3.51 | 220 | NA |
| | po | 10 | 1.45 | 0.5 | 247 | 215 | NA | NA | 28.3 |

| | | | | | | | | | |
|---|---|---|---|---|---|---|---|---|---|
| NA indicates that the data are not available. | | | | | | | | | |

As can be seen from the above table, compound **3** has good oral absorption characteristics, and has half-life (t_{½}), maximum plasma concentration (Cₘₐₓ), area under the drug-time curve (AUC₀₋ₜ), oral bioavailability metabolic parameters and the like thereof all superior to those of the control drug AZD-1775. Good oral absorption properties are of great significance in improving the efficacy of drugs, reducing the dose of administration and reducing the costs. Further experiments have proved that other compounds of the present invention also have good oral absorption characteristics, and have half-life (t_{½}), maximum plasma concentration (Cₘₐₓ), area under the drug-time curve (AUC₀₋ₜ), oral bioavailability metabolic parameters and the like thereof all superior to those of the control drug AZD-1775.

## Claims

1. A compound with a structure as shown in general formula (1) or isomers, crystalline forms, pharmaceutically acceptable salts, hydrates or solvates thereof: 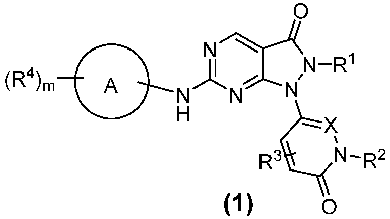 wherein
m is an integer of 1, 2 or 3;
X is Nor CH;
A is a divalent or more than divalent aryl, a divalent or more than divalent heteroaryl, a divalent or more than divalent cycloalkyl-aryl, a divalent or more than divalent heterocycloalkyl-aryl, or a divalent or more than divalent heterocycloalkyl-heteroaryl;
R¹ is C1-C6 alkyl, halogen-substituted C1-C3 alkyl, C3-C6 cycloalkyl, -CH₂(C3-C6) cycloalkyl or C3-C5 alkenyl;
R² is C1-C6 alkyl, C3-C6 cycloalkyl or (4- to 6-membered) heterocycloalkyl, wherein the alkyl, cycloalkyl and heterocycloalkyl can be optionally substituted with 1 to 3 of the following groups: H, halogen, OH, Me or OMe;
R³ is H, halogen, CN, C1-C3 alkyl, halogen-substituted C1-C3 alkyl or C1-C3 alkoxy;
each R⁴ is independently H, halogen, C1-C3 alkyl, halogen-substituted C1-C3 alkyl, hydroxyl-substituted C1-C3 alkyl, C1-C3 alkoxy, NMe₂-substituted C1-C3 alkyl, NMe₂-substituted C1-C3 alkoxy, 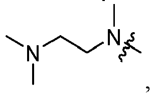 NMe₂, C3-C6 cycloalkyl, (4- to 12-membered) heterocycloalkyl or -CH₂(4- to 12-membered) heterocycloalkyl, wherein the (4- to 12-membered) heterocycloalkyl can be optionally substituted with 1 to 3 R⁵, and R⁵ is independently H, halogen, CN, OH, C1-C3 alkyl, halogen-substituted C1-C3 alkyl, hydroxyl-substituted C1-C3 alkyl, cyano-substituted C1-C3 alkyl, C3-C6 cycloalkyl, halogen-substituted C3-C6 cycloalkyl, 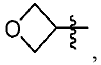 NR⁶R⁷ or -(C1-C3 alkyl)-NR⁶R⁷, wherein R⁶ and R⁷ are independently H or C1-C3 alkyl, or R⁶ and R⁷ form a 4- to 7-membered heterocycloalkyl with an N atom to which they are both attached; wherein two R⁴ can, together with C atoms, form a C2-C3 alkylene; wherein R⁴ or R⁵ cannot be halogen when connected to a heteroatom.

2. The compound or the isomers, the crystalline forms, the pharmaceutically acceptable salts, the hydrates or the solvates thereof according to claim 1, wherein R¹ is Me, Et, 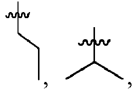 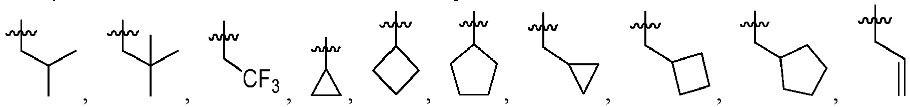 or

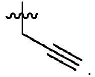

3. The compound or the isomers, the crystalline forms, the pharmaceutically acceptable salts, the hydrates or the solvates thereof according to claims 1 and 2, wherein R² is Me, Et, 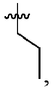 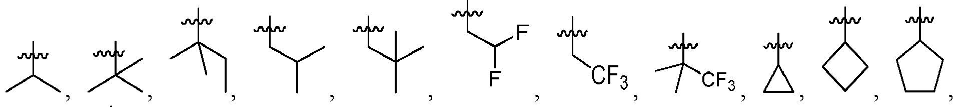 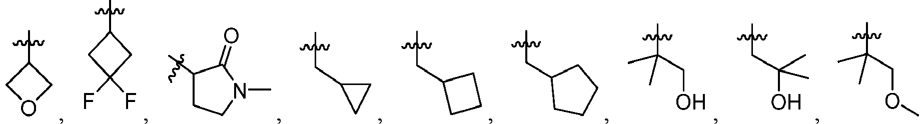 or

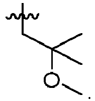

4. The compound or the isomers, the crystalline forms, the pharmaceutically acceptable salts, the hydrates or the solvates thereof according to claims 1-3, wherein R³ is H, F, Me, Et, 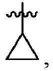 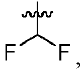 CF₃, OMe or OEt.

5. The compound or the isomers, the crystalline forms, the pharmaceutically acceptable salts, the hydrates or the solvates thereof according to claims 1-4, wherein in the general formula (1), 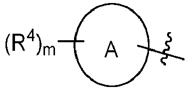 is the following group: 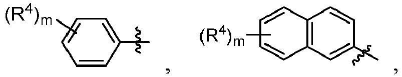 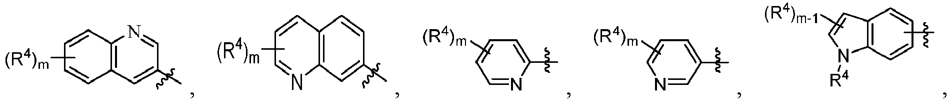 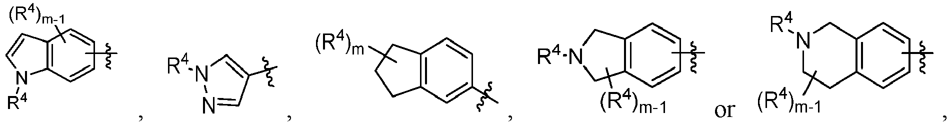 wherein m is an integer of 1, 2 or 3, each R⁴ is independently H, halogen, C1-C3 alkyl, halogen-substituted C1-C3 alkyl, hydroxyl-substituted C1-C3 alkyl, C1-C3 alkoxy, NMe₂-substituted C1-C3 alkyl, NMe₂-substituted C1-C3 alkoxy, 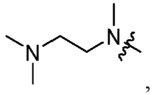 NMe₂, C3-C6 cycloalkyl, (4- to 12-membered) heterocycloalkyl or -CH₂(4- to 12-membered) heterocycloalkyl, wherein the (4-to 12-membered) heterocycloalkyl can be optionally substituted with 1 to 3 R⁵, and R⁵ is independently H, halogen, CN, OH, C1-C3 alkyl, halogen-substituted C1-C3 alkyl, hydroxyl-substituted C1-C3 alkyl, cyano-substituted C1-C3 alkyl, C3-C6 cycloalkyl, halogen-substituted C3-C6 cycloalkyl, 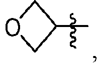 NR⁶R⁷ or -(C1-C3 alkyl)-NR⁶R⁷, wherein R⁶ and R⁷ are independently H or C1-C3 alkyl, or R⁶ and R⁷ form a 4- to 7-membered heterocycloalkyl with an N atom to which they are both connected; wherein two R4 can, together with C atoms, form a C2-C3 alkylene; wherein R⁴ or R⁵ cannot be halogen when connected to a heteroatom.

6. The compound or the isomers, the crystalline forms, the pharmaceutically acceptable salts, the hydrates or the solvates thereof according to claim 5, wherein in the general formula (1), each R⁴ is independently H, F, Cl, Me, Et, 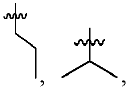 CF₃, CH₂CF₃, CH₂OH, CH₂CH₂OH, OMe, OEt, 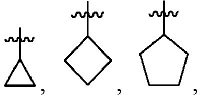 NMe₂, 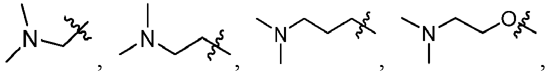 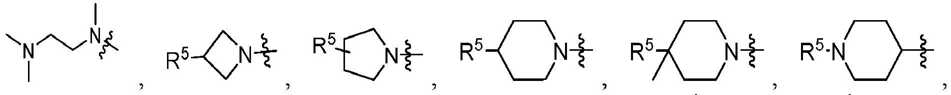 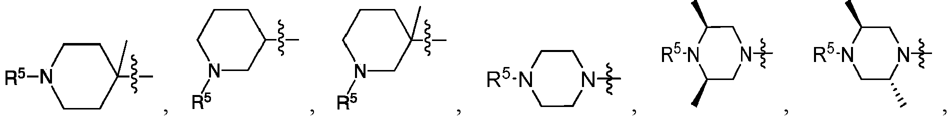 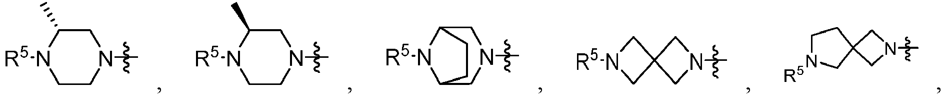 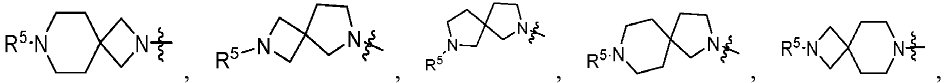 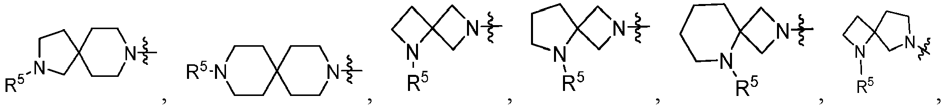 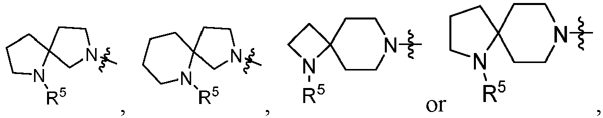 or wherein two R⁴, together with C atoms, form spirocyclopropyl ( 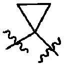 ) or spirocyclobutyl ( 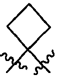 ), and R⁵ is H, halogen, CN, OH, C1-C3 alkyl, halogen-substituted C1-C3 alkyl, hydroxyl-substituted C1-C3 alkyl, cyano-substituted C1-C3 alkyl, C3-C6 cycloalkyl, halogen-substituted C3-C6 cycloalkyl, NH₂, NHMe, NMe₂,

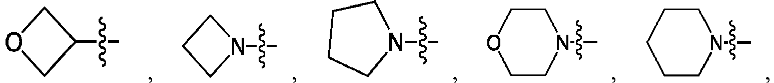

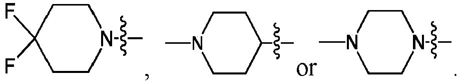

7. The compound or the isomers, the crystalline forms, the pharmaceutically acceptable salts, the hydrates or the solvates thereof according to claim 6, wherein in the general formula (1), 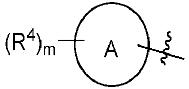 is the following group: 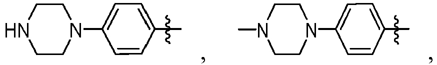 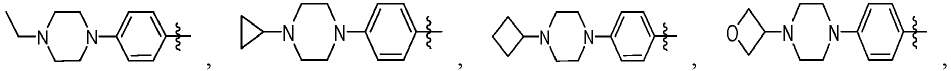 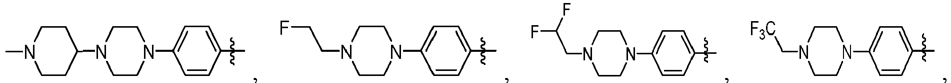 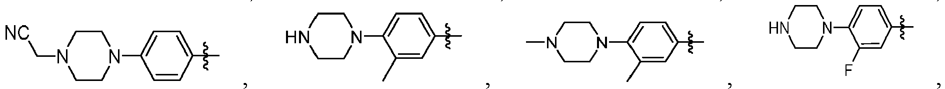 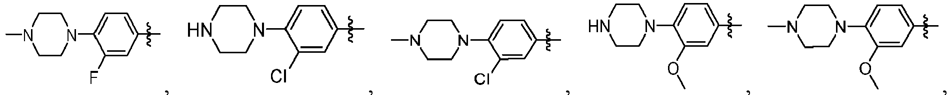 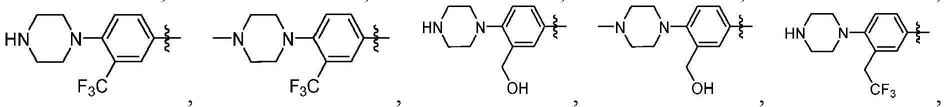 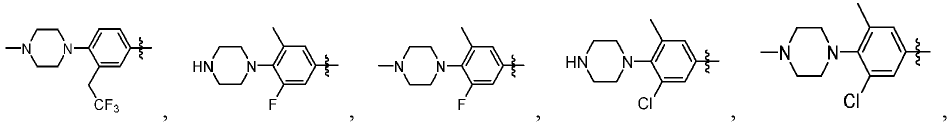 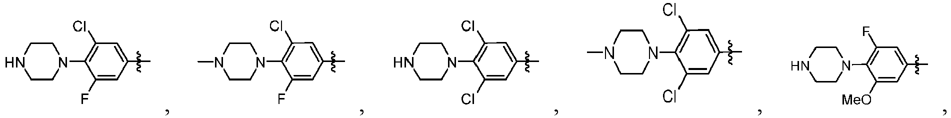 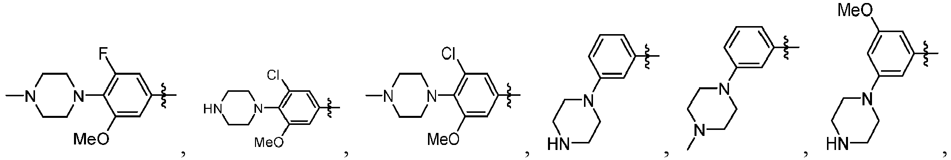 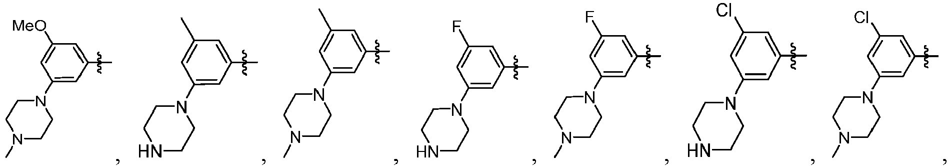 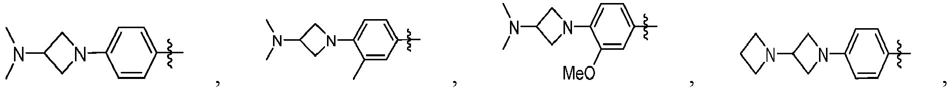 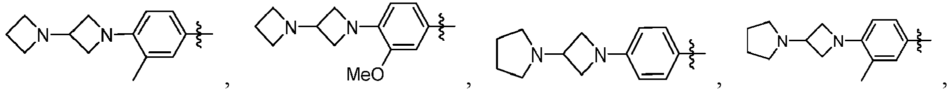 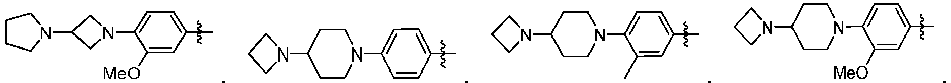 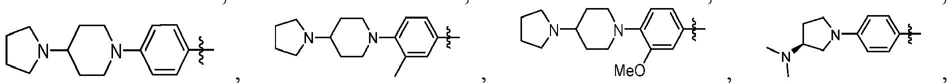 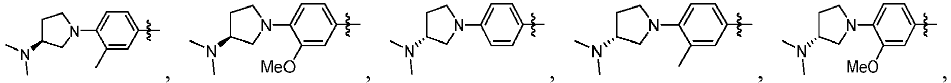 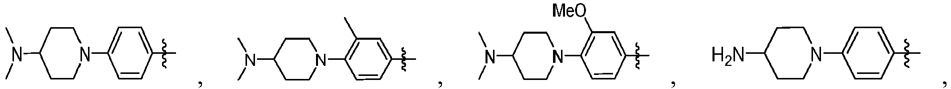 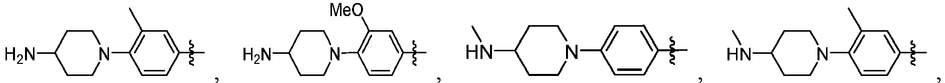 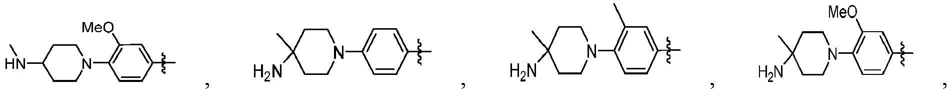 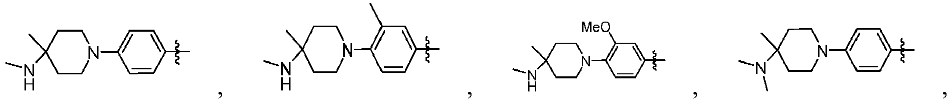 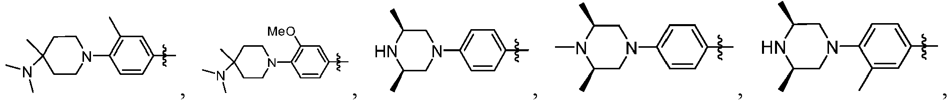 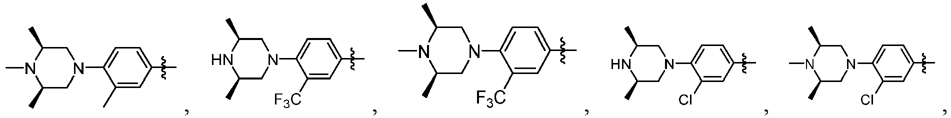 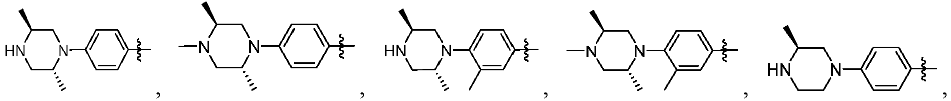 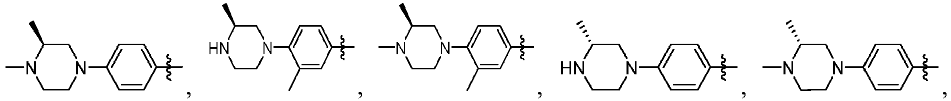 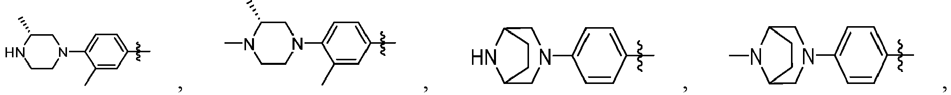 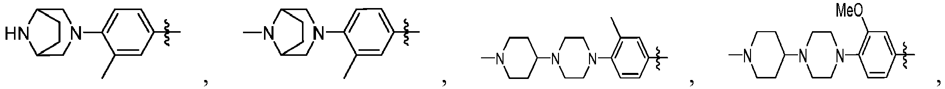 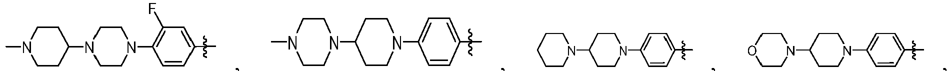 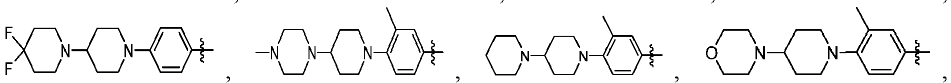 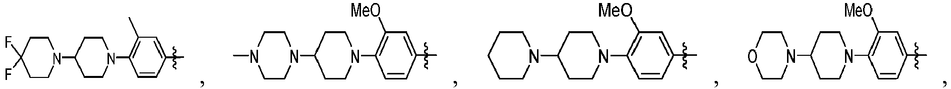 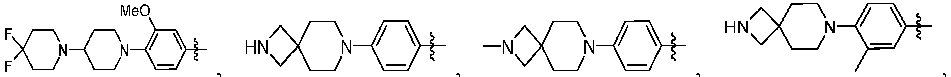 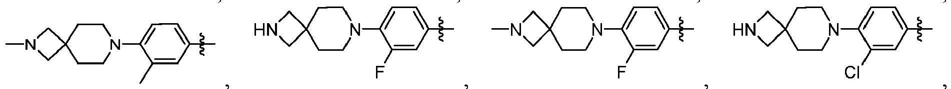 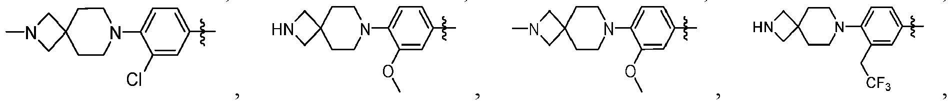 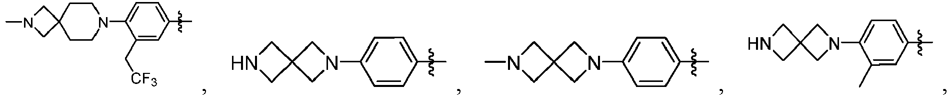 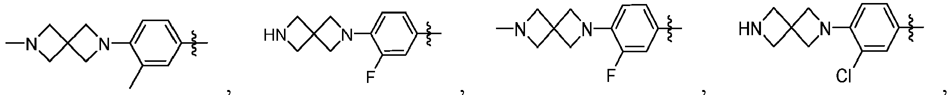 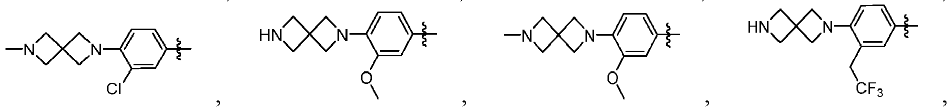 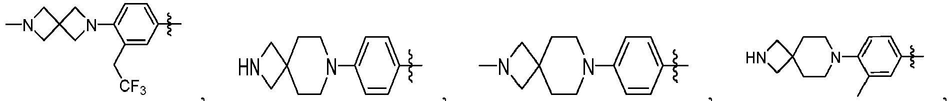 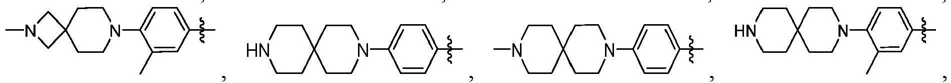 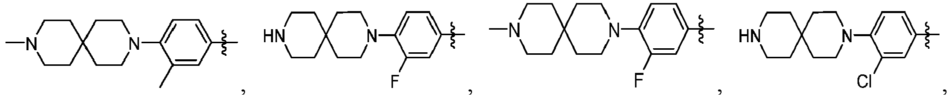 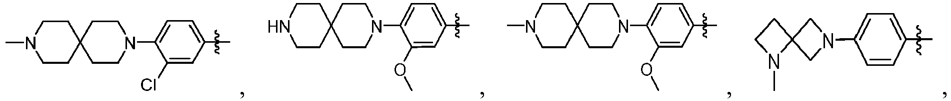 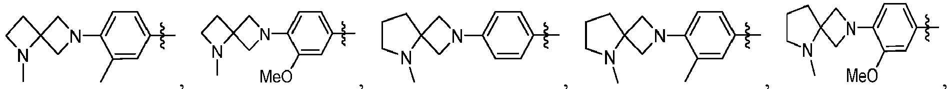 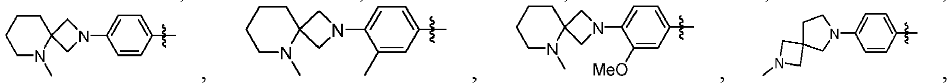 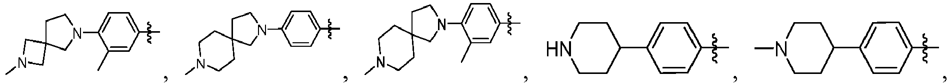 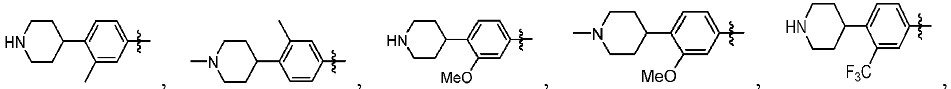 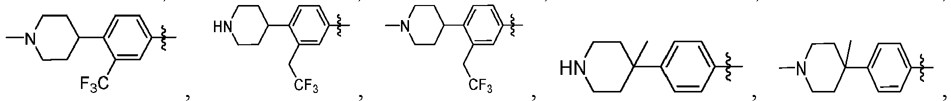 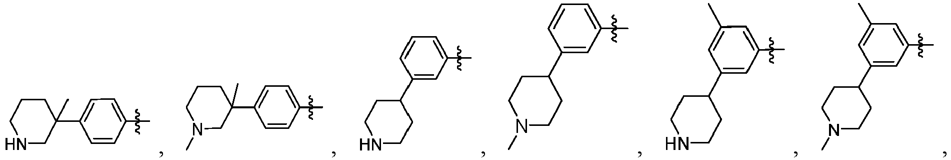 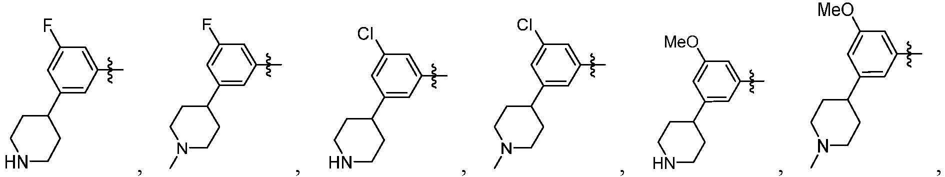 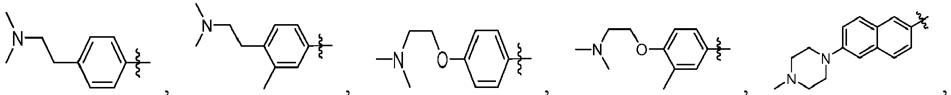 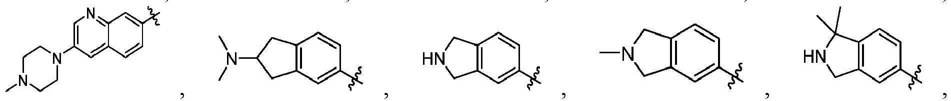 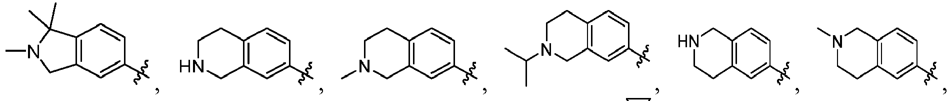 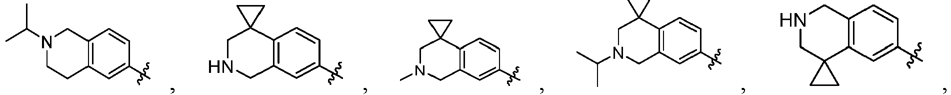 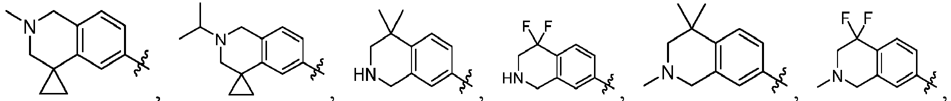 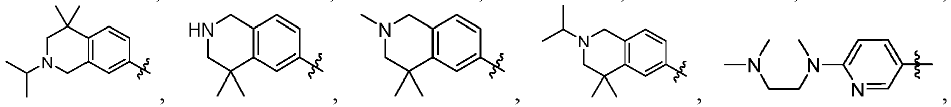 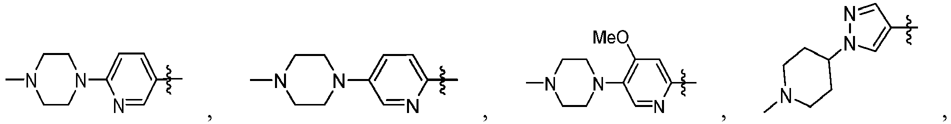 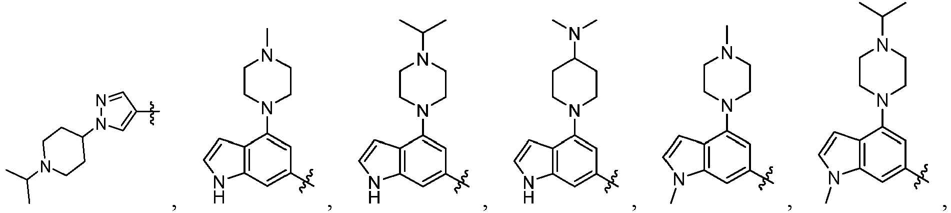 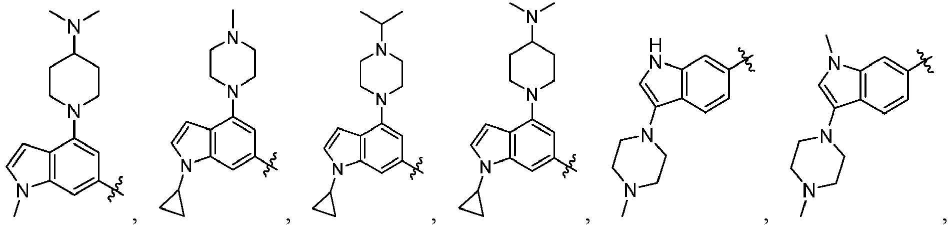 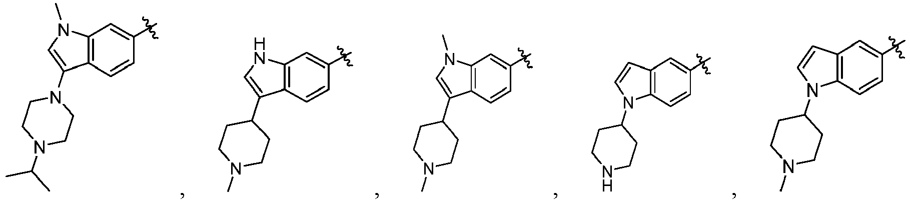 or

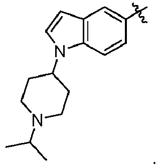

8. The compound, or the isomers, the crystalline forms, the pharmaceutically acceptable salts, the hydrates or the solvates thereof according to claims 1-7, wherein the compound has one of the following structures:

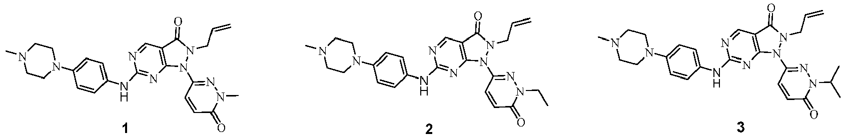

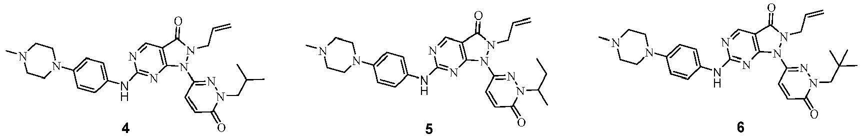

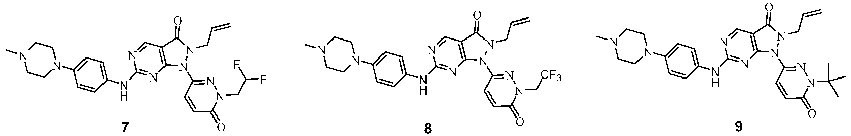

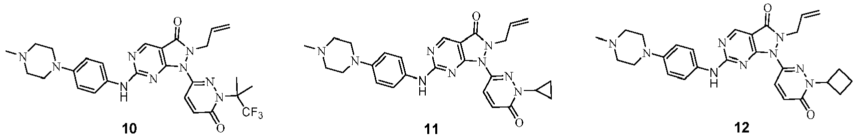

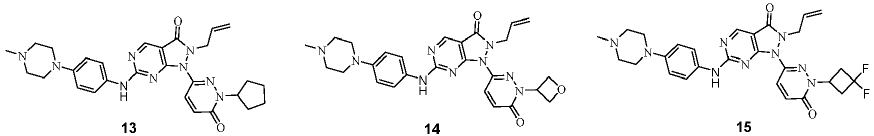

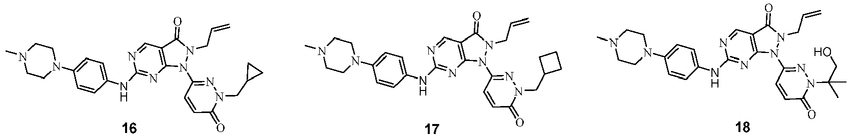

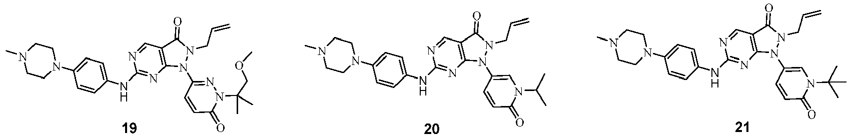

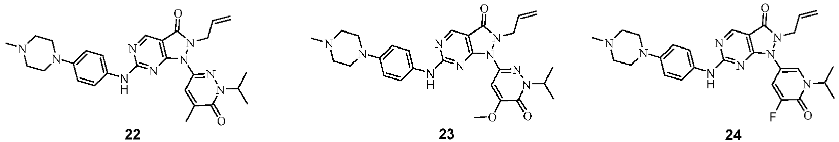

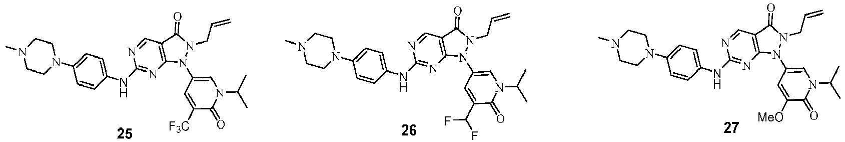

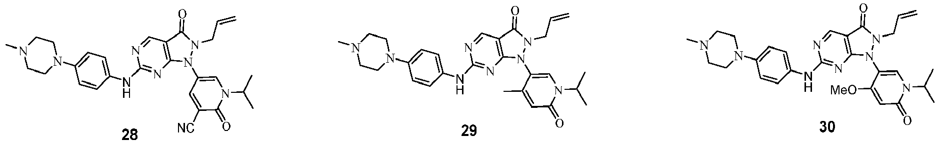

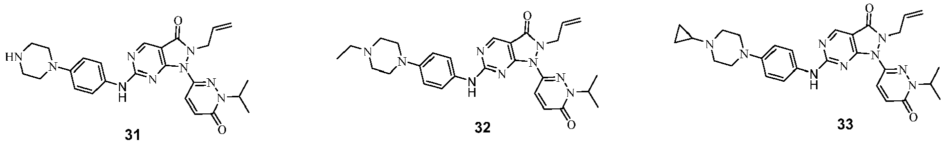

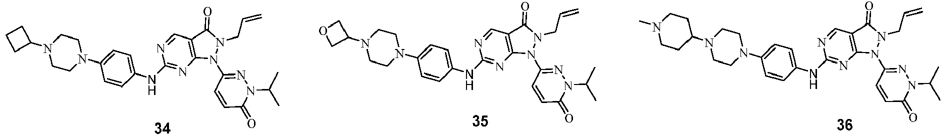

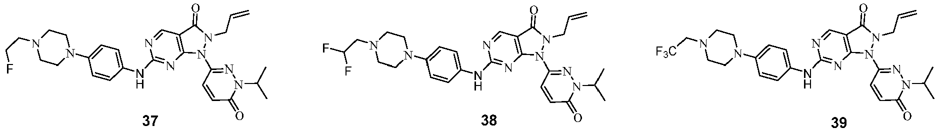

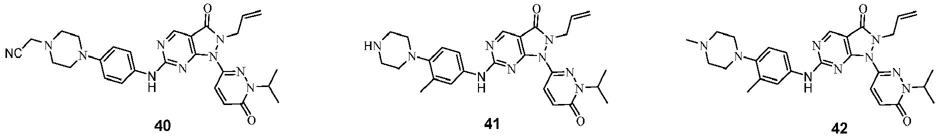

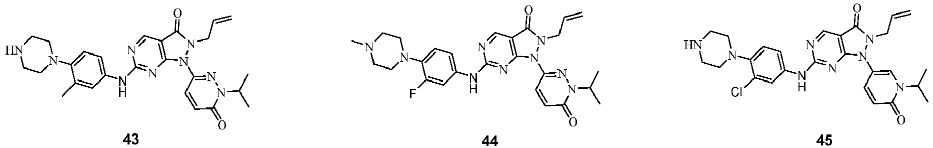

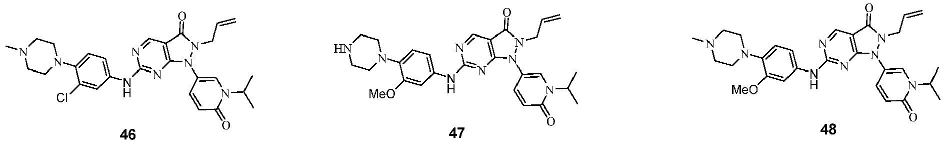

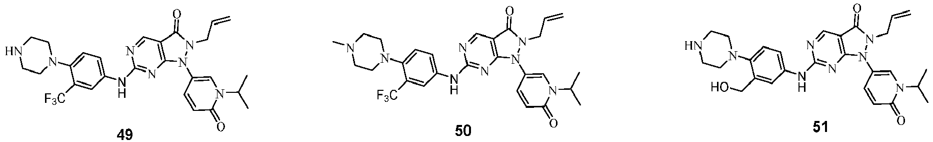

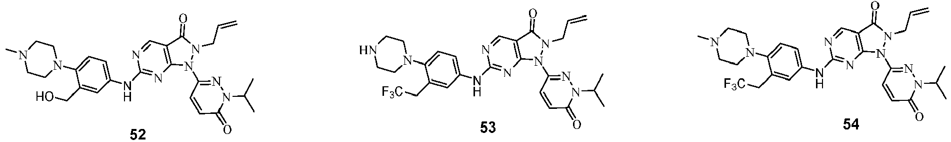

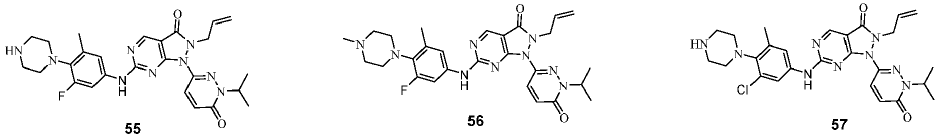

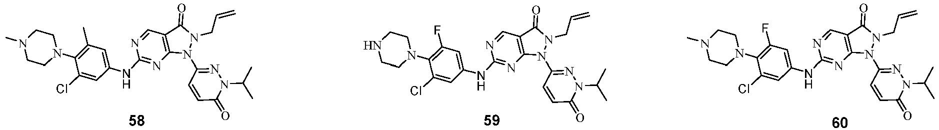

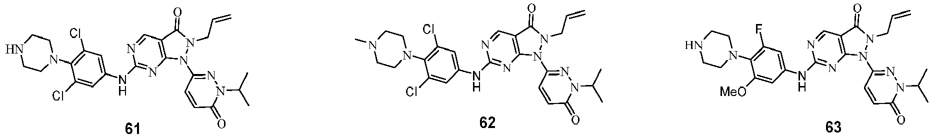

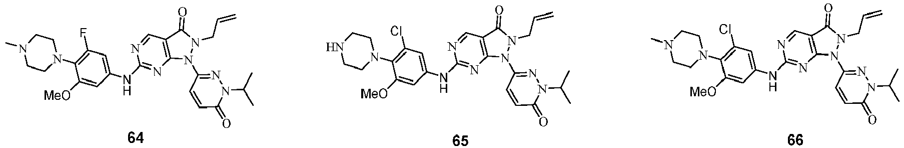

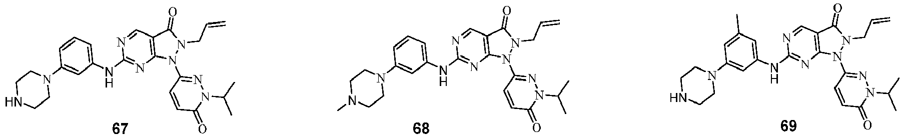

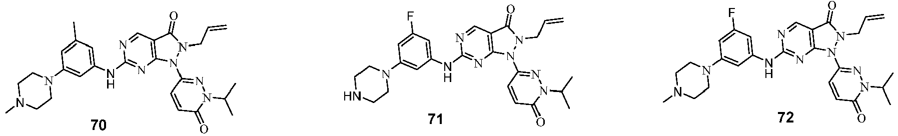

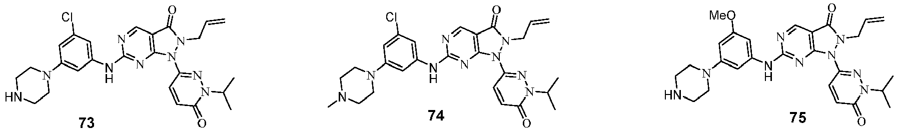

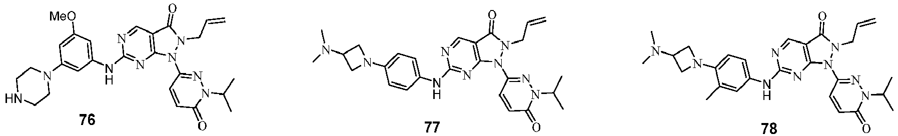

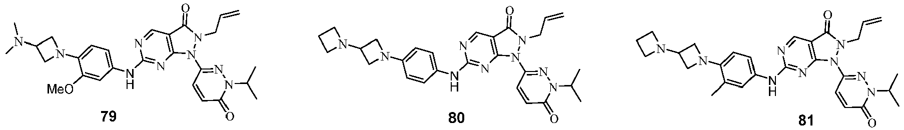

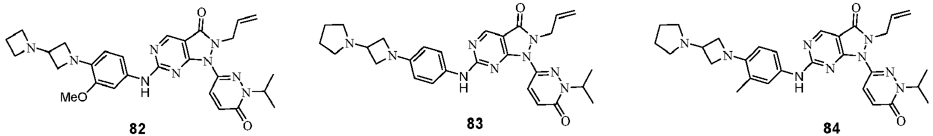

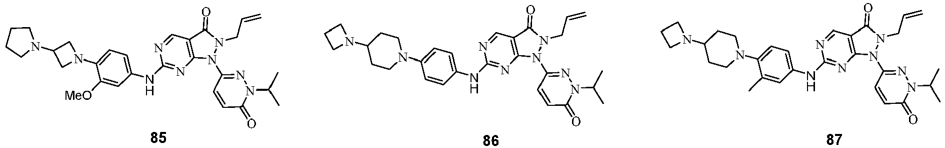

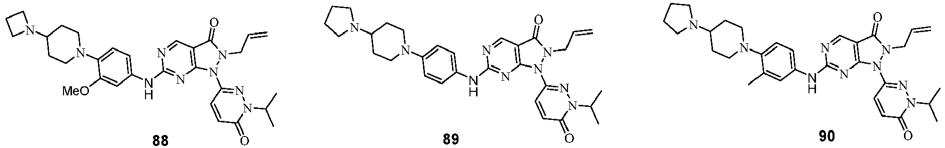

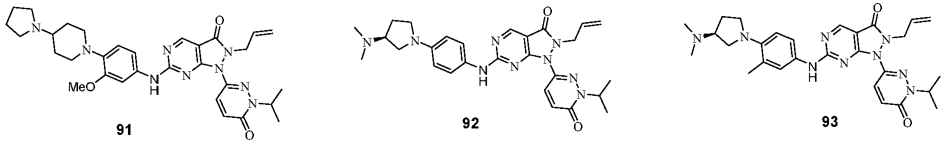

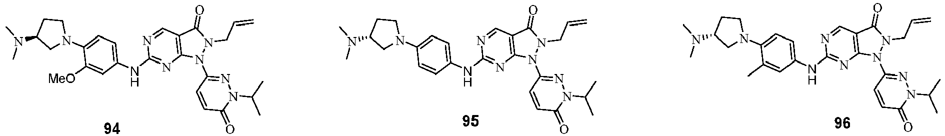

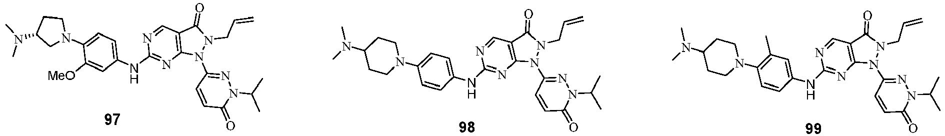

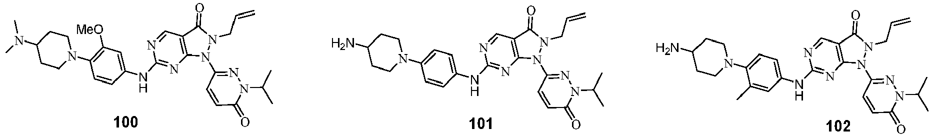

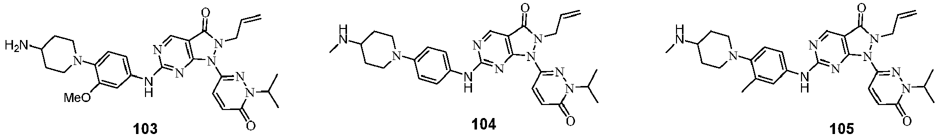

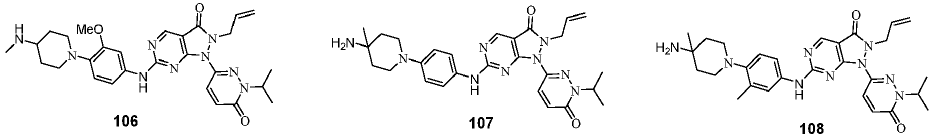

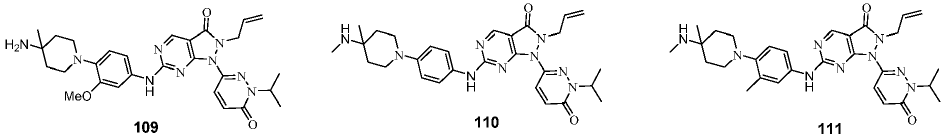

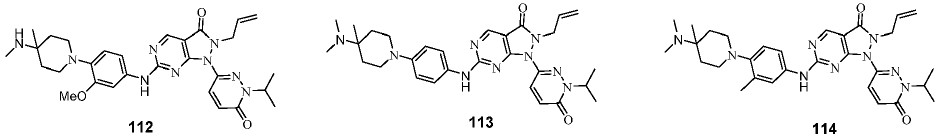

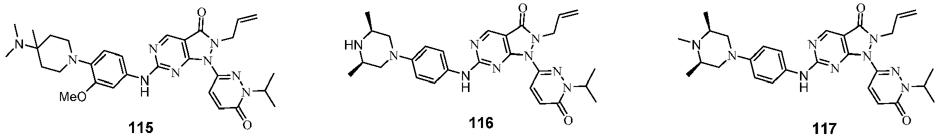

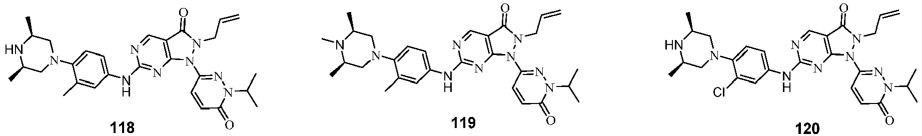

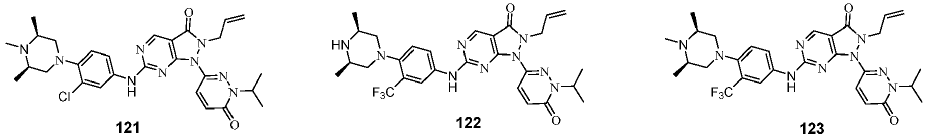

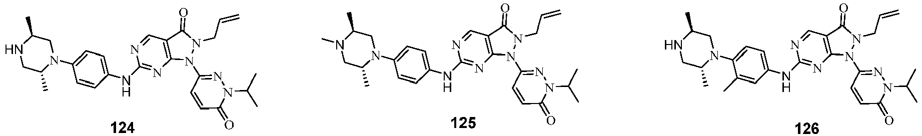

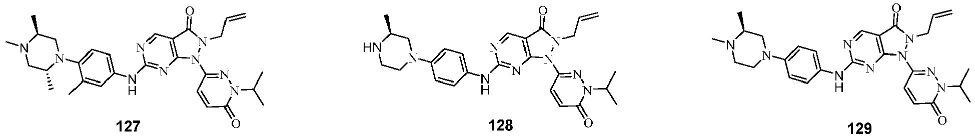

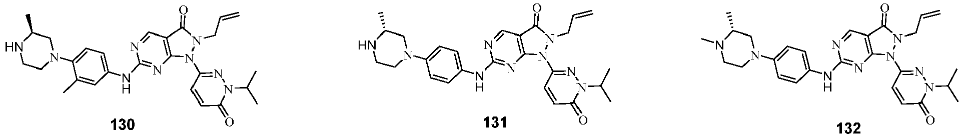

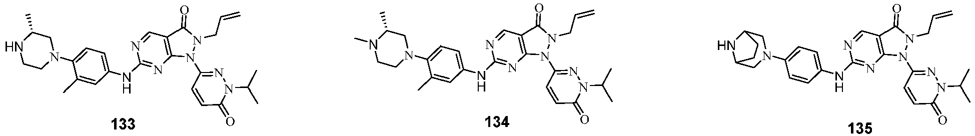

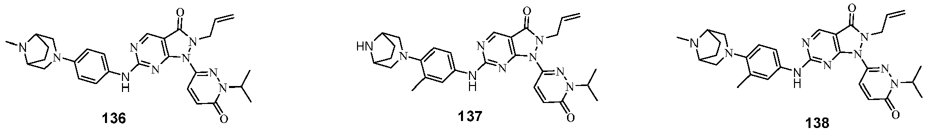

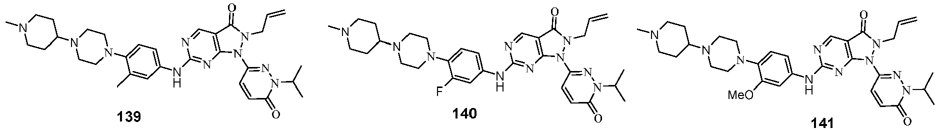

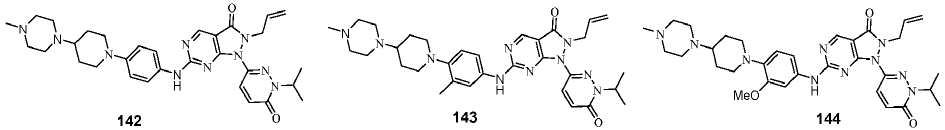

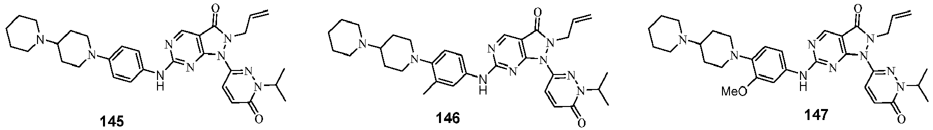

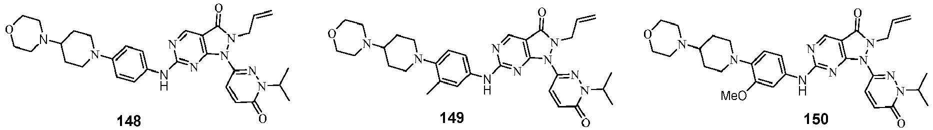

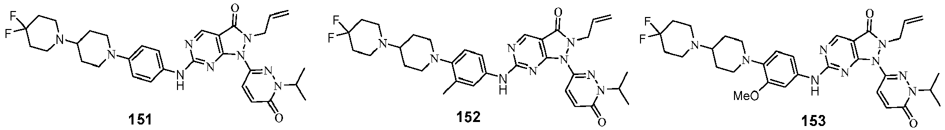

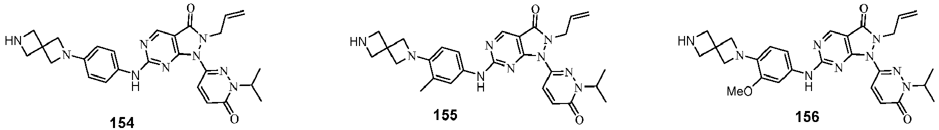

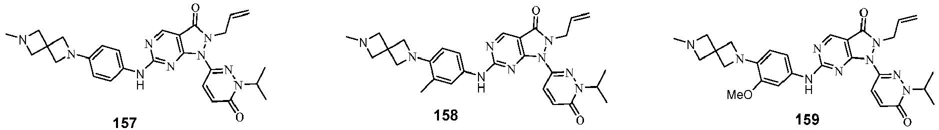

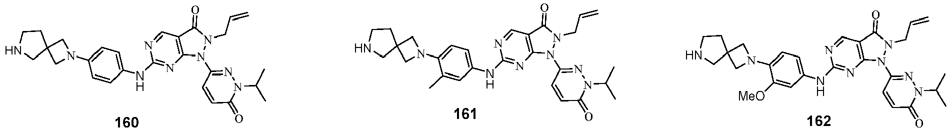

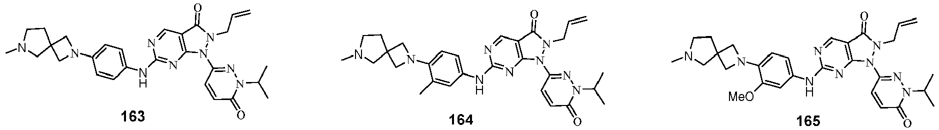

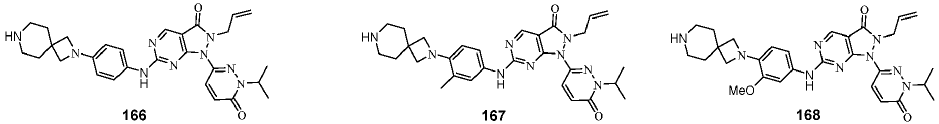

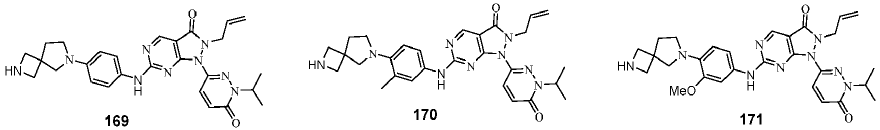

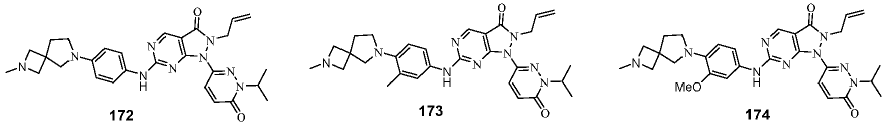

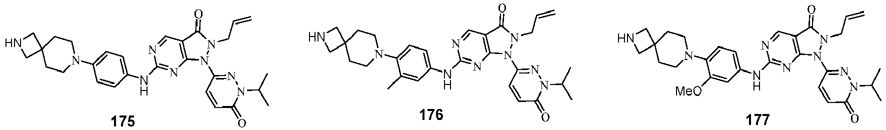

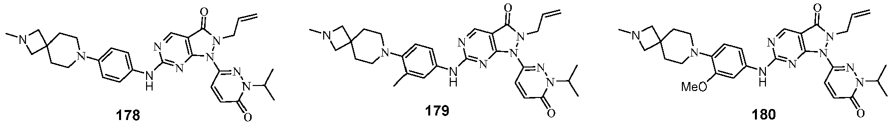

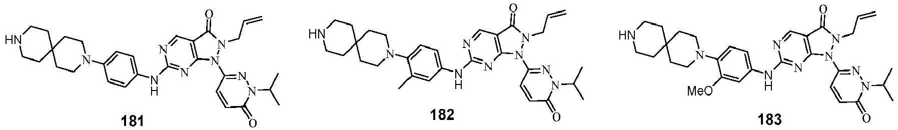

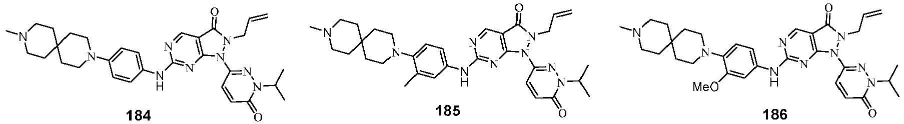

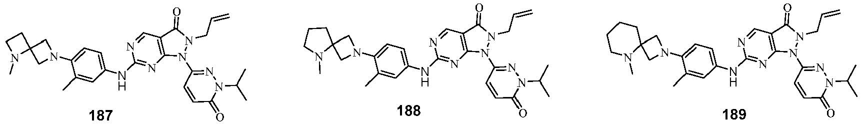

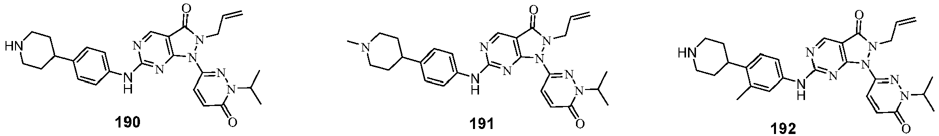

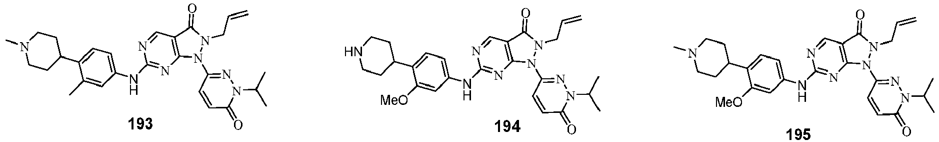

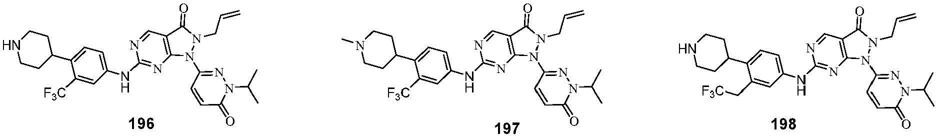

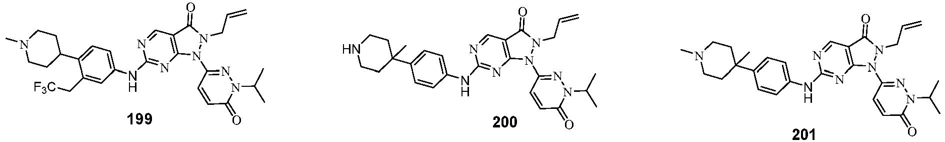

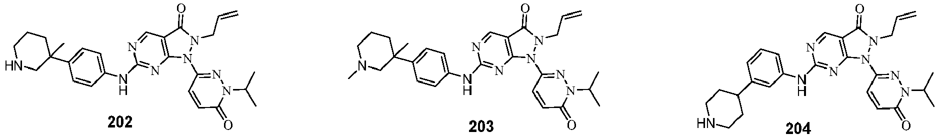

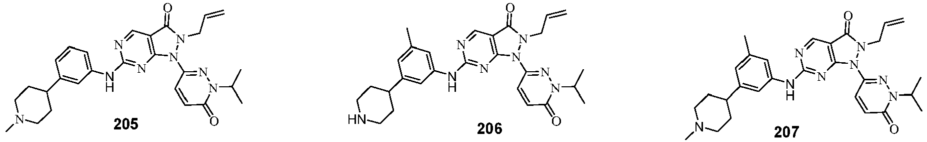

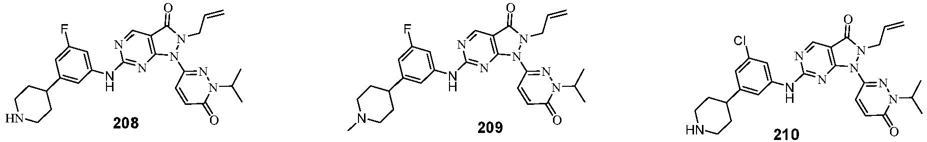

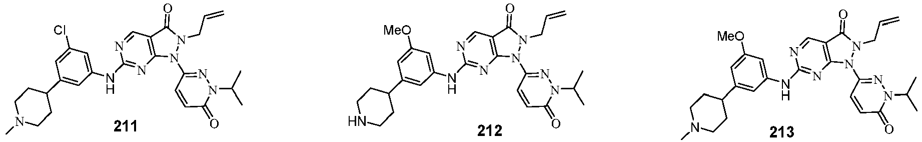

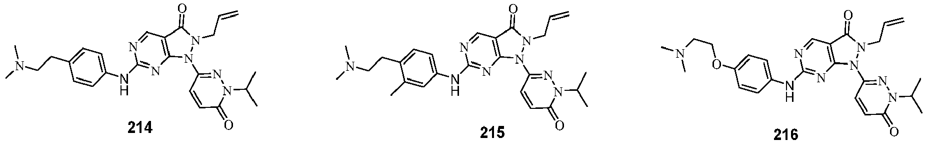

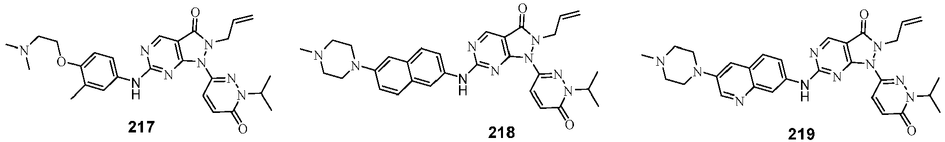

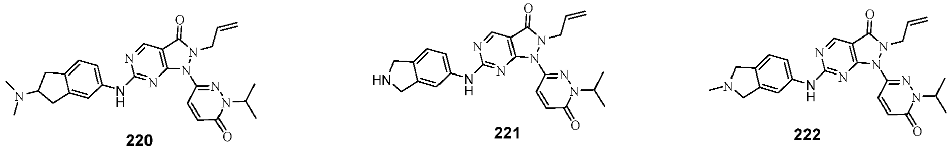

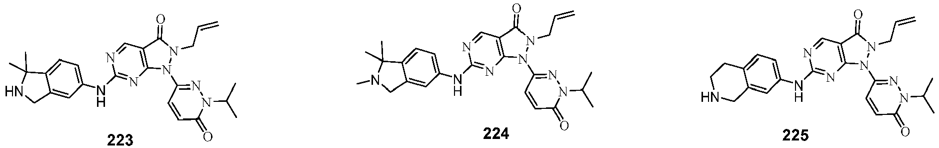

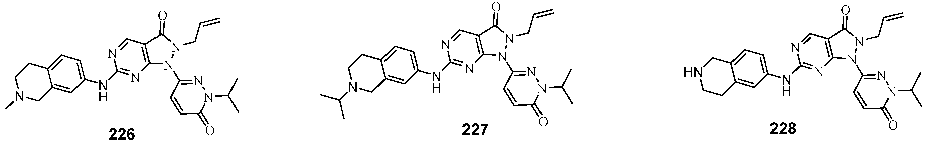

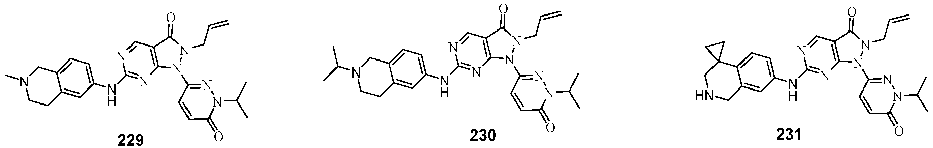

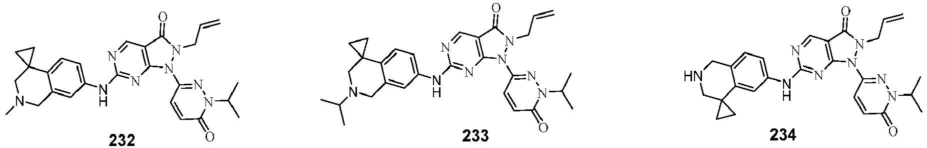

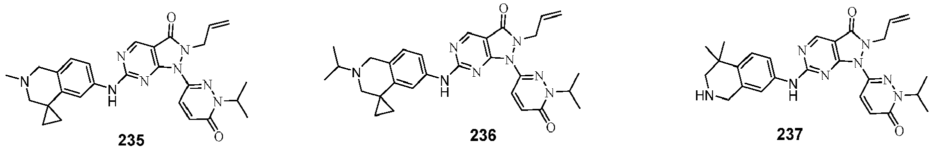

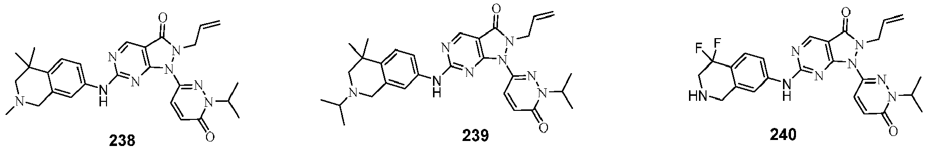

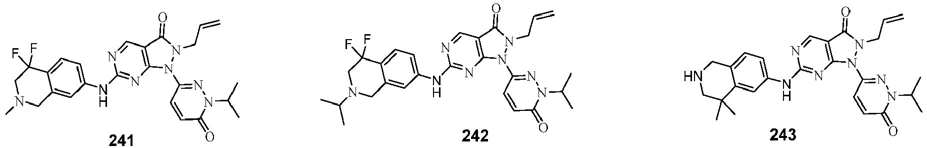

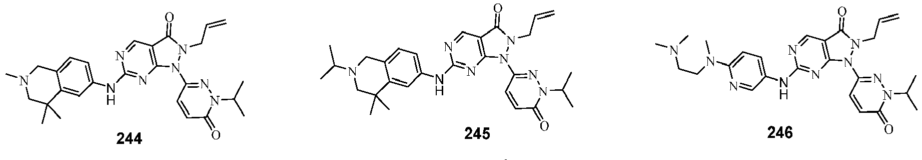

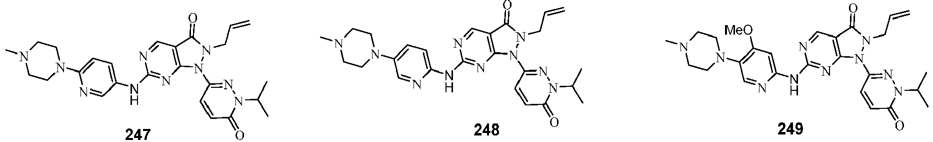

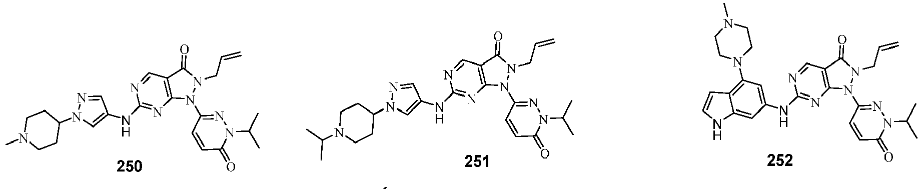

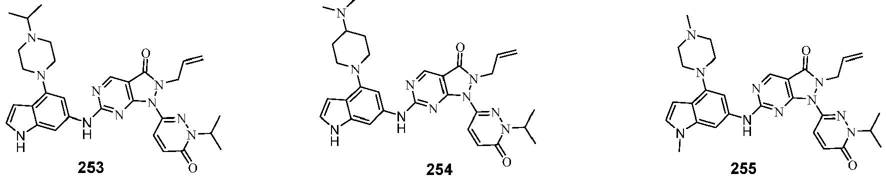

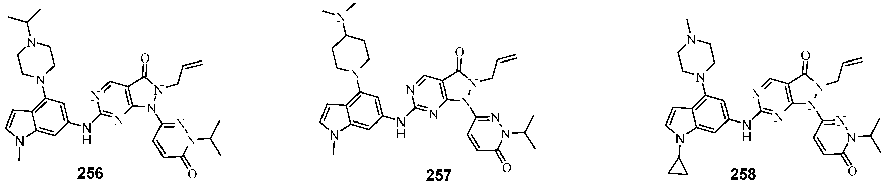

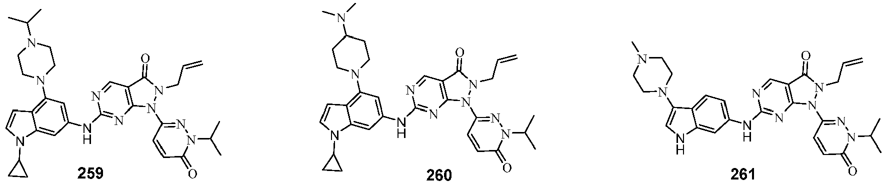

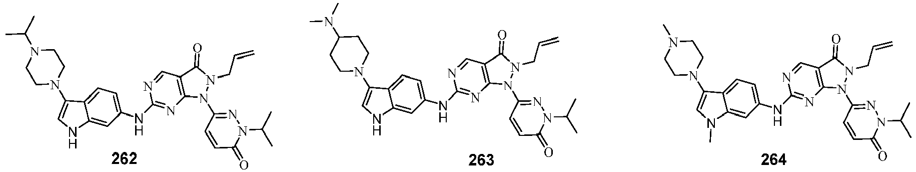

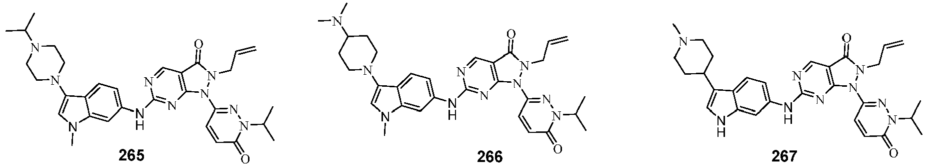

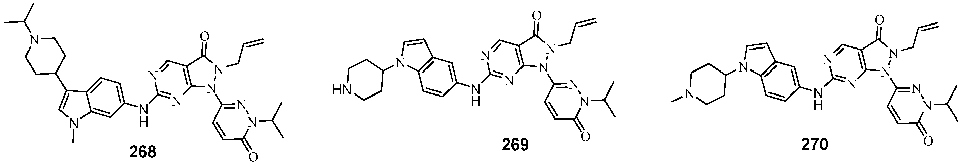

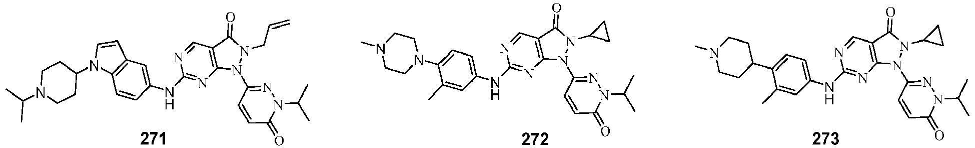

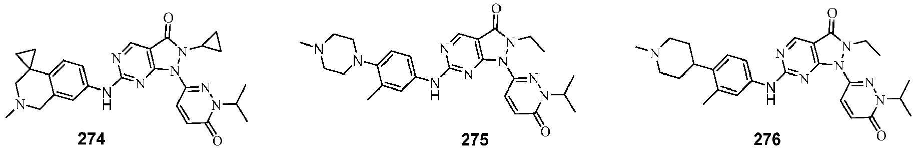

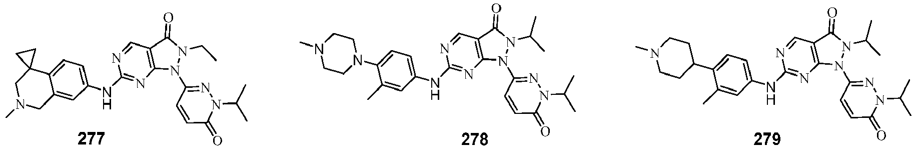

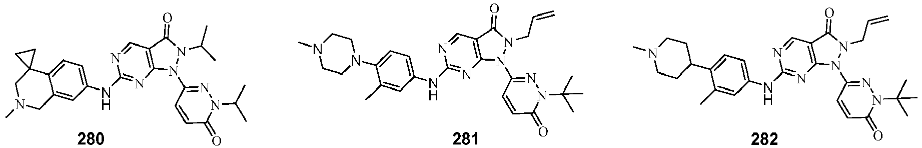

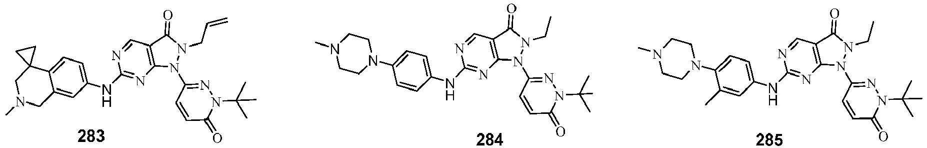

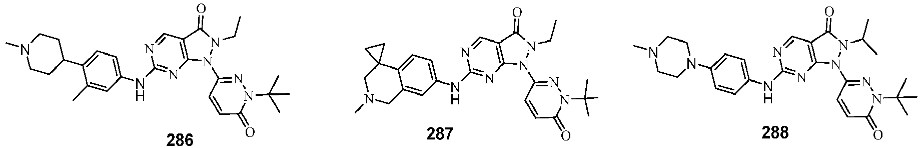

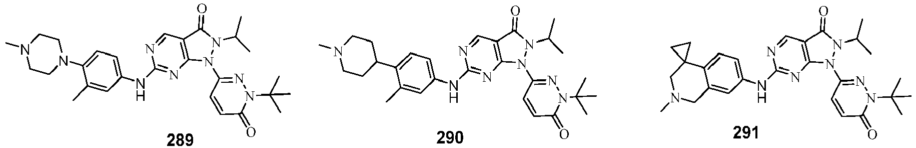

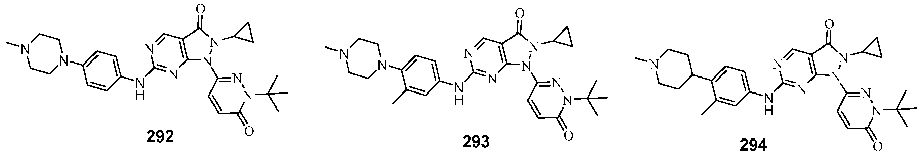

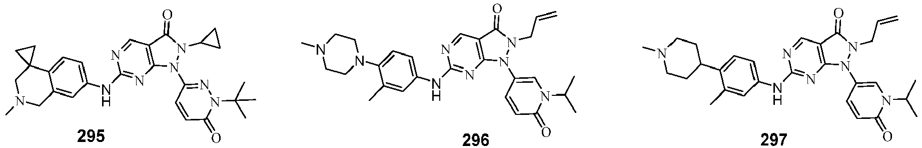

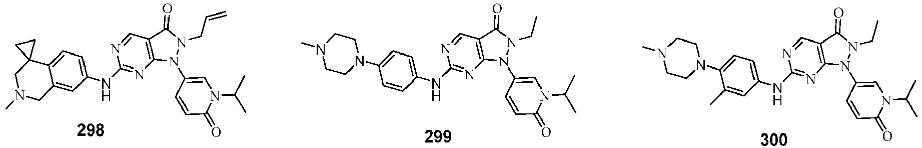

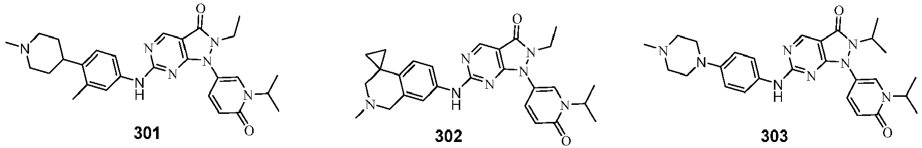

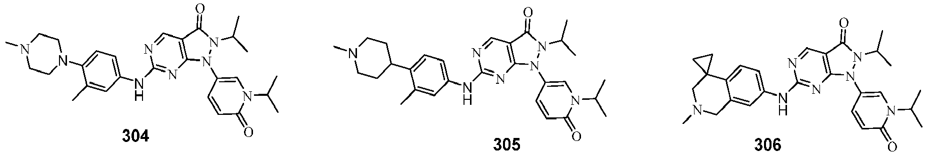

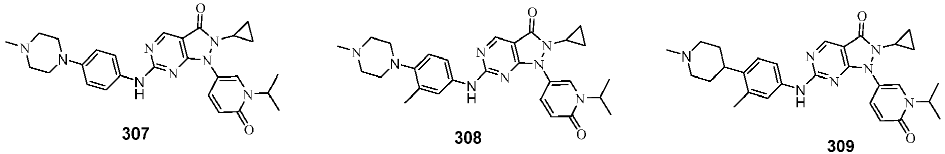

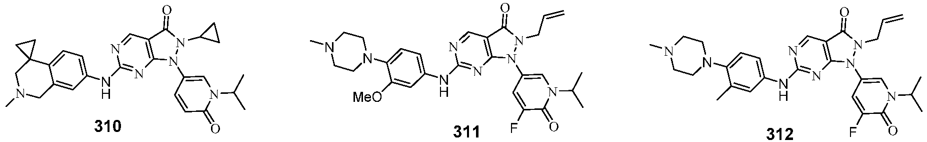

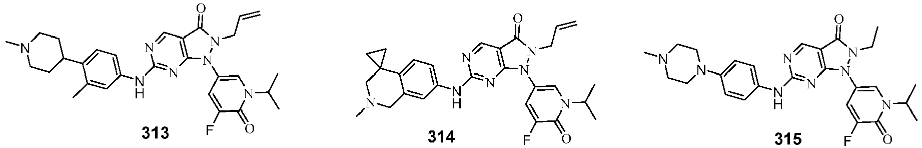

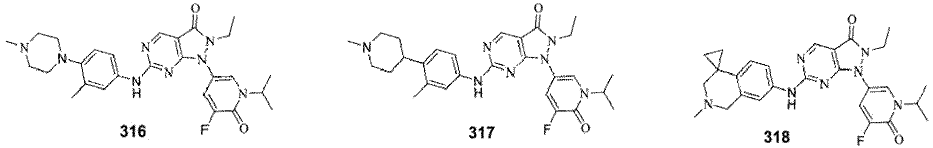

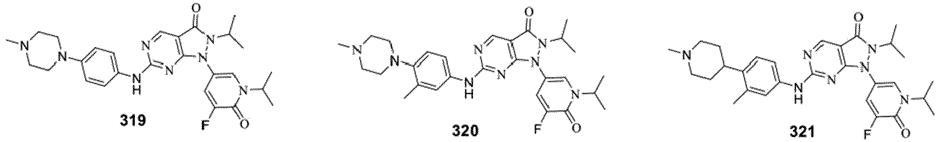

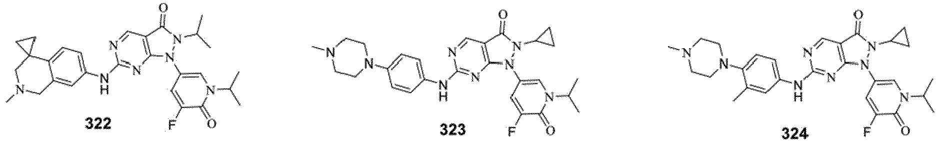

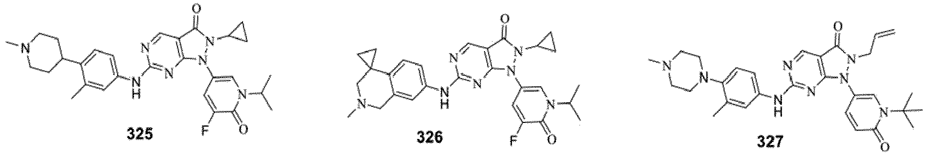

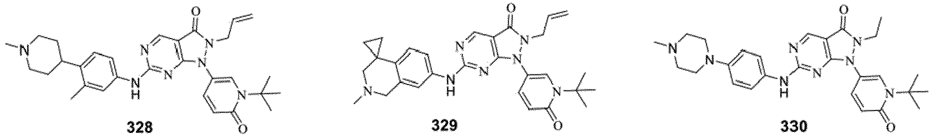

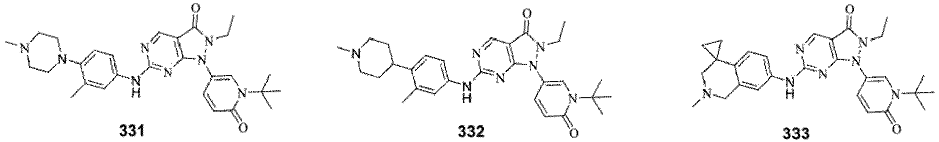

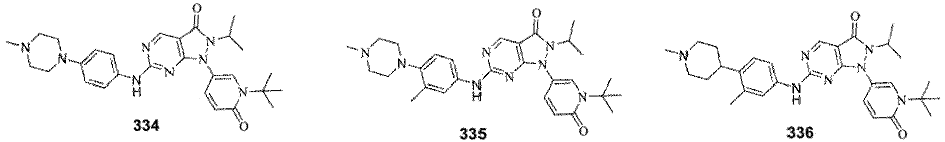

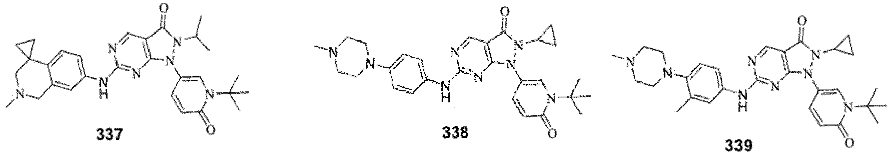

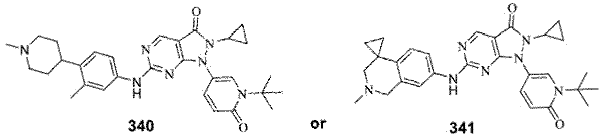

9. A pharmaceutical composition comprising a therapeutically effective dose of an active ingredient and a pharmaceutically acceptable adjuvant, wherein the active ingredient comprises the compound as shown in the general formula (1) or the isomers, the crystalline forms, the pharmaceutically acceptable salts, the hydrates or the solvates thereof according to any one of claims 1-8, and the pharmaceutically acceptable adjuvant is a pharmaceutically acceptable carrier, a diluent and/or an excipient.

10. Use of the compound of general formula (1) or the isomers, the crystalline forms, the pharmaceutically acceptable salts, the hydrates or the solvates thereof according to any one of claims 1-8, or the composition according to claim 9 in preparing a Wee-1 inhibitor.

11. Use of the compound of general formula (1) or the isomers, the crystalline forms, the pharmaceutically acceptable salts, the hydrates or the solvates thereof according to any one of claims 1-8, or the composition according to claim 9 in preparing a medicament for treating related disease mediated by Wee-1.
